(19) Europäisches Patentamt
European Patent Office
Office européen des brevets

(11) EP 2 776 585 B1

(12) **EUROPÄISCHE PATENTSCHRIFT**

(45) Veröffentlichungstag und Bekanntmachung des Hinweises auf die Patenterteilung:
**22.08.2018 Patentblatt 2018/34**

(21) Anmeldenummer: **12798176.9**

(22) Anmeldetag: **12.11.2012**

(51) Int Cl.:
***C12Q 1/68*** *(2018.01)*

(86) Internationale Anmeldenummer:
**PCT/EP2012/072402**

(87) Internationale Veröffentlichungsnummer:
**WO 2013/079307 (06.06.2013 Gazette 2013/23)**

(54) **BIFUNKTIONALE OLIGONUKLEOTIDSONDE ZUR UNIVERSELLEN ECHTZEIT MULTIANALYT DETEKTION**

BIFUNCTIONAL OLIGONUCLEOTIDPROBE SUITABLE FOR UNIVERSAL REAL-TIME MULTIANALYTE DETECTION

SONDE D'OLIGONUCLEOTIDE BIFONCTIONALE POUT LA DETECTION UNIVERSELLE DE PLUSIEURS ANALYTES EN TEMPS REEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.11.2011 DE 102011055247**

(43) Veröffentlichungstag der Anmeldung:
**17.09.2014 Patentblatt 2014/38**

(73) Patentinhaber: **Albert-Ludwigs-Universität Freiburg**
**79085 Freiburg (DE)**

(72) Erfinder:
- **ROTH, Günter**
  **79110 Freiburg (DE)**
- **FALTIN, Bernd**
  **70839 Gerlingen (DE)**
- **STETTEN, Felix**
  **79112 Freiburg (DE)**
- **WALDE, Simon**
  **79106 Freiburg (DE)**

(74) Vertreter: **Hertin und Partner**
**Rechts- und Patentanwälte PartG mbB**
**Kurfürstendamm 54/55**
**10707 Berlin (DE)**

(56) Entgegenhaltungen:


- **HALL J G ET AL: "Sensitive detection of DNA polymorphisms by the serial invasive signal amplification reaction", PROCEEDINGS OF THE NATIONAL ACADEMY OF SCIENCES, NATIONAL ACADEMY OF SCIENCES, US, Bd. 97, Nr. 15, 18. Juli 2000 (2000-07-18) , Seiten 8272-8277, XP002975406, ISSN: 0027-8424, DOI: 10.1073/PNAS.140225597**
- **INPLEX: "InPlex Technology", GOOGLE , 2009, XP002694248, Gefunden im Internet: URL:http://www.inplexcf.com/laboratory/inplextechnology.html [gefunden am 2013-03-19]**
- **LI X ET AL: "Universal molecular beacon-based tracer system for real-time polymerase chain reaction", ANALYTICAL CHEMISTRY, AMERICAN CHEMICAL SOCIETY, US, Bd. 78, Nr. 22, 15. November 2006 (2006-11-15), Seiten 7886-7890, XP008136133, ISSN: 0003-2700 [gefunden am 2006-10-14]**
- **NUOVO G J ET AL: "IN SITU AMPLIFICATION USING UNIVERSAL ENERGY TRANSFER-LABELED PRIMERS", JOURNAL OF HISTOCHEMISTRY AND CYTOCHEMISTRY, HISTOCHEMICAL SOCIETY, NEW YORK, NY, US, Bd. 47, Nr. 3, 1. März 1999 (1999-03-01), Seiten 273-279, XP008002684, ISSN: 0022-1554**
- **LOCKETT M R ET AL.: "Molecular beacon-style hybridization assay for quantitative analysis of surface invasive cleavage reactions", ANALYTICAL CHEMISTRY, Bd. 79, 2007, Seiten 6031-6036, XP002694249, in der Anmeldung erwähnt**


Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 776 585 B1

- LU M ET AL: "A surface invasive cleavage assay for highly parallel SNP analysis", HUMAN MUTATION, JOHN WILEY & SONS, INC, US, Bd. 19, Nr. 4, 1. April 2002 (2002-04-01), Seiten 416-422, XP002317018, ISSN: 1059-7794, DOI: 10.1002/HUMU.10071 in der Anmeldung erwähnt
- FALTIN B ET AL.: "Mediator probe PCR: a novel approach for detection of real-time PCR based on lavbel-free primary probes and standardized secondary universal fluorogenic reporters", CLINICAL CHEMISTRY, Bd. 58, 1. November 2012 (2012-11-01), Seiten 1546-1556, XP002694250,

## Beschreibung

Die Erfindung betrifft ein Verfahren zur Detektion von mindestens einem Zielmolekül, umfassend folgende Schritte: a) Bereitstellen einer Mediator-Sonde zur Detektion von mindestens einem Zielmolekül umfassend eine Sonden-Region und eine Mediator-Region, wobei die Mediator-Sonde ein Oligonukleotid ist und die Sonden-Region am 3'-Terminus und die Mediator-Region am 5'-Terminus des Oligonukleotides angeordnet ist, wobei zwischen den Regionen eine chemische, biologische und/oder physikalische Spaltstelle vorliegt und die Sonden-Region eine Affinität zu einem Vorlagemolekül und die Mediator-Region eine weitere Affinität zu einem Nachweismolekül aufweist; b) Bereitstellen eines Nachweismoleküls, wobei das Nachweismolekül ein Oligonukleotid ist und mindestens folgende Regionen aufweist: (i) eine erste Region an einem 5'-Terminus des Nachweismoleküls, die einen Fluoreszenzakzeptor oder einen Fluoreszenzdonor und/oder eine chemische Gruppe zur Bindung an eine Festphase und/oder eine chemische Schutzgruppe aufweist, (ii) eine zweite Region, die mit der Mediator-Region interagiert und (iii) eine dritte Region, die einen Fluoreszenzdonor oder einen Fluoreszenzakzeptor und/oder eine chemische Schutzgruppe aufweist; c) Bindung der Sonden-Region der Mediator-Sonde an eine Sequenz des Vorlagemoleküls und/oder des Zielmoleküls; d) Amplifikation des Vorlagemoleküls und/oder des Zielmoleküls mit Hilfe eine Hilfsmoleküls, bevorzugt durch einen Amplifikationsvorgang beinhaltend ein isothermales Verfahren und/oder Thermocycling-basiertes Verfahren und/oder durch eine PCR, besonders bevorzugt eine Real-time PCR; e) Spaltung der Mediator-Sonde an der Spaltstelle während Schritt d) durch mindestens das Hilfsmolekül aus Schritt d) oder weitere Hilfsmoleküle; und f) Bindung der abgespaltenen Mediator-Region der Mediator-Sonde an das Nachweismolekül; g) Signaldetektion.

## Stand der Technik

Im Stand der Technik sind zahlreiche molekularbiologische Untersuchungsmethoden beschrieben, die die Detektion und gegebenenfalls Analyse einer Probe ermöglichen. Eine dieser Methoden ist die Detektion und parallele Analyse von mehreren tausend Einzelnachweisen in einer geringen Menge biologischen Probenmaterials mittels eines Microarrays. Es gibt verschiedene Formen von Microarrays, die manchmal auch als "Genchips" oder "Biochips" bezeichnet werden, weil sie wie ein Computerchip viele Informationen auf kleinstem Raum enthalten können.

Mikroarrays erlauben die hoch-parallele Detektion verschiedener Analyte auf einem typischerweise planaren Substrat. Die immobilisierten Sondenmoleküle eines Mikroarrays werden auf einem geeigneten Substrat im Laufe des Herstellungsprozesses an einer definierten Stelle (Spot) eines Rasters (Array) durch Übertragung geringer Flüssigkeitsvolumina immobilisiert beziehungsweise synthetisiert. Diese zweidimensionale, ortsaufgelöste Immobilisierung von Fängermolekülen kann so gestaltet sein, dass Nukleinsäuren oder Peptide beziehungsweise Proteine detektiert werden können. In der Regel erlauben *in situ*-Lithographieverfahren nur die Synthese kurzer Oligonukleotide beziehungsweise Aminosäureketten. Die hergestellten DNA-Mikroarrays können unter geeigneten Bedingungen über Monate gelagert werden, Protein-Arrays können dagegen nur für eine kurze Zeit funktionell aufbewahrt werden.

Für eine Mikroarray-Analyse wird die zu untersuchende Probe mit einem geeigneten Puffer vermischt und in entsprechender Weise mit dem Mikroarray inkubiert, so dass typischerweise bei komplementären Sequenzabschnitten ein Hybridisierungsereignis stattfindet. Aufgrund der geringen Sensitivität von Mikroarray-Systemen wird die Probe im Falle von nachzuweisender Nukleinsäure in einem vorgelagerten Reaktionsschritt amplifiziert (zum Beispiel mittels Polymerase Ketten Reaktion (PCR), RT-PCR oder Whole Genome Amplification (WGA)) und für eine Detektion zum Beispiel mit einem Fluoreszenzfarbstoff markiert oder auf dem Mikroarray mit einer zusätzlichen Detektionssonde inkubiert. Peptide und Proteine können nicht enzymatisch amplifiziert werden und werden durch Aufreinigungen der zu untersuchenden Probe aufkonzentriert. Dem gegenüber stehen Ansätze, die eine Signalverstärkung auf dem Mikroarray nach erfolgter Hybridisierung, zum Beispiel mittels Rolling Circle Amplification (RCA), durchführen. Diese Vorgehensweise umfasst mehrere zeitintensive Arbeitsschritte und erhöht die Gefahr für Ungenauigkeiten und Kontaminationen. Typische Anwendungen finden Mirkoarrays bei Expressionsanalysen, sowie dem Nachweis von Pathogenen oder Resistenz-Markern. Ein Überblick über verschiedene Herstellungstechniken und Anwendungen ist für den Fachmann aus dem Stand der Technik bekannt.

Im Stand der Technik ist die Hybridisierung von amplifizierten DNA-Fragmenten an immobilisierte, allelspezifische Oligonukleotide beschrieben, die zu den Routineverfahren eines Fachmanns gehören. Ein vorgeschalteter Amplifikationsschritt mit Primermolekülen in unterschiedlichen Konzentrationen erlaubt die Generierung einzelsträngiger, markierter DNA, welche bevorzugt mit den Fängermolekülen des Mikroarrays interagiert. Nach Überführen des Mediums auf einen Mikroarray kann nach erfolgter Hybridisierung die Detektion direkt mittels Fluoreszenz oder durch Verwendung von Biotin-markierten Primer erfolgen, wobei nach dem Hybridisierungsschritt eine Inkubation insbesondere mit einer Streptavidin-modifizierten Meerrettichperoxidase erfolgt, welche ein lösliches Substrat in ein unlösliches Reaktionsprodukt umsetzt. Durch die Detektion des chromogenen Niederschlags kann ein Bindeereignis zwischen Ziel- und Fängermolekül nachgewiesen werden. Gegebenenfalls kann nach der Hybridisierung der voramplifizierten Zielsequenz(en) eine Verlängerungsreaktion eines immobilisierten Primers durchgeführt werden, bei der Biotinmarkierte Nukleotide eingebaut werden. Nach Inkubation mit einer Streptavidin-modifizierten Alkalischen Phosphatase und Zugabe eines geeigneten Substrats kann durch Bildung eines chromogenen Reaktionsprodukts die Anwesenheit der Zielsequenz nachgewiesen werden.

In einer weiteren Ausführung (Multiplex quantitative DNA-Array basierte PCR, MQDA-PCR) wird die nachzuweisende Nukleinsäure in einem mehrstufigen Prozess zuerst mit bifunktionellen Primern für wenige PCR-Zyklen parallel amplifiziert. Dabei entstehen Amplifikate mit universellem 5'-Ende, welches anschließend die kompetitive quasi-monoplex Amplifizierung mit universellen Primern ermöglicht. Anschließend wird in einem gesonderten Arbeitsschritt eine Zielsequenz-spezifische Sonde durch Addition eines modifizierten Nukleotids markiert und auf einem Mikroarray hybridisiert. Durch die Verwendung bifunktioneller Primer, kann eine zweistufige PCR-Reaktion mit erhöhtem Multiplexing-Grad durchgeführt werden, ohne dass die Reaktionseffizienz durch unterschiedliche Sequenzen signifikant beeinflusst wird. Nachteilig wirkt sich das komplexe Durchführungsprotokoll auf die Integration in den Arbeitsablauf aus. Die mehrmalige Zugabe und Entnahme von Reagenzien beziehungsweise Reaktionsprodukten sowie das Transferieren des Reaktionsansatzes zwischen mehreren Gefäßen beinhalten hohen Arbeitsaufwand ("handson time"), lange Wartezeiten ("time to result") und die Gefahr von Durchführungsfehlern. Neben dem Ansetzen der einzelnen PCR-Reaktionen beinhaltet die Ausführung mehrere Inkubationsschritte, in denen beispielsweise die bifunktionellen Primer verdaut, Sonden markiert und auf dem Mikroarray inkubiert werden. Bei dieser Methode besteht eine direkte Korrelation zwischen nachzuweisender Zielsequenz und immobilisierten Fängermolekül auf der Chip-Oberfläche. Dadurch kann das Verfahren nicht an abweichende Zielsequenzen angepasst werden, ohne einen neuen Mikroarray herzustellen. Die genannten Verfahren besitzen zusätzlich den Nachteil, dass das Probenmaterial nach der Amplifizierung zwischen zwei Reaktionsgefäßen transferiert werden muss, wobei Durchführungsfehler und Kontaminationen entstehen können.

Eine Möglichkeit, die Kontaminationsgefahr zu minimieren besteht darin, Amplifikation und Hybridisierung in einem Reaktionsgefäß durchzuführen. Durch räumliche Trennung zweier Reaktionskompartimente können verschiedene Reaktionen zeitlich voneinander getrennt in einem Gefäß ablaufen. In dem Stand der Technik ist ein "Microarray-in-a-tube"-System beschrieben, bei welchem zwei Kompartimente und ein Pufferreservoir in einem Reaktionsgefäß integriert sind (Liu,Q. et al, 2007, Microarray-in-a-tube for detection of multiple viruses. Clin Chem, 53, 188-194). Durch Invertieren des Gefäßes wird die Flüssigkeit vom unteren Abschnitt in den oberen Abschnitt überführt und mit einem vorgelagerten Reaktionspuffer vermischt, wobei das Reaktionsgefäß nicht geöffnet werden muss. Damit kann ein Amplifikationsschritt mit einer nachfolgenden Hybridisierung durchgeführt werden, ohne dass das Reaktionsmedium zwischen zwei Gefäßen transferiert oder mit Hilfe aktiver Elemente aktuiert werden muss. Da die immobilisierten Fängermoleküle von der Sequenz der nachzuweisenden Zielsequenz abhängen, können mit einem spezifischen Array-Layout nur ausgewählte Sequenzen detektiert werden. Das hergestellte Mikroarray kann daher nicht an neu ausgewählte Zielsequenzen adaptiert und muss gegebenenfalls in einem neuen Arbeitsablauf hergestellt und in das System integriert werden.

Weiterhin ist im Stand der Technik beschrieben, dass der Nachweis spezifischer Nukleinsäuren in einem Reaktionsansatz mittels Strangverlängerung einer immobilisierten Sonde erfolgen kann. Dazu wird die Zielsequenz in Gegenwart von dUTP amplifiziert und in einem nachgeschalteten Schritt in ungefähr 20 bis 50 Basenpaar lange Abschnitte enzymatisch mittels Uracil-N-Glycosylase fragmentiert. Anschließend wird der Ansatz auf einem Mikroarray inkubiert, welcher aus Zielsequenz-komplementären Sonden besteht, die am 5'-Terminus immobilisiert sind. Nach Hybridisierung entsteht typischerweise ein Duplex, bei dem der 5'-Bereich des Zielmoleküls den 3'-Bereich der Sonde überlappt. Nach Entfernen der ungebundenen Fragmente wird der Array mit einem Reaktionsmix inkubiert, welcher unter anderem eine Polymerase und markierte ddNTPs enthält. Für jedes der vier Nukleotide ist die Markierung (Fluoreszenzfarbstoff) verschieden. Die Verwendung von ddNTPs erlaubt die spezifische Addition genau eines Nukleotids an die Festphasen-Sonde in Gegenwart der komplementären Zielsequenz, da an ddNTPs kein weiteres Nukleotid geknüpft werden kann. Mit geeigneten Detektionsverfahren lässt sich somit ermitteln, welches Nukleotid an welchen Sondenabschnitt angefügt wurde. Daher eignet sich die Methode zur Detektion der Zielsequenz und Sequenzierung, sofern geeignete Sondenabschnitte verwendet werden. In einer speziellen Ausführung, erlaubt das Verfahren einen hohen Multiplex-Grad und den parallelen SNP-Nachweis von bis 640 Zielsequenzen. Wie bei anderen Verfahren des Standes der Technik, bei denen ein Reaktionsansatz nach durchgeführter Amplifikation transferiert werden muss, besteht auch bei diesem Ansatz die Gefahr der Kontamination und Durchführungsfehler. Typischerweise müssen Festphasensonden den kompletten nachzuweisenden Lokus abdecken, womit mehrere Sonden pro Sequenzabschnitt benötigt werden.

In der US 5,641,658 und US 6,300,070 B1 ist eine PCR-basierte Amplifikationsmethode beschrieben, bei welcher die benötigten Primermoleküle an einer Festphase immobilisiert sind. Dies hat zur Folge, dass die amplifizierten Nukleinsäuremoleküle ebenfalls ausschließlich an der Festphase vorliegen. Bei Vorhandensein der Zielsequenz in der zu untersuchenden Probe kann die Nukleinsäure an ‚Primer 1' binden und dieser enzymatisch verlängert werden. Im wiederkehrenden Denaturierungsschritt dissoziiert die Nukleinsäure-Vorlage vom neu gebildeten Nukleinsäurestrang und steht somit im nächsten Annealingzyklus zur Verfügung. Das gebildete Amplifikationsprodukt kann im nächsten Zyklus mit einem immobilisierten ‚Primer 2' hybridisieren, welcher verlängert werden kann und ebenfalls einen immobilisierten Nukleinsäurestrang bildet. Die durch diesen Vorgang gebildeten Nukleinsäurestränge können mit weiteren Primermolekülen interagieren und als Nukleinsäure-Vorlage dienen. Dieses Verfahren zum Nachweis von Nukleinsäuren wird als "BridgeTM-PCR" bezeichnet.

Durch Immobilisierung verschiedener Primerpaare in einem definierten Array können durch den somit erreichten Multiplexing-Grad mehrere Zielsequenzen parallel nachgewiesen werden. Ein Nachteil dieses Verfahren besteht in der Notwendigkeit, dass die verschiedenen Primermoleküle homogen und mit ausreichender Dichte im jeweiligen Spot verteilt vorliegen müssen, damit der durch Primerverlängerung entstandene Nukleinsäure-Strang eine ausreichende Anzahl weiterer Interaktionspartner für den nächsten Zyklen besitzt. Die Reaktionseffizienz dieser Festphasen-PCR liegt bei etwa 30 %, wobei der Anteil der linearen interphasischen Amplifikation ungefähr zehnmal höher ist als der der Oberflächenamplifikation. Zu vergleichbaren Reaktionseffizienzen (-34 %) kommt Pemov et al. (Pemov,A. et al, 2005, DNA analysis with multiplex microarray-enhanced PCR. Nucleic Acids Res., 33, e11.), wobei für diese Studien in Hydrogel immobilisierte Primer und zusätzliche Primer in der Flüssigphase verwendet wurden. Die 5'-immobilisierten Primer bestehen aus zwei funktionellen Abschnitten und setzten sich aus einem universellen 5'-Bereich und einem zielsequenzspezifischen 3'-Bereich zusammen. Frei in der Reaktionslösung vorliegende universelle Primer besitzen die identische Sequenz wie die universellen Abschnitte der Festphasenprimer. In einer Abfolge von interphasischer und Festphasen-Amplifikation wird eine modifizierte Kopie der Zielsequenz gebildet, die mittels universeller Flüssigphasenprimer in einer "quasi-monoplex" PCR amplifiziert werden kann. Dieses Festphasen-gestützte Multiplexing-Verfahren hat den Nachteil, dass post PCR-Schritte (zum Beispiel Inkubation mit modifizierten Detektionsoligonukleotiden beziehungsweise Wasch- und Inkubationsschritte nach Einbau modifizierter Nukleotide durchgeführt werden müssen.

Ein weiteres im Stand der Technik beschriebene Verfahren, um Amplifikationsprodukte auf einer Festphase während eines Amplifikationsschrittes zu immobilisieren ist die verschachtelte Festphasen-PCR (nested on-chip PCR (NOC PCR). Die Methode bedient sich mindestens drei verschiedener Primermoleküle, von denen zwei Primer das äußere Primerpaar (P1 und P2) bilden und das dritte Primermolekül (P3) innerhalb eines von P1 und P2 begrenzten Abschnitt bindet und P3 an einer Festphase immobilisiert vorliegt. P1 und P2 liegen im Vergleich zu P3 im Überschuss vor. Die Methode stellt ein kombiniertes Flüssigphasen/Festphasenamplifikationssystem dar. Die Vorteile dieses Verfahrens liegen analog zur verschachtelten PCR (nested PCR) in der erhöhten Sensitivität und Spezifität. Durch geeignete Immobilisierungsverfahren und Wahl des Substrats kann ein hoch-paralleler Nachweis mehrerer Zielsequenzen erfolgen. Die Analyse kann mittels sequenzspezifischer Sonden oder markierten Nukleotiden erfolgen, wobei bei letzterer Variante eine Detektion in Echtzeit möglich ist. Ein Nachteil dieses Ansatzes ist die Verwendung Zielsequenzsequenspezifischer P3-Primermoleküle, deren Sequenz direkt mit dem Zielmolekül korreliert.

Die Verwendung immobilisierter Primer-Moleküle zum spezifischen Nachweis von Nukleinsäuren ist nicht nur auf PCR-basierte Methoden beschränkt. Es ist im Stand der Technik beschrieben, dass mittels Helikase-abhängiger Amplifikation ("Helicase Dependent Amplification", HDA) der isotherme Nachweis von Pathogenen innerhalb von 120 Minuten erfolgreich durchgeführt wurde. Die Markierung des Amplifikationsproduktes erfolgt mittels Fluoreszenz-markierter Reverse-Primer. Nach Durchführung der Reaktion wird der Chip gewaschen und das Fluoreszenzsignal ausgewertet. Um eine spezifische und sensitive Reaktion durchzuführen, muss der Reaktionsansatz auf 65°C erwärmt werden. Zusätzlich muss der Chip an ein Pumpenelement angeschlossen werden, damit das relativ hohe Probenvolumen von 50 μL ausreichend durchmischt wird. Die Konnektierung von aktiven Aktuatoren an einen Biochip ist generell als nachteilig anzusehen und erhöht den Arbeitsaufwand. Die erreichte Sensitivität ist für die klinische Diagnostik zu gering. So wurden beispielsweise 5*10^4 Genomäquivalente von Staphylococcus aureus und 1,3*10^5 Genomäquivalente von Neisseria gonorrhoeae als Detektionslimit beschrieben (Andresen, D., von Nickisch-Rosenegk, M. and Bier, F.F.,2009, Helicase dependent OnChipamplification and its use in multiplex pathogen detection, Clin. Chim. Acta, 403, 244-248). Der durch diese Methode hergestellte Biochip ist ausschließlich zur Detektion festgelegter Zielsequenzen zu verwenden, da Analytspezifische Oligonukleotide immobilisiert vorliegen.

Weiterhin sind im Stand der Technik Methoden offenbart, bei denen der Nachweis der Zielsequenz in Echtzeit beziehungsweise ohne weitere Prozessierungsschritte erfolgt. Diese Methoden werden durch ein kombiniertes Amplifikations- und Detektionssystem realisiert. So ist beispielsweise beschrieben, dass die parallele Detektion und Quantifizierung drei verschiedener Viren in Spenderplasma-Proben möglich ist. Es werden ein äußeres und inneres Primerpaar benötigt, wobei das äußere Paar in einer Reversen Transkription verwendet wird. Das innere Paar, bei dem der Forward-Primer in einem Hydrogel-Pad immobilisiert, liegt während der Reverse-Primer frei in der Flüssigphase vor, wird für die on-chip PCR eingesetzt. Unter Verwendung eines geeigneten Farbstoffes werden doppelsträngige Amplifikationsprodukte in den Spots während des Annealingschritts markiert und detektiert. Das Verfahren besitzt einen dynamischen Bereich von 6 Log (10^0 bis 10^6 Kopien). Die Verwendung eines sequenz-unspezifischen Farbstoffes erlaubt die universelle Detektion beliebiger, doppelsträngiger Amplifikationsprodukte. Nachteilig ist allerdings, dass keine Spezifität gewährleistet ist, so dass unspezifische Amplifikationsprodukte nicht unterschieden werden können.

In der DE 103 16 159 A1 wird ein Verfahren beschrieben, bei dem Zielsequenz-spezifische Primermoleküle in einer temperierbaren Flusszelle immobilisiert vorliegen. Die Flusszelle besitzt weiterhin die Eigenschaft, mittels Totaler interner Resonanzfluoreszenz (Total internal resonance fluorescence, TIRF) das Anregungslicht durch eine in der Regel planare Fläche zu leiten. Nach Zugabe der Zielsequenz und typischer PCR-Reagenzien sowie Fluoreszenzmarkierten Nukleotiden (zum Beispiel Cy5-dUTP) kann die DNA-abhängige Festphasenprimerverlängerung durch geeignete Verfahren detektiert werden.

Liu et al. entwickelte eine Multiplex-Analyse für Nukleinsäuren, welche die Amplifikation durch PCR und Detektion mittels Mikroarray-Analyse in einer Reaktion kombiniert (Liu,H.P. et al, 2006, TaqMan probe array for quantitative detection of DNA targets, Nucleic Acids Research, 34.). Dies erfolgt mittels Primermolekülen in der Flüssigphase und eines Arrays Zielsequenz-spezifischer, 3'-immobilisierter TaqMan-Sonden. In Anwesenheit der komplementären Zielsequenz wird eine TaqMan-Sonde durch eine Polymerase gespalten, wodurch ein unterdrücktes Fluoreszenzsignal wiederhergestellt wird. Das örtlich-aufgelöste Signal kann mittels optischer Vorrichtungen detektiert werden. Unter Verwendung dieser Plattform erfolgte der Nachweis von fünf verschiedenen Zielsequenzen in einer Probe. Dieser Ansatz ist potentiell auch für eine Echtzeit-Auswertung geeignet. Da die Primer frei in der Reaktionslösung vorliegen, kann die enzymatische Amplifizierung der Zielsequenz auch ausschließlich in der Flüssigphase erfolgen. Die Sonde stellt daher keinen notwendigen Interaktionspartner dar, und daher wird gegebenenfalls die Amplifikationsreaktion nicht durch ein Fluoreszenz-Signal an der Festphase abgebildet. Weitere Fluorophor-markierte Oligonukleotid-Sonden werden für den spezifischen Nachweis von Zielsequenzen eingesetzt. Die Verwendung von Molecular Beacons als Biosensoren für sensitive und spezifische Nukleinsäuredetektion ist in dem Stand der Technik beschrieben. Die Verwendung von Molecular Beacon- oder TaqMan-Sonden für Mikroarray-Analysen hat den Vorteil, dass die Zielsequenz nicht wie bei Oligonukleotid-Mikroarrays in einem gesonderten Reaktionsschritt, welcher vor oder nach dem Hybridisierungsereignisses durchgeführt wird, typischerweise mit Fluoreszenzfarbstoffen markiert werden muss. Falls ein Voramplifikationsschritt außerhalb des Mikroarrays benötigt wird, entstehen jedoch eine erhöhter Arbeitsaufwand und Kontaminationsgefahr. Da immobilisierte Molecular Beacon- oder TaqMan-Sonden typischerweise mindestens drei Modifikationen (Gruppe zur Immobilisierung, Fluoreszenzdonor und -akzeptor) besitzen, entstehen unmittelbar bei Änderungen der Zielsequenz oder des Array-Layouts hohe Folgekosten für die Herstellung und Immobilisierung neuer Sonden.

Lockett et al (Anal. Chem. 2007, 79, 6031-6036; *Molecular beacon-style hybridization assay for quantitative analysis of surface invasive cleavage reactions*) beschreiben einen Assay zur quantitativen SNP-Analyse, der die Hybridisierung einer Molecular Beacon-Sonde anwendet. Lu et al (Hum. Mutat., 2002, 19, 416-422; *A surface invasive cleavage assay for highly parallel SNP analysis*) beschreiben die Parallelisierung der SNP-Analyse mit Hilfe von strukturspezifischem *invasive cleavage.*

Weiterhin beschreiben Li et al (Analytical Chemistry, Bd. 78, Nr. 22, (2006-11-15), Seiten 7886-7890) ein Nachweisverfahren, das der vorliegenden Erfindung ähnliche, bei dem aber eine Mediator-Sonde (5'-UT primer) verwendet wird, die keine Spaltstelle enthält.

Ein Artikel von Nuovo et al (journal of histochemistry and cytochemistry, Bd. 47, Nr. 3, (1999-03-01), Seiten 273-279) beschreibt die Verwendung einer FRET-markierte Nachweissonde in Kombination mit einer Polymerase und dNTPs in einer Echtzeit-PCR.

Weiterhin ist im Stand der Technik ein sogenannter "Invader Assay" beschrieben, der von der Firma Hologic (früher: Third Wave Technology) entwickelt wurde. Der Assay kann unter isothermen Bedingungen die Anwesenheit einer Zielsequenz nachweisen. Das Verfahren benötigt im einfachsten Fall eine Struktur-spezifische Nuklease, zwei Zielsequenz-spezifische Oligonukleotide - ein "Invader"-Oligonukleotid und eine Sonde, welche über einen Fluoreszenzdonor und -akzeptor verfügt. Während der Nachweisereaktion hybridisieren die beiden Oligonukleotide an einen Strang der Zielsequenz, wobei sich der 3'-Terminus des "Invader"-Oligonukleotids und der 5'-Terminus der Sonde überlappen und eine Triplex-Struktur (ternärer Komplex) bilden. Die gebildete Triplex-Struktur stellt das Substrat für eine Struktur-spezifische Nuklease dar, die die Sonde spaltet (Primärreaktion) und dabei ein zuvor unterdrücktes Fluoreszenz-Signal wiederherstellt. In einer weiteren Ausführung des "Invader Assays" besitzt die Sonde keine Fluoreszenz-Modifikationen, die freigesetzte 5'-Region der Sonde kann jedoch eine nachfolgende Detektionsreaktion (Sekundärreaktion) aktivieren, indem es mit einem FRET-Detektionsmolekül interagiert und eine lokale Triplex-Struktur bildet. Nach Spaltung dieses Komplexes entsteht ein Fluoreszenzsignal. Bei der beschriebenen Reaktion findet eine Signalamplifizierung aber keine Zielsequenzamplifizierung statt. Der "Invader-Assay" kann unter anderem für die Detektion von Einzelnukleotid-Polymorphismen (SNP) verwendet werden.

Ein Artikel von Hall et al (PNAS, Bd. 97, Nr. 15, (2000-07-18), S. 8272-8277) beschreibt die Verwendung einer Oligonukleotidsonde bestehend aus einer 3'-Region welche komplementär an die Zielsequenz binden kann, einer Spaltstelle, gefolgt von einer 5'-Region, welche komplementär zu einer Nachweissonde und unabhängig von der Zielsequenz ist, sowie einer Nachweissonde in einem "Invader Assay".

Das von dem "Invader Assay" abgeleitete InPlex®-System kombiniert eine Voramplifikation der Zielsequenzen mit dem Invader® Assay. Dabei werden die Zielsequenzen zuerst mittels PCR amplifiziert und anschließend in eine Reaktionskartusche überführt und mehrere Stunden inkubiert (Detektionsreaktion). Die InvaderPlus®-Reaktion kombiniert eine PCR mit der "Invader"-Reaktion in einem Reaktionsgefäß, wobei eine Polymerase aus Thermus aquaticus und das Enzym Cleavase® verwendet werden. Dabei wird zunächst mittels PCR die Zielsequenz amplifiziert und anschließend die Polymerase bei 99 °C inaktiviert. Im nächsten Schritt wird der Reaktionsmix auf eine Temperatur abgekühlt, bei der sich ein Invader-Oligonukleotid und eine Sonde an die Zielsequenz anlagern. Diese Struktur wird von der Cleavase® erkannt, worauf eine Spaltungsreaktion mit Signalgenerierung ablaufen kann. Diese Endpunkt-Reaktion dauert typischerweise zwei Stunden. Nachteilig ist, dass eine hohe Anzahl an Zielmolekülen vorliegen muss, damit diese nachgewiesen werden können. Eine parallele Durchführung der Nachweismethode und einer Amplifikation ist nicht möglich. Daher ist die Sensitivität dieser Assays für viele Fragestellungen nicht ausreichend.

Im Stand der Technik sind mehrere Festphasen-basierte Ansätze bekannt, bei denen die Zielsequenz-spezifische Sonde örtlich-aufgelöst auf einer geeigneten Oberfläche immobilisiert ist und zur Detektion von SNPs in genomischer DNA verwendet werden kann. Dabei erfolgt die Detektion direkt über eine Fluoreszenzänderung beziehungsweise indirekt nach erfolgter Ligation eines Spaltungsproduktes mit einem Primer und universeller Rolling Circle Amplifikation sowie Markierung mit einem sequenzunspezifischen Fluoreszenzfarbstoff beziehungsweise Biotin-markierten Oligonukleotid und anschließender Inkubation mit Streptavidin-beschichteter Goldpartikel. Da bei diesem isothermen Verfahren keine Zielsequenz-Amplifizierung erfolgt, liegt die typische Inkubationsdauer einer Analyse bei bis zu 24 Stunden gefolgt von einer Fluoreszenzmessung.

Mikroarray-Analysen umfassen mehrere Arbeitsschritte, typischerweise Auswahl der Sequenz des immobilisierten Fängermoleküls, Probenvorbereitung und Amplifikation, Hybridisierung beziehungsweise Inkubation gefolgt von anschließenden Waschschritten sowie Signalmessung und Datenverarbeitung. Die im Stand der Technik bisher beschriebenen Mikroarrays basieren auf dem Prinzip der direkten Wechselwirkung zwischen Ziel- und immobilisierten Fängermolekül, daher muss ein geändertes Fängermolekül verwendet werden, sobald ein abweichendes Zielmolekül detektiert werden soll. Dies bedingt prinzipiell die Herstellung eines geänderten Arrays und ist ein wesentlicher Zeit- und Kostenfaktor. Aufgrund des aufwändigen Herstellungsprozesses, werden aus wirtschaftlichen Gründen größere Stückzahlen eines Sequenzlayouts gefertigt. Somit bieten Arbeiten mit Mikroarrays geringe Flexibilität in Bezug auf das nachzuweisende Zielmolekül, da ein geändertes Sequenzlayout hohe Folgekosten und Prozessierungsaufwand nach sich zieht. Ferner sind im Stand der Technik universelle Mikroarrays offenbart. Universelle Nukleinsäure-Mikroarrays, bei denen die Sequenz der immobilisierten Fängermoleküle unabhängig von der Zielsequenz ist, sind z. B. kommerziell erhältlich, beispielsweise Affymetrix "GeneChip Universal Tag Arrays". Diese Verfahren basieren auf einem universellen Mikroarray-Sequenzlayout, bei dem die immobilisierten Oligonukleotide (ZIP code beziehungsweise Universal Tag) unabhängig von der nachzuweisenden Sequenz sind und nicht mit dieser interferieren. Bei einer typischen Nachweisreaktion wird die Zielsequenz in der Regel in einem gesonderten Reaktionsgefäß amplifiziert und gegebenenfalls aufgereinigt. Anschließend erfolgt ein Ligationsschritt, bei dem in Anwesenheit der Zielsequenz eine spezifische Detektionssonde und eine Fluoreszenzsonde direkt nebeneinander an der Basenabfolge der Zielsequenz hybridisieren. Die Detektionssonde besitzt einen Zielsequenz-unspezifischen Überhang (complementary ZIP code, cZIP code), welcher als Adressierungssequenz dient und komplementär zu einer ZIP code-Sonde des Mikroarrays ist. Durch die Ligation entsteht ein Produkt aus Detektions- und Fluoreszenzsonde, welches für eine Hybridisierung auf einem ZIP code-Mikroarray verwendet wird. Ein Fluoreszenzsignal an einem spezifischen ZIP code-Spot ist ein indirekter Hinweis auf die Anwesenheit der Zielsequenz im Reaktionsmix. Als Festphase können auch kodierte Mikrokugeln (Beads) verwendet werden. Dabei wird jedem Fängermolekül ein eindeutig identifizierbares Bead zugewiesen, was durch eine definierte Nukleotidsequenz oder Färbung beziehungsweise Intensität erreicht wird. Durch diese Zuordnung kann in einem späteren Arbeitsschritt eine automatisierte Detektion des Beads sowie die parallele Analyse durchgeführt werden. Diese Methode besitzt den Vorteil, dass die Beads in der Flüssigphase homogenisiert vorliegen und die daraus resultierenden Reaktionskinetiken höher sind als bei vergleichbaren Flüssig-/Festphaseninteraktionen.

Ein als "cDNA-vermittelte Annealing, Selektion, Extension und Ligation" (DASL) betiteltes Verfahren zur Expressionsanalyse mittels universellen Mikroarrays ist ebenso im Stand der Technik beschrieben. Dabei wird zunächst RNA mittels Biotin-markierten Oligo-d(T)18 und Primermolekülen zufälliger Sequenz in cDNA umgeschrieben. Anschließend erfolgt ein Hybridisierungsschritt zweier lokus-spezifscher Oligonukleotide, wobei beide Olignukleotide universelle, lokus-unspezifische Sequenzüberhänge besitzen. Das 3'-terminale Oligonukleotid verfügt über eine Adressierungsequenz, welche zwischen dem sequenzspezifischen Bereich und dem unspezifischen Sequenzüberhang integriert ist. Die Adressierungsequenz ist komplementär zu einer definierten Sequenz auf dem universellen Mikroarray. In einer Verlängerungsreaktion wird der Bereich zwischen den Oligonukleotiden komplementär ergänzt und im nächsten Schritt ligiert. Unter Verwendung spezieller Oligonukleotide kann anschließend eine Vervielfältigung des Ligationsprodukts mittels PCR erfolgen, wobei durch Fluoreszenz-markierte Primer eine Markierung des amplifizierten Ligationsprodukt erfolgt. Im nächsten Schritt wird eine Hybridisierung an ein universelles Array beziehungsweise Bead durchgeführt. Das DASL-Verfahren bietet im Vergleich zu den vorherig beschriebenen universellen Mikroarrays den Vorteil, dass mehr Möglichkeiten zur Sequenzauswahl der zu ligierenden Oligonukleotide besteht, da eine Verlängerungsreaktion die fehlenden Nukleotide ergänzt. Allerdings muss durch diese Reaktion ein weiterer fehleranfälliger Arbeitsschritt in den Ablauf integriert werden. Durch die zahlreichen Prozessierungs- und Analyseschritte sind diese Methoden sehr zeitintensiv, zusätzlich besteht Kontaminationsgefahr, da amplifizierte Nukleinsäure-Fragmente zwischen Reaktionsgefäßen überführt werden müssen. Des Weiteren ist die Methode nur für Endpunkt-Messungen ausgelegt.

In der US 5,653,939 und US 6,099,803 ist ein Verfahren beschrieben, dass durch lokale Manipulation eines elektrischen Feldes den elektrophoretischen Transport geladener (Bio)Moleküle zu definierten Mikrolokationen (Spots) auf einem Mikroelektronikchip ermöglicht. Dieser "aktiver Mikroelektronik-Array", der unter der Bezeichnung NanoChip® kommerzialisiert wurde, besteht aus einem geordneten Aufbau individuell adressierbarer Elektroden. Durch Anlegen einer Spannung an eine oder mehrere Elektroden können geladene Analyte spezifisch an definierte Spots auf dem Halbleiter-Chip bewegt und konzentriert werden. Werden die Spots mit komplementären oder affinen Fängermolekül versehen, kann durch elektrophoretischen Transport ein Hybridisierungs- oder Affinitätsereignis innerhalb kurzer Zeitspannen stattfinden. Da Polarität und anliegende Spannung der Elektroden beliebig geändert werden kann, ermöglicht dies die Manipulation von Teilchen mit negativer (zum Beispiel Nukleinsäuren, teilweise Proteine) und positiver (zum Beispiel teilweise Proteine) Nettoladung. Durch Belegung mehrerer Spots mit verschiedenen Fängermolekülen kann eine Multiplex-Analyse mit einer aufgetragenen Probe durchgeführt werden. Durch Umpolen der Elektroden erfolgt ein Abstoßen der Zielmoleküle vom Fängermolekül, wodurch die Stärke der Wechselwirkung ermittelt (Selektivität) und unspezifische Bindungen minimiert werden ("elektrische Stringenz"). Die Immobilisierung der Fängermoleküle erfolgt typischerweise über Biotin-Modifikationen, die selektiv an das Streptavidin-modifizierte Polymer-Gel der Chipoberfläche binden. Die NanoChip-Geräteplattform eignet sich prinzipiell für DNA-Hybridisierungen, SNP- oder STR-Analysen sowie Zelltyp-Bestimmungen und on-chip SDA-Reaktionen. Die Detektion kann durch passive Hybridisierung markierter Sonden oder durch gängige Antikörper-Techniken erfolgen. Durch die angelegte Spannung entstehen im Bereich der Elektroden Elektrolyseprodukte (H+, OH-, H2, O2 und freie Radikale), welche das Zielmolekül schädigen können. Um diesen Effekt zu minimieren ist die Applikation einer separierenden Zwischenschicht in Form eines Polymer-Gels notwendig. Da diese Plattform ausschließlich Molekülinteraktionen zu detektieren vermag, muss das Probenmaterial in der Regel in einem vorgelagerten Schritt angereichert werden. Dies ist vor allem für Nukleinsäuren notwendig, die typischerweise mittels asymmetrischer PCR vervielfältigt werden. Die Geräteplattform besteht aus einer System- und Mikrochipkontrolleinheit, welche beispielsweise die Steuerung der angelegten Spannung und Fluidik regeln.

Ein weiterer universeller Ansatz zur Detektion von Zielmolekülen wurde von High-Throughput Genomics (HTG) vermarktet (z. B. US 2001/0034025 A1 oder US 6,238,869 B1). Grundlage für diesen Ansatz bildet ein universelles Array verschiedener Anker-Oligonukleotide, welche am 3'-Terminus an einer Festphase (Mikrotiterplatte) immobilisiert sind. Linker-Moleküle, die einen komplementären 5'-Abschnitt zu den beschriebenen Anker-Oligonukleotide und einen Zielmolekül-spezifischen 3'-Abschnitt aufweisen, ändern nach erfolgter Hybridisierung die Bindespezifität an dieser Arrayposition. Sollen mit dem neu konfigurierten Array Nukleinsäuren detektiert werden, besteht das Linker-Molekül aus einem Oligonukleotid, für den Nachweis von Proteinen besteht das Molekül typischerweise aus einem Oligonukleotid-Antikörper-Konjugat. Eine von HTG vorgestellte Ausführung zur Detektion von mRNA für Expressionsanalysen ermöglicht den parallelen Nachweis von 16 verschiedenen Loci pro Kavität einer handelsüblichen Mikrotiterplatte. Dazu werden beispielsweise Zellen in einem gesonderten Gefäß in Anwesenheit von mRNA-komplementären DNA-Sonden lysiert und denaturiert. Nach erfolgter Hybridisierung werden durch Zugabe einer S1 Nuklease einzelsträngige Nukleinsäuren (mRNA und Überschuss der DNA-Sonden) verdaut. Durch alkalische Hydrolyse wird der RNA-Anteil des Duplex degradiert, so dass nach Neutralisation stöchiometrische Mengen der DNA-Sonden im Ansatz vorhanden sind. Anschließend wird der Ansatz auf das Array transferiert, worauf sequentielle Hybridisierungen einer zur DNA-Sonde teilkomplementären Detektions-Sonde und ein dazu komplementäres DetektionsKonjugat, welches mit einer Peroxidase-Modifikation versehen ist, durchgeführt werden. Nach Zugabe geeigneter Substrate entsteht an der Position des Arrays ein lokales Chemilumeneszenz-Signal, an dem der beschriebene Hybridisierungskomplex erfolgreich gebildet werden konnte. Durch die zahlreichen Prozessierungsschritte ist das Verfahren arbeitsintensiv. Ein weiterer Nachteil besteht in der geringen Sensitivität des Assays, da keine Amplifikationsreaktion in das Assay-Protokoll integriert ist. Somit können beispielsweise sehr geringe Nukleinsäurekonzentrationen nicht ausreichend empfindlich nachgewiesen werden. Zusätzlich können die zahlreichen Hybridisierungsschritte zu Interferenzen und somit zu unerwünschten unspezifischen Reaktionen oder falsch-positiven Signalen führen.

Verfahren zum Proteinnachweis mittels Nukleinsäure-basierter Methoden sind in dem Stand der Technik offenbart. Dabei interagiert der Analyt mit einem immobilisierten Protein (zum Beispiel ein Antikörper) und wird anschließend mit einem Protein-Nukleinsäurekonjugat inkubiert. In einem folgenden Waschschritt werden nicht-gebundenen Moleküle entfernt. Das Konjugat enthält eine Nukleotidsequenz, die komplementär zu einem zirkulären DNA-Molekül ist, welches anschließend hinzugegeben wird. Durch Prozessierung mit einer geeigneten Polymerase wird der Primer verlängert und mittels RCA Konkatamere des DNA-Moleküls generiert. Nach Inkubation mit Gold- modifizierten Sonden oder Sequenz-unspezifischen Fluoreszenzfarbstoffen, werden die Bereiche eines Mikroarrays spezifisch markiert, an denen Fängermolekül und Detektionskonjugat an den Analyten gebunden haben. Mittels geeigneter Detektionsverfahren lassen sich Bindungsereignisse des Analyten somit ortsaufgelöst nachweisen. Nachteilig ist, dass zwei verschiedene Proteine beziehungsweise Protein-Nukleinsäure-Konjugate verwendet werden, welche direkt mit dem jeweiligen Analyten korrelieren. Die Verwendung von Proteinen und den davon abgeleiteten Verfahren ist aufgrund der Synthese ein großer Kostenfaktor. Einen prinzipiell ähnlichen Ansatz verfolgt die Firma Chimera Biotec mit der entwickelten Imperacer®-Technologie, wobei Antikörper-DNA-Chimäre, d.h. synthetische DNA-Fragmente, an die ein Antikörper gekoppelt wurde, verwendet werden. Bindet dieser Antikörper an das passende Antigen, kann nach anschließenden Waschschritten das gekoppelte DNA-Fragment mittels real-time PCR amplifiziert und detektiert werden.

In Zentrallaboratorien wird die labormedizinische Diagnostik mit Hilfe von Analysenautomaten durchgeführt und umfasst Bereiche der klinischen Chemie, medizinischen Mikrobiologie und medizinischen Immunbiologie sowie Transfusionsmedizin. Neben diesen Hochdurchsatz- Systemen gibt es deutlich kleinere Geräte, die eine patientennahe ("point of care") Multianalyt-Analyse zum Beispiel wichtiger Blutwerte oder Markerproteinen ermöglichen. Diese nutzen verschiedene Prinzipien der Detektion, wie Absorptionsmessung einer chromogenen Reaktion der Probe mit in der Reaktionskartusche vorgelagerten Reagenzien (z. B. Abaxis Piccolo® Xpress), Durchfluss-Immunoassay mittels einer mit Antikörper-markierten Membran und anschließender Markierung des Analyten mit Gold-modifizierten Detektionsantikörpern (z. B. Axis-Shield Afinion) oder eines linearen Teststreifens (z. B. Abbott Point-of-Care i-STAT®, Roche Cobas h232, BioSite® Triage®-System). Bei diesen Verfahren wird die Testflüssigkeit in eine spezielle Reaktionskartusche appliziert, in welcher ein saugfähiges Material die Probe durch Kapillarkräfte aufnimmt, transportiert und gegebenenfalls separiert. Typischerweise basieren die angebotenen Testkits auf Immunofluoreszenz-Technologie. An definierten Zonen sind auf dem Material Reagenzien vorgelagert (Detektionsantikörper) beziehungsweise immobilisiert (Fängerantikörper). Die Durchführung erfordert typischerweise keine Probenpräparation, so dass die Tests mit Vollblut, Blutplasma oder Urin durchgeführt werden können (gegebenenfalls mit internem Filter für Blutzellen und Partikel) und detektieren in den meisten Fällen mehrere Analyte innerhalb einer Reaktion. Die Ergebnisse sind innerhalb von etwa 15 Minuten verfügbar. Die zur Verfügung stehenden Testkits umfassen Marker für Herzkrankheiten, Pathogene sowie Stoffwechselprodukte von diversen Medikamenten. Die Vorteile der beschriebenen Ausführungen bestehen in der hohen Benutzerfreundlichkeit (einfaches Durchführungsprotokoll, keine Probenvorbereitung) und geringen Prozessierungsdauer. Nachteilig ist, dass diese vor allem in der klinischen Diagnostik eingesetzten Geräte nur mit proprietärem Verbrauchsmaterial kompatibel sind und die Verwendung auf klinisch-relevante Marker und Parameter (zum Beispiel kardiovaskuläre Erkrankungen) beschränkt ist. Außerdem ist der Einsatz sehr stark durch den Hersteller beschränkt, da nur für bestimmte Zielmoleküle Nachweise durchgeführt werden können, für die der Hersteller freigegebene Testkits anbietet.

Das beschriebene Teststreifen-Prinzip findet ebenfalls Anwendung in der Nukleinsäureanalytik. Dabei wird die nachzuweisende Nukleinsäure amplifiziert und auf einen Teststreifen appliziert, auf dem sich Zielsequenz-spezifische Fänger- und Nachweismoleküle befinden. Durch Kapillarkräfte passiert die Zielsequenz verschiedene Zonen auf dem Teststreifen und interagiert mit den verschiedenen komplementären beziehungsweise affinen Molekülen. Durch Ermittlung einer Nachweisbande (zum Beispiel mittels Gold-markierter Nachweismoleküle) an einer definierten Stelle des Teststreifens kann das Vorhandensein der Zielsequenz in der zu untersuchenden Probenlösung ermittelt werden. Ein universeller Ansatz zur Detektion beliebiger Nukleinsäurensequenzen ist in dem Stand der Technik beschrieben (z. B. Baeumner,A.J. et al, 2004, A universal nucleic acid sequence biosensor with nanomolar detection limits, Anal. Chem., 76, 888-894.). Dabei wird die Probenlösung amplifiziert und mit bifunktionalen Reporter-Sonden inkubiert. Ein Abschnitt der Sonde hybridisiert an die Zielsequenz, wohingegen ein anderer Abschnitt an Vesikel mit immobilisierten Oligonukleotiden komplementärer Sequenz bindet. Ein weiterer Abschnitt der amplifizierten Zielsequenz bindet an ein Biotin-modifiziertes Oligonukleotid. Nach Applizierung des Teststreifens in die Lösung, erfolgt ein gerichteter Transport des Hybridisierungskomplexes, welcher an einer Streptavidin-modifizierten Zone akkumuliert und detektiert werden kann. Die Teststreifen-basierte Nukleinsäureanalytik benötigt einen vorgeschalteten Amplifikationsschritt und die damit verbundene Handhabung von post-PCR-Produkten. Zusätzlich sind die Systeme durch einen geringen Multiparameter-Grad, geringe Sensitivität und eingeschränkte Quantifizierung limitiert.

In verschiedenen Bereichen der klinischen Analytik und *in vitro*-Diagnostik besitzen Multianalyt-Nachweise große Bedeutung, wozu im Folgenden einige Beispiele aufgeführt werden (ohne jedoch hierauf begrenzt zu sein): Beispielsweise ist für die Blutgruppenbestimmung nicht nur die AB0-Genotypisierung relevant, sondern auch die Erstellung des Humanen Neutrophilen Antigen-Profils (HNA), welches für Blut- und Gewebetransfusionen ermittelt werden muss. Auch die parallele Überprüfung von Blutspenderproben auf HIV-Varianten und Hepatitis-B beziehungsweise -C-Viren wird routinemäßig mit Immunoassays oder Nukleinsäure-basierten Techniken durchgeführt. Der spezifische Nachweis von Pathogenen bedingt die Bestimmung mehrerer genomischer Loci, um eine davon abgeleitete Diagnose nach kurzen Analysezeiten zu ermöglichen.

Die Aktivitätsbestimmung verschiedener Marker- und Kontrollgene erlaubt die Erstellung eines Expressionsprofils. Dies kann beispielsweise verwendet werden, um Onkogene, welche Zellteilung und -differenzierung beeinflussen und daher in enger Korrelation zu Krebserkrankungen stehen, zu identifizieren oder Vorhersagen über die Wirksamkeit bestimmter Medikamente abhängig vom Genotyp des Patienten zu treffen (Personalisierte Medizin). Auch häufig vertretene Erbkrankheiten lassen sich in der molekularbiologischen (Pränatal-)Diagnostik nachweisen, dazu zählen unter anderem Zystische Fibrose (Mukoviszidose), Phenylketonurie (Stoffwechselstörung) und Thalassämie (Degradation der Erythrozyten). Des Weiteren erlaubt die gemeinsame Detektion von Entzündungsmarkern, wie Procalcitonin oder Cytokine, einen Rückschluss auf den Schweregrad einer Infektion.

Zahlreiche diagnostische Fragestellung erfordern die Analyse mehrerer Zielmoleküle, genetischer Loci oder anderer Marker sowie interner Kontrollen beziehungsweise Referenzen, so dass Methoden, die nur die Bestimmung eines einzelnen Parameters pro Analyse erlauben, in der Regel wenig aussagekräftig sind. Werden zur Erfassung mehrerer Parameter verschiedene Einzelanalysen parallel durchgeführt, ist das andererseits unökonomisch: Die Probenlösung muss in mehrere homogene Reaktionsansätze aufgeteilt werden, in denen unterschiedliche Zielmoleküle nachgewiesen werden. Ein Nachteil dabei ist, dass durch das Aufteilen der Probenlösungen in "n" Aliquote die Stoffmenge in der Einzelreaktion um Faktor 1/n reduziert wird, wobei sich die Sensitivität der Nachweisreaktion entsprechend erniedrigt.

Ein weiterer Nachteil st, dass die Analytik von Proben mit niedriger Nukleinsäure- oder Protein-Konzentration ohne einen Analyt-abhängigen vorgeschalteten Aufkonzentrationsbeziehungsweise Amplifikationsschritt aufgrund der niedrigen Sensitivität mancher Detektionsverfahren nicht möglich ist. Bei der parallelen, Mikroarray-basierten Analyse niedrig konzentrierter Nukleinsäuren stellt die Voramplifikation einerseits einen zusätzlichen Arbeitsschritt dar und bringt ferner das Problem mit sich, dass abhängig von der initialen Stoffmenge und Reaktionseffizienz keine homogene Amplifikation stattfindet und quantitative Aussagen nur eingeschränkt möglich sind. Als nachteilig ist außerdem das Transferieren von amplifizierten Produkten zwischen verschiedenen Reaktionsgefäßen und Geräten anzusehen, da dies nicht nur mit einem zusätzlichen Arbeitsschritt verbunden ist, sondern zusätzlich ein Kontaminationsrisiko darstellt.

Mikroarray-Analysen beruhen auf der direkten Interaktion zwischen einem typischerweise örtlich aufgelösten, immobilisierten Fängermolekül (Bindungspartner oder Sonde) auf einem planaren Substrat und einem Zielmolekül, welches frei diffusiv in der Flüssigphase vorliegt. Ein Nachteil dieser Methoden ist, dass sie mehrere Arbeitsschritte umfassen: eine vorgeschaltete Amplifikation beziehungsweise Anreicherung, die Markierung der Zielmoleküle, um die Interaktion der Zielmoleküle mit dem Fängermolekül nachzuweisen, sowie mehrmalige Hybridisierungs- und Waschschritte. Des Weiteren tritt bei der beschriebenen direkten Abhängigkeit zwischen Fänger- und Zielmolekül das Problem auf, dass die Immobilisierung einer anderen Sonde erforderlich ist, wenn sich eine neue experimentelle Fragestellung ergibt, beispielsweise ein abweichender Genotyp eines Virus nachgewiesen werden soll.

Ein weiterer Nachteil ist, dass aufgrund des aufwändigen Herstellungsprozesses und den hohen Rüstkosten (Konfiguration des Arrays, Reinigung der Prozessierungselemente) die Fertigung eines Sequenzlayouts nur in größeren Stückzahlen wirtschaftlich (Skaleneffekte) ist. Diese eingeschränkte Flexibilität reduziert den Vorteil der hoch-parallelen und automatisierbaren Prozessierungen. Nachweise mit universellen Nukleinsäure-Mikroarrays, deren Sonden unabhängig vom nachzuweisenden Zielmolekül sind, können diesen Nachteil umgehen, allerdings ist der Arbeitsaufwand dieser Methoden sehr hoch, wodurch sich diese nicht durchgesetzt haben. Weiterhin ist neben den beschriebenen technischen Nachteilen der universellen Mikroarrays bislang kein biochemisches System aus dem Stand der Technik bekannt, welches kombinierte Multiplex-Analysen in verschiedenen Stoffklassen, wie zum Beispiel Nukleinsäuren und Proteine, erlaubt, so dass für unterschiedliche Nachweise verschiedene Verfahren oder Geräte verwendet werden müssen.

Die US 20020110826 betrifft die Verwendung von Festphasen-Hairpin-Oligonukleotiden. Durch eine Hybridisierungsreaktion von einer Target-Sequenz an ein Hairpin-Oligonukleotid wird eine enzymatische Reaktion begünstigt, welche zur Abspaltung des zur Targetsequenz komplementären Teils führt. Dadurch kann ein Label an das Hairpin-Oligonukleotid binden. Nachteilig ist, dass die Target-Sequenz zu dem Festphasen-Hairpin-Oligonukleotiden diffundieren muss. Die Diffusionsgeschwindigkeit wird dabei durch die Größe der Target-Sequenz verlangsamt.

Aufgabe der Erfindung war es demgemäß ein System, ein Mittel oder ein Verfahren bereitzustellen, dass die Detektion unterschiedlicher Biomoleküle ermöglicht, wobei es universell einsetzbar ist und nicht die Nachteile oder Mängel des Standes der Technik aufweist.

**Beschreibung**

Gelöst wird die Aufgabe durch Anspruch 1. Vorteilhafte Ausführungsformen ergeben sich aus den Unteransprüchen.

Die Erfindung betrifft ein Verfahren zur Detektion von mindestens einem Zielmolekül, umfassend folgende Schritte:

a) Bereitstellen einer Mediator-Sonde zur Detektion von mindestens einem Zielmolekül umfassend eine Sonden-Region und eine Mediator-Region, wobei die Mediator-Sonde ein Oligonukleotid ist und die Sonden-Region am 3'-Terminus und die Mediator-Region am 5'-Terminus des Oligonukleotides angeordnet ist, wobei zwischen den Regionen eine chemische, biologische und/oder physikalische Spaltstelle vorliegt und die Sonden-Region eine Affinität zu einem Vorlagemolekül und die Mediator-Region eine weitere Affinität zu einem Nachweismolekül aufweist,

b) Bereitstellen eines Nachweismoleküls, wobei das Nachweismolekül ein Oligonukleotid ist und mindestens folgende Regionen aufweist:

- eine erste Region an einem 5'-Terminus des Nachweismoleküls, die einen Fluoreszenzakzeptor oder einen Fluoreszenzdonor und/oder eine chemische Gruppe zur Bindung an eine Festphase und/oder eine chemische Schutzgruppe aufweist,

- eine zweite Region, die mit der Mediator-Region interagiert und
- eine dritte Region, die einen Fluoreszenzdonor oder einen Fluoreszenzakzeptor und/oder eine chemische Schutzgruppe aufweist,

c) Bindung der Sonden-Region der Mediator-Sonde an eine Sequenz des Vorlagemoleküls und/oder des Zielmoleküls,

d) Amplifikation des Vorlagemoleküls und/oder des Zielmoleküls mit Hilfe eine Hilfsmoleküls, bevorzugt durch einen Amplifikationsvorgang beinhaltend ein isothermales Verfahren und/oder Thermocycling-basiertes Verfahren und/oder durch eine PCR, besonders bevorzugt eine Real-time PCR,

e) Spaltung der Mediator-Sonde an der Spaltstelle während Schritt d) durch mindestens das Hilfsmolekül aus Schritt d) oder weitere Hilfsmoleküle, und

f) Bindung der abgespaltenen Mediator-Region der Mediator-Sonde an das Nachweismolekül,

g) Signaldetektion.

Im erfindungsgemäßen Verfahren verwendet wird eine Mediator-Sonde zur Detektion von mindestens einem Zielmolekül verwendet, wobei die Mediator-Sonde eine Sonden-Region und eine Mediator-Region umfasst, dadurch gekennzeichnet, dass die Mediator-Sonde ein Oligonukleotid ist und die Sonden-Region am 3'-Terminus und die Mediator-Region am 5'-Terminus des Oligonukleotides angeordnet ist, wobei zwischen den Regionen eine chemische, biologische und/oder physikalische Spaltstelle vorliegt und die Sonden-Region eine Affinität zu einem Vorlagemolekül und die Mediator-Region eine weitere Affinität zu einem Nachweismolekül aufweist, und wobei die Mediator-Sonde während eines Amplifikationsvorgangs des Vorlagemoleküls an der Spaltstelle gespalten wird und wobei eine Interaktion der abgespaltenen Mediator-Region mit dem Nachweismolekül ein detektierbares Signal auslöst.

Es war völlig überraschend, dass eine Mediator-Sonde zur Detektion an ein Zielmolekül und/oder ein Nachweismolekül bereitgestellt werden kann, die nicht die Nachteile oder Mängel der im Stand der Technik offenbarten Sonden oder Systeme aufweist. Besonders vorteilhaft ist, dass die Anwesenheit der freigesetzten Mediator-Region eine Nachweisreaktion auslöst. Die Kopplung zwischen Vorhandensein eines Zielmolekül und der Nachweisreaktion hängt dabei nur von den Eigenschaften der Mediator-Region beziehungsweise der Mediator-Sonde ab und erlaubt somit eine freie Kopplung zwischen einem beliebigen Zielmolekül und einer beliebigen Nachweisreaktion beziehungsweise Nachweismolekül.

Die Mediator-Sonde kennzeichnet also insbesondere ein Molekül, welches mindestens zwei funktionelle Regionen besitzt, die mit dem Zielmolekül und/oder Vorlagemolekül beziehungsweise dem Nachweismolekül interagieren können. Die Mediator-Sonde löst vorteilhafterweise bei Vorhandensein eines Zielmoleküls - gegebenenfalls unter Interaktion mit Hilfsmolekülen - eine Nachweisreaktion aus.

Die Sonden-Region ist bevorzugt komplementär zu einem Abschnitt des Vorlage und/oder Zielmoleküls. Die Sonden-Region der Mediator-Sonde bindet dabei an ein Vorlagemolekül, welches amplifiziert wird. Die Bindung erfolgt nur mit der Sonden-Region der Mediator-Sonde, da diese eine Affinität zu dem Vorlagemolekül aufweist. Die Mediator-Region weist keine Affinität zu dem Vorlagemolekül auf und verfügt auch nicht über einen komplementären Sequenzabschnitt. Daher bindet dieser Teil der Mediator-Sonde nicht an das Vorlagemolekül, sodass eine Flap-Struktur entsteht. Während der Amplifikationsreaktion wird die Mediator-Sonde an der Spaltstelle gespalten, so dass die Mediator-Region frei wird. Die Mediator-Region verfügt bevorzugt über einen komplementären Bereich zu einem Abschnitt eines Nachweismoleküls. Die Mediator-Region bindet an ein Nachweismolekül, wodurch ein detektierbares Signal ausgelöst wird. Durch das detektierbare Signal können so Rückschlüsse auf das Vorhandensein des Vorlagemoleküls geschlossen werden. Dabei kann das Vorlagemolekül selbst das zu detektierenden Zielmolekül sein, oder aber mit diesem assoziiert sein, so dass sich über das Vorlagemolekül Informationen über das Vorhandensein des Zielmoleküls generieren lassen.

Durch die Spaltung der Mediator-Sonde wird ein Mediator-Molekül freigesetzt, welches außer dem Nachweismolekül keinen Interaktionspartner besitzt. Im Vergleich zu konventionellen Nukleinsäure-basierten Ansätzen besteht somit kein Bedarf, wie im Falle von asymmetrischer PCR oder LATE-PCR, durch zusätzliche Optimierung der Primerverhältnisse ein Wiederanlagern (Re-annealing) des nachzuweisenden Stranges zu verhindern. Der Aufwand wird dadurch stark vermindert. Durch eine Länge von typischerweise 20 bis 25 Nukleotiden besitzt das Mediator-Molekül eine höhere Diffusionskonstante als durch Amplifikationsmechanismen generierte Nukleinsäurefragmente, welche zum Beispiel für eine Hybridisierungsreaktion generiert werden.

Der Begriff Nachweismolekül kennzeichnet im Sinne der Erfindung insbesondere ein Molekül, mit dem die Mediator-Region direkt oder indirekt interagieren kann und gegebenenfalls durch Prozessierung eine Nachweisreaktion (zum Beispiel Änderung eines Fluoreszenzsignals) hervorrufen kann.

Der Begriff Amplifikation bezeichnet insbesondere eine Vervielfältigung eines Nukleinsäuremoleküls.

Ein Hilfsmolekül bezeichnet insbesondere ein Molekül, welches in Anwesenheit des Zielmoleküls und/oder Vorlagemoleküls zur Zustandsänderung der Mediator-Sonde beiträgt. Es können verschiedene Hilfsmoleküle aus einer oder verschiedenen Stoffklassen verwendet werden, zum Beispiel Enzyme (Polymerasen), Nukleinsäuren (Oligonukleotide). Es ist bevorzugt, dass die, an das Ziel- oder Vorlagemolekül gebundene Sonden-Region durch ein Hilfsmolekül enzymatisch verlängert wird.

Es ist bevorzugt, dass die Mediator-Sonde 1 bis 70, bevorzugt 15 bis 60, besonders bevorzugt 35 bis 45 Nukleotide aufweist. Durch diese Größen werden besonders bevorzugte Ergebnisse erzielt, da die Mediator-Region nach Abspaltung aufgrund der geringen Größe mit einer hohen Diffusionsgeschwindigkeit zu dem Nachweismolekül diffundieren kann. Dadurch ist die Erfindung vorteilhaft gegenüber Ausführungsformen im Stand der Technik, in denen das Target selbst zu einem Nachweismolekül gelangen muss.

Besonders vorteilhaft ist dabei außerdem, dass die Mediator-Sonde aus einem Oligonukleotid besteht, welches ohne technisch komplexe Modifikationen wie zum Beispiel Fluoreszenzdonoren und/oder -akzeptoren kostengünstig synthetisiert werden kann.

Es ist bevorzugt, dass das Zielmolekül und/oder das Vorlagemolekül ein Biomolekül ist, ausgewählt aus der Gruppe umfassend DNA, RNA, Protein, Aptamer und/oder einer Kombination hieraus. Es kann auch bevorzugt sein, dass nur Teile eines Moleküls, zum Beispiel Erkennungssequenzen oder Epitope, nachgewiesen werden sollen und somit Zielmolekül im Sinne der Erfindung sind. Das Zielmolekül oder die Zielmoleküle liegen dabei bevorzugt in einer Probenlösung vor. Eine Kombination der Zielmoleküle kann im Sinne der Erfindung insbesondere auch als Gemisch bezeichnet werden. Überraschenderweise können Moleküle verschiedener Stoffklassen (zum Beispiel Protein und DNA oder DNA und RNA) einzeln oder parallel in einem Ansatz detektier werden, so dass ein universell einsetzbares Mittel zur Verfügung steht.

Ein Aptamer beschreibt im Sinne der Erfindung insbesondere ein Oligonukleotid, das mit Molekülen aus anderen Stoffklassen (zum Beispiel Proteinen) aufgrund ihrer strukturellen Eigenschaften interagieren beziehungsweise an diese binden kann. Ein Aptamer ist bevorzugt eine einzelsträngige Nukleinsäure, welche eine erhöhte Bindungsaffinität für andere Moleküle, zum Beispiel Proteine, zeigt. Ein bevorzugtes Aptamer besitzt zusätzlich terminale Regionen "Region i" und "Region ii", die miteinander interagieren können (im Sinne der Erfindung als geschlossene Form bezeichnet). Davon begrenzt sind zwei Regionen, wobei "Region iii" die Affinität für das Zielmolekül besitzt und die "Region iv" eine Bindesequenz für ein Primermolekül und eine Mediator-Sonde darstellt. "Region iv" erlaubt nur eine Bindung des Primers und der Mediator-Sonde, sofern "Region iii" mit dem Zielmolekül interagiert beziehungsweise assoziiert ist.

Es ist bevorzugt, dass das Zielmolekül gleichzeitig das Vorlagemolekül ist. Diese Ausführungsform wird zum Beispiel eingesetzt, wenn das Zielmolekül eine DNA-Sequenz ist. In diesem Fall wird kein weiteres Vorlagemolekül für die Amplifikationsreaktion gebraucht, so dass das Zielmolekül selbst amplifiziert wird.

Wenn das Zielmolekül nicht selbst amplifiziert werden kann, ist es vorteilhaft, dass ein Vorlagemolekül für die Amplifikationsreaktion eingesetzt wird, wobei das Stattfinden der Amplifikationsreaktion Rückschlüsse auf das Vorhandensein des Zielmoleküls zulassen muss, damit eine Detektion des Zielmoleküls erfolgen kann. Dies kann erfindungsgemäß auf unterschiedliche Weisen erfolgen. So kann es bevorzugt sein, dass das Vorlagemolekül erst durch Vorhandensein des Zielmoleküls gebildet wird, oder aber, dass das Vorlagemolekül mit dem Zielmolekül interagiert und dadurch seine Struktur verändert.

Es ist zum Beispiel bevorzugt, dass wenn es sich bei dem Zielmolekül um ein Protein handelt, das zugehörige Vorlagemolekül ein Aptamer ist. Das Aptamer weist eine Bindestelle für die Sonden-Region auf. Es ist dabei bevorzugt, dass das Aptamer an das Protein bindet und erst während dieser Bindung die Bindestelle für die Sonden-Region zugänglich wird. So wird verhindert, dass allein das Vorhandensein des Aptamers detektiert wird, ohne dass Rückschlüsse auf das Vorhandensein des Zielmoleküls (Protein) gezogen werden können. Erst bei Vorhandensein des Zielmoleküls kann das Aptamer an dieses binden und seine Bindestelle für die Sonden-Region wird bevorzugt durch einen Veränderung der Sekundärstruktur zugänglich. Die Sonden-Region der Mediator-Sonde kann nun an das Aptamer binden. Durch die Amplifikation des Aptamers wird die Mediator-Region von der Sonden-Region abgespalten und kann so an das Nachweismolekül binden. Über das Vorhandensein des Aptamers kann somit das Protein detektiert werden.

Wenn es sich bei dem zu detektierenden Zielmolekül um eine RNA-Sequenz handelt, ist bevorzugt, dass das Vorlagemolekül die entsprechenden cDNA ist. Diese wird bevorzugt durch eine reverse Transkriptase generiert. Die so hergestellte cDNA ist dann das Vorlagemolekül für die Amplifikation. Für die reverse Transkription kann die Verwendung von modifizierten Primern mit 5'-Sequenzüberhang vorteilhaft sein. Diese Ausführungsform ist vor allem dann vorteilhaft, wenn neben auch die originäre DNA auch vorhanden ist. Denn so wird sicher gestellt, dass die Mediator-Sonde nur an die cDNA bindet und nicht auch an den ursprünglichen DNA-Locus der Vorlage für die RNA war. Durch diese Ausführungsform wird es auch möglich Detektionen vorzunehmen, welche Aussagen über die Genexpression verschiedener Gene erlauben. Denn es können parallel DNA des Gens und die daraus transkribierte RNA (über die cDNA mit Primerüberhang) detektiert werden. Bei einem solchen Verfahren kommen zwei verschiedene Mediator-Sonden zum Einsatz, wobei eine der beiden Sonden-Regionen an einen Bereich bindet, der einen Teil des Primerüberhangs umfasst. Diese Sonden-Region kann daher nur an die cDNA und nicht an die originäre DNA binden.

Auch ein Komplex aus Aptamer und assoziiertem Zielmolekül oder ein Interaktionsprodukt aus zwei oder mehreren Stoffklassen, wie zum Beispiel Nukleinsäuren und Proteine können als Zielmolekül verwendet werden. Dabei lassen sich verschiedene Zielmoleküle in einem Reaktionsansatz einzeln oder parallel nachweisen. Es ist bevorzugt, dass die Mediator-Sonde aus einem Oligonukleotid oder einem entsprechenden Derivat besteht, das Zielmolekül eine Nukleinsäure, ein entsprechendes Derivat oder ein Molekül umfassend DNA, RNA, Protein, Aptamer und/oder ein Komplex aus Aptamer und assoziierter DNA, RNA oder Protein darstellt und das Nachweismolekül ein Oligonukleotid oder davon abgeleitetes Derivat ist.

In einer weiteren bevorzugten Ausführungsform des erfindungsgemäßen Verfahrens überlappen die Sonden-Region und die Mediator-Region der Mediatorsonde funktionell und/oder räumlich, bevorzugt mit einem Nukleotid.

Es ist bevorzugt, dass die Mediator-Sonde neben der Sonden-Region und der Mediator-Region eine weitere Region umfasst. Bei dieser Region handelt es sich bevorzugt um eine Lock-Region, welche komplementär oder affin zu der Mediator-Region ist. Die Lock-Region ist vorteilhafterweise an dem 3'-Ende der Sonden-Region angeordnet. Dabei können die drei Regionen sowohl funktionell als auch räumlich überlappend sein. Eine direkte oder indirekte Interaktion der Sonden-Region mit dem Vorlagemolekül erzeugt eine Veränderung der Mediator-Region und/oder der Lock-Region und verändert damit die Affinität und/oder die Interaktion zwischen der Mediator-Region und der Lock-Region beziehungsweise der kompletten Mediator-Sonde. Eine Mediator-Region umfassend eine Lock-Region ist vorteilhaft, da hierdurch ein zusätzlicher Schutz geschaffen wird, der verhindert, dass die Mediator-Region an das Vorlagemolekül bindet oder anlagert. Dadurch, dass die Mediator-Region und die Lock-Region eine Affinität zueinander aufweisen oder komplementär zueinander sind, kann die Mediator-Sonde bei Abwesenheit von Vorlage- und/oder Zielmolekülen eine Haarnadelstruktur ausbilden.

Es ist außerdem bevorzugt, dass die Mediator-Sonde an ihrem 3'-Ende eine chemische Schutzgruppe aufweist. Diese Ausführungsform ist vorteilhaft, da dadurch verhindert wird, dass eine enzymatisch-katalysierte Sequenzverlängerung der ungespaltenen Mediator-Sonde stattfindet. Die chemische Schutzgruppe kann ausgewählt sein aus der Gruppe umfassend Phosphatgruppe, Biotin, invertiertes Nukleotid, Nukleotide, die nicht komplementär zur Zielsequenz sind. Dem Fachmann sind weitere chemische Schutzgruppen bekannt, die eine Verlängerung einen Oligonukleotides, insbesondere des 3'-Terminus verhindern können.

Es ist bevorzugt, dass die Sonden-Region und die Mediator-Region unabhängig voneinander frei kombiniert werden können. So kann ein Nachweismolekül auch mit einem anderen Zielmolekül korreliert werden, indem die passende Mediator-Region mit einer beliebigen Sonden-Region verknüpft und synthetisiert wird. Dadurch wird eine besonders hohe Flexibilität im Einsatz der Mediator-Sonde im erfindungsgemäßen Verfahren erzielt.

In einer weiteren bevorzugten Ausführungsform betrifft die Erfindung ein Verfahren umfassend das Bereitstellen eines Systems umfassend eine Mediator-Sonde und ein Nachweismolekül, dadurch gekennzeichnet, dass das Nachweismolekül ein Oligonukleotid ist und mindestens folgende Regionen aufweist:

a. eine erste Region an einem 5'-Terminus des Nachweismoleküls, die einen Fluoreszenzakzeptor oder einen Fluoreszenzdonor und/oder eine chemische Gruppe zur Bindung an eine Festphase und/oder eine chemische Schutzgruppe aufweist,

b. eine zweite Region, die mit der Mediator-Region interagiert und

c. eine dritte Region, die einen Fluoreszenzdonor oder einen Fluoreszenzakzeptor und/oder eine chemische Schutzgruppe aufweist.

Es war völlig überraschend, dass ein System zur Verfügung gestellt werden konnte, das universell einsetzbar ist und insbesondere zur Minimierung der Kontaminationsfälle bei molekularbiologischen Nachweismethoden beiträgt. Es wird mittels einer biochemischen Reaktion eine Detektion verschiedener Moleküle, bevorzugt auf einem universellen Detektions-Chip mit standardisierten Nachweismolekülen erzielt. Dies wird insbesondere dadurch ermöglicht, dass die direkte, physikalische Wechselwirkung zwischen Zielmolekül und Nachweismolekül aufgehoben wird. Eine Mediator-Sonde fungiert als Übermittler (Informationsträger) zwischen Ziel- und Nachweismolekül. Durch Interaktion mit dem Zielmolekül oder dem Vorlagemolekül wird die Mediator-Sonde (in Anwesenheit von weiteren Hilfsmolekülen) bevorzugt gespalten und setzt ein aktiviertes Mediator-Molekül frei, welches eine Nachweisreaktion initiiert.

Das erfindungsgemäße Verfahren erlaubt die Zielmolekül-unabhängige Gestaltung des Nachweismoleküls. Somit können mit einem standardisierten Satz von Nachweismolekülen unterschiedliche Zielmoleküle in einer Probe nachgewiesen werden, wodurch die Reaktion kostengünstig durch Anpassen der Mediator-Sonde und Verwendung geeigneter Hilfsmoleküle (zum Beispiel Primer) oder Vorlagemoleküle (zum Beispiel Aptamere) an das jeweilige Zielmolekül adaptiert werden kann. Durch diese vorteilhafte Eigenschaft wird das im Stand der Technik beschriebene Problem der typischerweise direkten Korrelation zwischen Zielmolekül und immobilisierten Fängermolekül gelöst.

Die Mediator-Region liegt nach der Spaltung vorteilhafterweise diffusiv in der Reaktionslösung vor und kann mit der Region 2, der Mediator-Hybridisierungssequenz, des Nachweismoleküls interagieren. Das Nachweismolekül kann bevorzugt an eine Festphase gebunden oder auch frei in einer Lösung vorliegen.

Die genannten Nachweismoleküle wechselwirken nicht physikalisch mit den genannten Zielmolekülen. Eine Kopplung zwischen Zielmolekül und Nachweismolekül erfolgt nur indirekt über die entsprechenden Mediator-Sonden. Durch die Verwendung der Mediator-Sonde erfolgt eine freie Zuordenbarkeit eines Zielmoleküls zu einem beliebigen Nachweismolekül.

Wenn die Nachweismoleküle an einer Festphase immobilisiert sind, kann ein universeller Mikroarray oder Detektionsarray bereitgestellt werden. Die so hergestellten universellen Mikroarrays können unter definierten Lagerbedingungen über längere Zeit aufbewahrt werden, was vor allem gegenüber Protein-Arrays aus dem Stand der Technik ein deutlicher Vorteil ist. Dadurch ist eine Bevorratung unabhängig von geplanten Experimenten unkritisch.

Durch die Erfindung kann somit erstmals ein vom Zielmolekül unabhängiger, standardisierter Mikroarray zur Verfügung gestellt werden, der für verschiedene Multianalyt-Analysen verwendet werden kann, da die spezifische Flüssigphasenreaktion schnell und kostengünstig an das Zielmolekül anpasst werden kann.

Durch die Herstellung eines standardisierten Mikroarrays können daher verschiedene Experimente mit einer Reaktionskartusche ohne Vor- beziehungsweise Nachprozessierungsschritte durchgeführt werden, wodurch Kosten eingespart und hohe Stückzahlen produziert werden können (Skaleneffekte). Somit lassen sich mit einer Charge der standardisierten Reaktionskartusche verschiedene Nachweisreaktionen (zum Beispiel im Bereich der Routineuntersuchungen) durchführen.

Ein Mikroarray bezeichnet bevorzugt eine ortsaufgelöste, mindestens 1-dimensionale Anordnung von immobilisierten Fängermolekülen auf einer geeigneten (typischerweise planaren) Festphase. Alternative Methoden ermöglichen einen Festphasen-gestützten Ansatz unter Verwendung von Beads, die beispielsweise durch unterschiedliche Färbungen eine eindeutige Diskriminierung erlauben. An eine definierte Bead-Klasse kann ein bestimmtes Fängermolekül immobilisiert werden.

Ein Bead kennzeichnet bevorzugt Mikrokugeln mit einem Durchmesser von insbesondere 5 - 100 $\mu$m. Diese können gegebenenfalls auf der Oberfläche beziehungsweise im Inneren modifiziert beziehungsweise funktionalisiert vorliegen. Die Verwendung von Beads erlaubt die Bereitstellung großer Oberflächen in einem definierten Reaktionsvolumen.

Durch ein geeignetes Hilfsmolekül, zum Beispiel ein Enzym, insbesondere eine Polymerase, erfolgt eine Elongation der Mediator-Region, wobei Region 1 des Nachweismoleküls sequentiell abgebaut wird. Dabei wird das Nachweismolekül bevorzugt durch Abspaltung des 5'-Terminus und den damit assoziierten Fluoreszenzakzeptors Q, verändert und das zuvor unterdrückte Fluoreszenzsignal des Fluoreszenzdonors F wiederhergestellt. Wird durch die Abspaltung die Interaktion von Region 1 und Region 3 unterbunden, wird die Ausbildung der Sekundärstruktur aufgehoben. In diesem Fall kann das Mediator-Molekül durch das beschriebene Hilfsmolekül unter bestimmten Bedingungen bis zum neu entstehenden 5'-Terminus des Nachweismoleküls komplementär verlängert werden. Durch diese Elongation besitzt das verlängerte Mediator-Molekül einen Sequenzabschnitt, der komplementär zu Region 1 und Region 2 des Nachweismoleküls ist.

Das erfindungsgemäße Verfahren erlaubt den Nachweis verschiedener Zielmoleküle in einem geschlossenen Reaktionsgefäß, welches nach Prozessierung ohne Kontaminationsrisiko entsorgt werden kann. Dies stellt einen erheblichen Vorteil im Vergleich zu dem Stand der Technik dar.

Weiterhin vorteilhaft ist, dass das Nachweismolekül

d. eine vierte Region an einem 3'-Terminus des Nachweismoleküls aufweist, wobei die vierte Region eine chemische Gruppe zur Bindung an eine Festphase und/oder eine chemische Schutzgruppe umfasst.

Diese Variante ist vorteilhaft, da das Nachweismolekül so immobilisiert werden kann und zum Beispiel so ein Mikroarray hergestellt werden kann. Beispielhaft sind mögliche chemische Gruppen zur Immobilisierung eines Oligonukleotids aufgelistet. Die chemische Gruppe ist abhängig von der verwendenden Oberflächenchemie und evtl. benötigten Kopplungsmolekülen: -OH (Hydroxyl), -NH2 (Amino), -Ph (Phosphat), -Acrydite oder-Silan. Dem Fachmann sind Methoden bekannt, um Oligonukleotide an einer Oberfläche zu immobilisieren. Um insbesondere eine putative Immobilisierung des 5'-Terminus zu ermöglichen, weist das Nachweismolekül eine chemische Gruppe und/oder eine chemische Schutzgruppe auf.

Bevorzugt ist, dass die Haarnadel-Struktur ausgebildet ist, indem der 5'-Terminus des Nachweismoleküls mit einem internen Sequenzabschnitt komplementär hybridisiert und der 3'-Terminus des Nachweismoleküls einen ungepaarten Sequenzabschnitt umfasst. Nach Anlagerung der Mediator-Region an einen Sequenzbereich des ungepaarten 3'-Sequenzabschnitt wird die Mediator-Region bevorzugt durch eine Polymerase verlängert, wobei Nukleotide des 5'-Terminus der Haarnadelstruktur des Nachweismoleküls aufgrund der Nuklease-Aktivität der Polymerase entfernt werden. Nach Ausbildung dieser Struktur interagieren Fluoreszenzdonor F und Fluoreszenzakzeptor Q miteinander, wobei das Fluoreszenzsignal von F unterdrückt wird (Fluoreszenz-Resonanz-Energietransfer, FRET).

Es ist bevorzugt, dass die Mediator-Sonde und/oder das Nachweismolekül fluoreszenzmarkierte Nukleotide aufweisen. Insbesondere ist es bevorzugt, dass das Nachweismolekül mindestens eine Fluoreszenz-Modifikation am 5'-Terminus und/oder innerhalb der Haarnadelstruktur aufweist. Das Nachweismolekül weist eine oder mehrere Fluoreszenz-Modifikationen auf, die zu einem Fluoreszenz-Resonanz-Energie-Transfer befähigt sind und die nach der Abspaltung räumlich voneinander getrennt werden können, woraus eine Änderung des Fluoreszenzsignals detektiert werden kann.

Es können vorteilhafterweise sequenzspezifische oder -unspezifische, fluorogene und/oder chromogene Sonden oder Fluoreszenzfarbstoffe mit mindestens einer Region der Mediator-Sonde und/oder dem Nachweismolekül interagieren. Weiterhin kann vorteilhaft sein, wenn das Nachweismolekül über mindestens eine Fluoreszenz-Modifikationen am 5'-terminalen Bereich und/oder innerhalb der Haarnadelstruktur verfügt und nach der Reaktion mit der Mediator-Region mittels eines Hilfsmoleküls von dem Nachweismolekül die Fluoreszenz-Modifikationen abgespalten und/oder der 5'-Terminus der Haarnadelstruktur des Nachweismoleküls entfernt wird und eine Änderung des Fluoreszenzsignals am Nachweismolekül detektiert wird.

Es ist bevorzugt, dass das Nachweismolekül in Folge einer direkten oder indirekten Interaktion mit der Mediator-Region verändert wird, umfassend Änderung der Sekundärstruktur, Änderung in Fluoreszenz, Phosphoreszenz, Masse, Absorption, Streuung des Lichts, elektrischen Leitfähigkeit, enzymatische Aktivität und/oder der Affinität.

Bevorzugter Weise erfolgt dabei durch eine indirekte oder direkte Interaktion der Mediator-Region mit der zweiten Region des Nachweismoleküls eine physikalisch oder chemisch messbare Veränderung des Nachweismoleküls.

Es hat sich außerdem als vorteilhaft erwiesen, dass die an die zweite Region des Nachweismoleküls gebundene Mediator-Region durch mindestens ein Hilfsmolekül enzymatisch verlängert wird, wobei das Hilfsmolekül bevorzugt an den 3'-Terminus der gebundenen Mediator-Region bindet.

Das Hilfsmolekül ist ausgewählt ist aus der Gruppe umfassend Katalysatoren, Proteine, Nukleinsäuren, Naturstoffe, Enzyme, Enzym-Systeme, Zell-Lysate, Zell-Bestandteile, aus Zell-Bestandteilen abgeleitete Derivate und/oder synthetische Moleküle.

Es ist außerdem bevorzugt, dass das Hilfsmolekül ein Molekül aus einem Nukleinsäureamplifikationssystem und/oder einem Restriktionsenzymsystem ist.

Das Nachweismolekül kann außerdem in einer bevorzugten Ausführungsform über eine oder mehrere Fluoreszenz-Modifikationen am 5'-terminalen Bereich und/oder innerhalb der Haarnadelstruktur verfügen, wobei nach der Prozessierung der hybridisierten Mediator-Region mithilfe eines geeigneten Enzyms von diesem Nachweismolekül die 5'-terminalen Nukleotide abgespalten und eine Änderung des Fluoreszenzsignals am Nachweismolekül detektiert werden kann. Das freigesetzte Mediator-Molekül interagiert vorzugsweise mit mindestens einem frei in der Lösung vorliegenden oder an einer Festphase immobilisierten Nachweismolekülkomplex. Der Nachweismolekülkomplex kann über eine bis mehrere verschiedene oder gleichartige chemische Modifizierungen verfügen und nach der Interaktion mit der Mediator-Region ein detektierbares Signal generieren. Es ist bevorzugt, dass durch eine indirekte oder direkte Interaktion der Mediator-Region mit der zweiten Region des Nachweismoleküls eine physikalisch oder chemisch messbare Veränderung des Nachweismoleküls erfolgt. Diese Region kann erst nach der Spaltung der Mediator-Sonde ein Signal auslösen. Die Mediator-Region der Mediator-Sonde löst sofern sie noch assoziiert an die Mediator-Sonde vorliegt, bevorzugt kein Signal aus, weder direkt (zum Beispiel Hybridisierung) noch indirekt (zum Beispiel Prozessierung durch Polymerase), da sonst eine Zielmolekül-unabhängige Signalentstehung erfolgt. Diese messbare Veränderung kann auch durch die Hilfsmoleküle (z. B. Polymerase) hervorgehen.

Bei einer direkten Nachweisreaktion der Mediator-Region verändert diese direkt das Nachweismolekül. Bei einer indirekten Nachweisreaktion induziert die Mediator-Region durch Interaktion mit Nachweishilfsmolekülen, insbesondere einer Polymerase, welche die Mediator-Region verlängert, die Veränderung des Nachweismoleküls. Vorteilhafterweise verursacht die Mediator-Region während der Nachweisreaktion eine Veränderung des Nachweismoleküls und erfährt durch geeignete Nachweishilfsmoleküle selbst eine Änderung. Diese Ausführungsform ist vorteilhaft, das dadurch ein Mediator-Molekül, das in der Nachweisreaktion verändert wurde, von einem Mediator-Molekül, das unmittelbar aus einer Mediator-Sonden-Spaltung hervorgegangen ist, eindeutig unterschieden werden kann.

Ein Nachweishilfsmolekül beschreibt insbesondere ein Molekül, welches vorteilhafterweise mit der Mediator-Region und dem Nachweismolekül interagiert, wobei bevorzugt eine Nachweisreaktion ausgelöst wird. Es können verschiedene Nachweishilfsmoleküle aus einer oder verschiedenen Stoffklassen verwendet werden.

Die Nachweishilfsmoleküle sind bevorzugt aus verschiedenen Stoffklassen, wie Katalysatoren, Proteinen, Nukleinsäuren, Naturstoffen, Enzymen, Enzym-Systemen, Zell-Lysaten, Zell-Bestandteilen, daraus abgeleitete Derivaten oder synthetischen Molekülen oder einer Mischung aus verschiedenen Molekülen dieser Stoffklassen ausgewählt.

Ferner ist bevorzugt, dass das Nachweishilfsmolekül strukturspezifisch spaltet. Es ist dabei bevorzugt, dass die Hilfsmoleküle, Moleküle aus einem Nukleinsäureamplifikationssystem und/oder einem Restriktionsenzymsystem umfassen. Die Sonden-Region der Mediator-Sonde kann bevorzugt mit dem Zielmolekül und/oder Vorlagemolekül durch Basenpaarung interagieren und ein Hilfsmolekül kann die Mediator-Sonde spalten, wobei die Mediator-Region mit einem Nachweismolekül durch Basenpaarung interagiert und ein Nachweishilfsmolekül Bestandteile des Nachweismoleküls abspaltet, wobei diese Abspaltungsreaktion als indirekter Nachweis für das Zielmolekül dient. Der abgespaltene Bestandteil des Nachweismoleküls kann vorzugsweise einen Fluoreszenzdonor oder einen Fluoreszenzakzeptor darstellen.

Es kann jedoch auch bevorzugt sein, dass das Nachweishilfsmolekül sequenzspezifisch spaltet. Hierbei sind die Hilfsmoleküle ein Nukleinsäureamplifikationssystem und das Nachweishilfsmolekül ein Restriktionsenzymsystem oder eine Mischung aus einem Nukleinsäureamplifikationssystem und einem Restriktionsenzymsystem. Das Nachweismolekül ist ein Oligonukleotid oder ein Derivat und enthält die entsprechende Erkennungssequenz für das Restriktionsenzymsystem. Hierbei bindet die Mediator-Region an das Nachweismolekül an den komplementären Abschnitt und wird durch das Nukleinsäureamplifikationssystem verlängert. Der generierte Sequenzduplex enthält somit mindestens ein Erkennungssequenzmotiv des Restriktionsenzymsystems, welches diesen in mindestens zwei Teile spaltet. Nach der Spaltung des Sequenzduplex kann bevorzugt ein Signal, zum Beispiel die Änderung der Fluoreszenz oder der Masse, detektiert werden. Es kann jedoch auch vorteilhaft sein, wenn nach Spaltung des Sequenzduplex mindestens ein Spaltfragment eine Amplifikationsreaktion mit vorliegenden komplementären oder teilkomplementären Nukleinsäuresequenzen initiieren kann, wobei besagte Nukleinsäuresequenzen frei in Lösung oder an einer Festphase immobilisiert vorliegen können.

In einer bevorzugten Ausführungsform kann die Amplifikation durch Inkorporation von Fluoreszenz- oder anderweitig markierter Nukleotide oder durch Anlagerung sequenzspezifischer, fluorogener oder chromogener Sonden oder durch Anlagerung eines sequenzunspezifischen Fluoreszenzfarbstoffes detektiert werden. Die Amplifikationsprodukte können vorteilhafterweise direkt oder indirekt nachgewiesen werden, wobei die Detektion als indirekter Nachweis für das Zielmolekül dient.

Die freigesetzte Mediator-Region kann bevorzugt in Anwesenheit einer geeigneten Nukleinsäure durch eines oder mehrerer verschiedener Enzyme, wie zum Beispiel Polymerasen, eine enzymatisch-katalysierte Amplifikations- oder Polymerisationsreaktion initiieren. Dabei kann die geeignete Nukleinsäure einzel- oder doppelsträngig vorliegen und in der Nachweisreaktion zusätzlich ein gegenläufiger Primer (Reverse Primer) verwendet werden.

Es ist bevorzugt, dass das Nachweismolekül an eine Festphase gebunden oder frei in einer Lösung vorliegt.

Außerdem ist es vorteilhaft, dass das Nachweismolekül ein einzelsträngiges Nukleinsäuremolekül oder Nukleinsäurederivat ist, welches bevorzugt eine Haarnadel-Struktur aufweist. Es ist dabei vorteilhaft, dass die Haarnadel-Struktur so ausgebildet ist, dass der 5'-Terminus des Nachweismoleküls mit einem internen Sequenzabschnitt komplementär hybridisiert und der 3'-Terminus des Nachweismoleküls einen ungepaarten Sequenzabschnitt umfasst.

Das Nachweismolekül verfügt vorzugsweise über eine oder mehrere gleichartige oder verschiedene Modifikationen (beispielsweise abasische Nukleotide und/oder Phosphothioate und/oder funktionelle Gruppen wie Fluoreszenzfarbstoffe). Vorteilhafterweise erfolgt eine Änderung des Nachweismoleküls durch direkte oder indirekte Interaktion mit dem Mediator und kann eine oder mehrere Änderungen der Fluoreszenz, Phosphoreszenz, Masse, Absorption, Streuung des Lichts, elektrischen Leitfähigkeit, enzymatischen Aktivität oder der Affinität betreffen und dadurch physikalisch erfassbar werden. In Anwesenheit des Mediators kann das Nachweismolekül bevorzugt eine chemische Modifizierung, wie zum Beispiel Phosphorylierung, Dephosphorylierung, Amidierung, Anbindung oder Abspaltung einer chemischen Gruppe, Fluoreszenz-, Phosphoreszenz- oder Lumineszenzänderung erfahren.

Im Sinne der Erfindung beschreibt ein abasisches Nukleotid insbesondere einen DNA-Baustein, dessen Desoxyribose nicht an eine Base verknüpft ist und somit nur ein Phosphat-Zucker-Rückgrat darstellt. In DNA-Duplexen erfolgt an dieser Position keine Wasserstoffbrückenbildung. Diese Modifikation kann unter Verwendung von Tetrahydrofuran (THF) synthetisiert werden.

Es ist außerdem bevorzugt, dass das Nachweismolekül mindestens eine Fluoreszenz-Modifikationen am 5'-Terminus und/oder innerhalb der Haarnadelstruktur aufweist.

Die Erfindung betrifft ein Verfahren zur Detektion von mindestens einem Zielmolekül, umfassend folgende Schritte:

a) Bereitstellen einer Mediator-Sonde zur Detektion von mindestens einem Zielmolekül umfassend eine Sonden-Region und eine Mediator-Region, wobei die Mediator-Sonde ein Oligonukleotid ist und die Sonden-Region am 3'-Terminus und die Mediator-Region am 5'-Terminus des Oligonukleotides angeordnet ist, wobei zwischen den Regionen eine chemische, biologische und/oder physikalische Spaltstelle vorliegt und die Sonden-Region eine Affinität zu einem Vorlagemolekül und die Mediator-Region eine weitere Affinität zu einem Nachweismolekül aufweist,

b) Bereitstellen eines Nachweismoleküls, wobei das Nachweismolekül ein Oligonukleotid ist und mindestens folgende Regionen aufweist:

- eine erste Region an einem 5'-Terminus des Nachweismoleküls, die einen Fluoreszenzakzeptor oder einen Fluoreszenzdonor und/oder eine chemische Gruppe zur Bindung an eine Festphase und/oder eine chemische Schutzgruppe aufweist,
- eine zweite Region, die mit der Mediator-Region interagiert und
- eine dritte Region, die einen Fluoreszenzdonor oder einen Fluoreszenzakzeptor und/oder eine chemische Schutzgruppe aufweist,

c) Bindung der Sonden-Region der Mediator-Sonde an eine Sequenz des Vorlagemoleküls und/oder des Zielmoleküls,

d) Amplifikation des Vorlagemoleküls und/oder des Zielmoleküls mit Hilfe eines Hilfsmoleküls, bevorzugt durch einen Amplifikationsvorgang beinhaltend ein isothermales Verfahren und/oder Thermocycling-basiertes Verfahren und/oder durch eine PCR, besonders bevorzugt eine Real-time PCR,

e) Spaltung der Mediator-Sonde an der Spaltstelle während Schritt d) durch mindestens das Hilfsmolekül aus Schritt d) oder weitere Hilfsmoleküle, und

f) Bindung der abgespaltenen Mediator-Region der Mediator-Sonde an das Nachweismolekül,

g) Signaldetektion.

Dabei ist bevorzugt, dass die Spaltung der Mediator-Sonde eine Veränderung der physikalischen und/oder chemischen Eigenschaften mindestens einer Region der Mediator-Sonde bewirkt, ausgewählt aus der Gruppe umfassend molekulare Masse, enzymatische Aktivität, Bindungseigenschaften umfassend Affinität oder Avidität, chemische Reaktivität, Vorhandensein chemischer Gruppen, elektrischer Eigenschaft umfassend Leitfähigkeit, Polarisierbarkeit oder Ladung und/oder optischer Eigenschaft umfassend Absorption und Emission von Licht.

In einer besonders vorteilhaften Ausführung kann eine Nukleinsäure-basierte Amplifikation das Zielmolekül direkt beziehungsweise ein Vorlagemolekül (zum Beispiel Aptamer) indirekt amplifizieren, womit die Sensitivität erhöht wird. Dabei lassen sich Amplifikations- und Nachweisreaktion kombinieren und parallel durchführen. Damit hebt sich die Erfindung deutlich von der im Stand der Technik beschriebenen Invader®-Reaktion ab, bei welcher die Detektion mit linearer Signalamplifikation beziehungsweise eine konsekutive Amplifikation und Detektion durchgeführt wird.

Die Interaktion des Zielmoleküls und/oder Vorlagemoleküls mit der Mediator-Sonde führt bevorzugt zu einer direkten oder indirekten Spaltung der Mediator-Sonde, wobei bevorzugt folgende Spaltfragmente erzeugt werden:

- ein Fragment aus Mediator-Region und ein Fragment aus Sonden-Region oder
- Mediator-Region und ein Fragment aus Sonden-Region oder
- Mediator-Region und einen Bereich von Sonden-Region als ein zusammenhängendes Fragment und ein Fragment aus Sonden-Region oder
- ein Fragment aus Mediator-Region, Lock-Region beziehungsweise ein Fragment aus Lock-Region und ein Fragment aus Sonden-Region oder
- Mediator-Region sowie Lock-Region beziehungsweise ein Fragment aus Lock-Region und ein Fragment aus Sonden-Region oder
- Mediator-Region und einen Bereich von Sonden-Region als ein zusammenhängendes Fragment sowie Lock-Region beziehungsweise ein Fragment aus Lock-Region und ein Fragment aus Sonden-Region.

Es ist besonders bevorzugt, dass die abgespaltene Mediator-Region oder ein Fragment einhaltend einen Teil der Mediator-Region an die zweite Region des Nachweismoleküls bindet.

Eine Detektion des Mediator-Moleküls erfolgt mit Hilfe einer Nachweisreaktion. Der Reaktionsmechanismus kann parallel zu der beschriebenen Amplifikation des Zielmoleküls und/oder Vorlagemoleküls erfolgen.

Das erfindungsgemäße Verfahren ist vorteilhaft, da dadurch die Entwicklung einer neuartigen Flüssigphasenreaktion gelungen ist, die die Abhängigkeit von Ziel- und Nachweismolekül entkoppelt und in Verbindung mit einem standardisierten Mikroarrays nahezu jedes beliebige Zielmolekül detektiert. Durch die Kombination aus der neuartigen Flüssigphasenreaktion und einem universellen Mikroarray wird der parallele Nachweis verschiedener Zielmoleküle ermöglicht.
Es ist bevorzugt, dass die Amplifikation des Vorlagemoleküls und/oder Zielmoleküls durch eine PCR erfolgt, bevorzugt eine Real-time PCR.

PCR kennzeichnet insbesondere die Polymerase Kettenreaktion (Polymerase Chain Reaction), also ein Verfahren, bei welchem ein von Primermolekülen begrenzter Nukleinsäureabschnitt exponentiell amplifiziert wird. Dabei wird der Reaktionsansatz zyklisch erhitzt und abgekühlt.

Ein Primer beschreibt dabei bevorzugt ein Oligonukleotid, welches typischerweise komplementär zu einem Abschnitt der zu amplifizierenden Nukleinsäure ist und diesen Abschnitt begrenzt. Typischerweise werden zwei Primer, die ein Amplikon definieren, als Forward Primer und Reverse Primer bezeichnet. Da die Polymerisation vom 5'-Terminus in Richtung 3'-Terminus durchgeführt wird, benötigt ein Primer einen 3'-OH-Terminus, an die Polymerase die weiteren Nukleotide kovalent verknüpft.

Es ist besonders bevorzugt, die Mediator-Sonde im Sinne der Erfindung während einer Real-Time PCR einzusetzen. Die Mehrzahl der etablierten Techniken der Überwachung von Real-Time PCR Reaktionen machen von individuellen targetspezifischen fluorogenen Sonden Gebrauch. Dadurch werden die Kosten für die Synthese in die Höhe getrieben, was einen großen Nachteil dieser Systeme darstellt. Es bestand daher seit langem Interesse an einer universellen Methode die zum Überwachen von Amplifikationsreaktionen eingesetzt werden kann, welche Sequenzspezifität und niedrige Kosten verbindet. Die Erfindung konnte diese Aufgabe mit der im erfindungsgemäßen Verfahren verwendeten Mediator-Sonde lösen, welche bevorzugt während einer Amplifikationsreaktion, besonders bevorzugt einer Real-Time-PCR eingesetzt werden kann. Bei dieser Verwendung ist das Zielmolekül eine DNA Neukleinsäure, welche mit gewöhnlichen Olignukleotid-Primern und einer Polymerase amplifiziert wird. Die sequenzspezifische Detektion, bevorzugt Echt-Zeit-Detektion, wird durch die bifunktionale Mediator-Sonde realisiert, welche während der Amplifikation nach Interaktion mit der Zielsequenz gespalten wird. Die Spaltung wird durch die Polymerase katalysiert. Die Mediator-Region ist bevorzugt so gestaltet, dass diese nicht-komplementär zu der Zielsequenz ist. Die Mediator-Region diffundiert anschließend zu einem Nachweismolekül, welches entweder immobilisiert oder in Lösung vorliegt. Das Nachweismolekül ist eine abgeschlossene Einheit und targetunabhängig. Dadurch wird das Nachweismolekül universell einsetzbar und ist nicht an das konkrete Target gebunden. Somit können die Kosten für diese Art der Detektion erheblich gesenkt werden, da das Nachweismolekül nicht an jede Reaktion und an jedes Zielmolekül angepasst werden muss.

Bevorzugt weist das Nachweismolekül dabei eine Haarnadelstruktur auf und enthält ein Fluorophor und ein Quencher, welche bevorzugt in räumlicher Nähe zu einander angeordnet sind. Durch diese Anordnung wird ein besonders effizienter Nachweis mit FRET ermöglicht. An dem ungepaarten 3'-Ende enthält das Nachweismolekül außerdem eine Mediatorhybridisierungsstelle, welche komplementär zu der Mediator-Region ist.

FRET bezeichnet bevorzugt einen Fluoreszenz-Resonanz-Energie-Transfer, insbesondere die Energie-Übertragung von einem Donor- zu einem Akzeptor-Molekül.

Während der Real-Time-PCR finden Targetamplifikation und Detektion gleichzeitig in einer abgestimmten Reaktion statt. Während des Denaturierungsschritts der DNA-Vorlagen werden diese in zwei Einzelstränge geteilt. Während des Herunterkühlens zum Erreichen der Annealing-Temperatur hybridisieren sowohl die Primer als auch die Sonden-Region der Mediator-Sonde. Die 5'-Region (die Mediator-Region) bindet nicht an die Ziel-DNA. Dadurch entsteht eine Flap-Struktur. Während der Primerverlängerung wird die Mediator-Region in die Nukleasedomäne der Polymerase gezogen und so abgespalten. Der abgespaltene Bereich (die Mediator-Region) weist nun eine 3'-OH Gruppe auf. Die Sonden-Region wird anschließend während der Verlängerung der Primer abgebaut. Bei jeder Duplikation eines Targets wird so eine Mediator-Region freigesetzt. Die Mediator-Regionen diffundieren zu den Nachweismolekülen und hybridisieren mit deren Mediator-Hybridisierungsstelle. Es ist bevorzugt, dass die Polymerase das 3'-Ende der Mediator-Region verlängert, was zu dem fluoreszierenden Dequenching führt. Hierbei sind zwei unterschiedliche Signalwege bevorzugt. Durch die 5'-Nukleaseaktivität der Polymerase wird der 5'-Terminus des Nachweismoleküls abgebaut und der Quencherrest wird abgespalten. Es ist aber auch bevorzugt, dass die Polymerase den Stammduplex des Nachweismoleküls destabilisiert und die Haarnadelstruktur entfaltet wird ohne dass der 5'-Terminus abgebaut wird. Beide Wege führen letztlich zu dem Dequenching des Fluorophors. Beide Wege können parallel stattfinden, da beispielsweise die Taq-Polymerase bekannt für unterschiedliche Exonukleaseaktivitäten ist. Ein Vorteil der Erfindung ist, dass sich die Fluoreszenzemission mit jedem Amplifikationszyklus sukzessiv ansammelt. Dadurch kann ein Nachweis auch bei besonders geringen Ausgangsmengen an Zielmolekülen erfolgen.

Die Real-Time-PCR mit dem Einsatz der Mediator-Sonde als Detektionsmittel ist vorteilhaft gegenüber Systemen aus dem Stand der Technik (zum Beispiel SISAR oder Invadersystem). Durch die Kopplung des Nachweises an eine Amplifikationsreaktion wird eine sensitive Detektion des Zielmoleküls ermöglicht, wodurch die Analysen an Genauigkeit gewinnen. Das erfindungsgemäße Verfahren benötigt beides, Polymerisation und Taq-Polymeraseaktivität. Falsch positive Resultate werden ausgeschlossen, da Fehlamplifikationen durch Falschbindungen der Primer nicht zum Abspalten der Mediator-Region führen, da keine komplementäre Region für die Sondenregion vorhanden ist, sodass diese auch nicht binden kann. Außerdem kann die Mediator-Sonde in einem Multiplex-PCR-System und/oder einem Duplex-PCR-System verwendet werden, in welchen dann über mehrere verschiedene Nachweismoleküle mit mehreren verschiedenen Fluorophoren gearbeitet werden kann. Dies ist ein großer Vorteil im Vergleich zu den Systemen im Stand der Technik, da so eine Vielzahl von Zielmolekülen parallel nachgewiesen werden kann. So kann ein erfindungsgemäßes Verfahren umfassend ein Multiplex-PCR-System zum Beispiel in der Diagnostik verwendet werden und hier Nachweisprozesse beschleunigen und vereinfachen, wodurch letztlich Arbeitsaufwand, Material und Kosten eingespart werden können.

Bei Multiplex-Ansätzen können verschiedene Gruppen multipler Analyten mit der gleichen Gruppe an verschiedenen Nachweismolekülen detektiert werden. Vorteilhaft ist dabei, dass die Multiplex-Ansätze mit der gleichen Gruppe an verschiedenen Nachweismolekülen durchgeführt werden können. Dies ist im Stand der Technik bisher nicht möglich gewesen.

Ein weiterer großer Vorteil der Erfindung ist die Entkoppelung der Amplifikationsreaktion von der Detektion. Dadurch wird es möglich ein standardisiertes Nachweismolekül zu verwenden, welches somit in großen Mengen hergestellt werden kann und daher die Produktionskosten minimiert.

Außerdem ist bevorzugt, dass das Nachweismolekül durch eine Interaktion mit der Mediator-Region durch mindestens ein Nachweishilfsmoleküle verändert wird, umfassend Spaltung, Verdau, Strangverdoppelung, interne Hybridisierung, Phosphorylierung, Dephosphorylierung, Amidierung, Anbindung oder Abspaltung einer chemischen Gruppe, Fluoreszenz-, Phosphoreszenz- oder Lumineszenzänderung. Besonders bevorzugt ist dabei, dass das Nachweismolekül über mindestens eine Fluoreszenz-Modifikationen am 5'-terminalen Bereich und/oder innerhalb der Haarnadelstruktur verfügt und nach der Reaktion mit der Mediator-Region mittels eines Nachweishilfsmoleküls von dem Nachweismolekül die Fluoreszenz-Modifikationen abgespalten und/oder der 5'-Terminus der Haarnadelstruktur des Nachweismoleküls entfernt wird und eine Änderung des Fluoreszenzsignals am Nachweismolekül detektiert wird.

Es ist außerdem ein Verfahren bevorzugt, in dem mindestens ein Zielmolekül eine RNA ist. Hierbei wird die RNA zunächst in cDNA umgeschrieben, bevorzugt durch eine reverse Transkription. Die so entstandene cDNA stellt nun das Vorlagemolekül dar an welches die Sonden-Region der Meddiator-Sonde bindet und welche amplifiziert wird. Es ist besonders vorteilhaft, dass durch die Transkription in cDNA das RNA-Zielmolekül auch amplifiziert werden kann, da somit nur geringe Mengen an RNA benötigt werden, um eine Detektion zu ermöglichen.

Es ist außerdem bevorzugt, dass das bei der Umschreibereaktion, bevorzugt der reversen Transkription ein Primer mit Sequenzüberhang eingesetzt wird. Dies ermöglicht, dass eine Mediator-Sonde eingesetzt werden kann, die an die cDNA jedoch nicht an die originäre DNA des Gens binden kann. Hierfür wird eine Mediator-Sonde eingesetzt, deren Sonden-Region an einen Bereich umfassend cDNA und Überhangsequenz bindet. Da die originäre DNA keine Überhangsequenz aufweist, kann die Mediator-Sonde so zwischen DNA und cDNA unterscheiden. So wird sichergestellt, dass nur das Vorhandensein der DNA detektiert wird und nicht die DNA ein entsprechendes Signal auslösen kann.

Außerdem kann dieses Verfahren dazu eingesetzt werden, um RNA (über die entsprechende cDNA mit Überhangsequenz) und DNA des gleichen Gen parallel zu detektieren. Hierfür werden bevorzugt zwei unterschiedliche Nachweismoleküle und zwei unterschiedliche Mediator-Sonden eingesetzt.

Werden Proben aus eukaryotischen Zellen untersucht, können für die Differenzierung zwischen RNA und DNA des gleichen Gens auch Intron/Exon-Sequenzen verwendet werden. So kann die Sonden-Region für die Bindung an die cDNA so gewählt sein, dass diese einen Bereich aus zwei transkribierten Exons abdeckt. Da in der DNA des Gens diese Bereiche durch ein Intron getrennt vorliegen, kann die Mediator-Region nicht auch an die DNA, sondern nur an die cDNA binden.

In einem weiteren bevorzugten Verfahren ist mindestens ein Zielmolekül ein Peptid oder ein Protein. Das Vorlagemolekül ist bevorzugt ein Aptamer ist und wobei das Aptamer an das Peptid oder das Protein bindet und dabei die Bindestelle für die Sonden-Region der Mediator-Sonde zugänglich wird. Es ist bevorzugt, dass das Aptamer allein keine zugängliche Bindestelle für die Sonden-Region aufweist. Die Bindungsstelle wird erst frei und zugänglich, wenn das Aptamer mit dem Zielmolekül (Peptid oder Protein) interagiert hat. Die geschieht bevorzugt durch eine Veränderung der Sekundärstruktur. Die Sonden-Region der Mediator-Sonde kann nun an das Aptamer binden. Gleichzeitig wird steht das Aptamer für die Amplifikationsreaktion zur Verfügung. Durch die Amplifikation des Aptamers wird die Mediator-Region von der Sonden-Region abgespalten und kann so an das Nachweismolekül binden. Über das Vorhandensein des Aptamers kann somit das Protein detektiert werden. Da das Aptamer amplifiziert wird, wird das Signal außerdem verstärkt, so dass auch eine sehr geringe Zahl an Peptiden und/oder Proteinen detektiert werden kann.

Es kann außerdem bevorzugt sein, mehrere Zielmoleküle gleichzeitig zu detektieren, beispielsweise DNA und Protein oder Protein und RNA.

Abhängig von den Reaktionsbedingungen ermöglicht die Erfindung über eine Multianalyt-Analyse die parallele Detektion verschiedener Moleküle und Molekülklassen, wie zum Beispiel Proteine und Nukleinsäuren in einem Arbeitsschritt, womit ein kombiniertes DNA-RNA-Protein-Profil einer Probe erstellt werden kann

Das erfindungsgemäße Verfahren kann daher auch zur Detektion von einem oder mehreren gleichartigen oder verschiedenen Biomolekülen in einem Gemisch verwendet werden.

Bevorzugt findet dies unter Verwendung der Aktivität eines Restriktionsenzyms statt, dadurch gekennzeichnet, dass das Nachweismolekül über eine chemische Schutzgruppe am 3'-terminalen Bereich verfügt, die nach der Reaktion mit der Mediator-Region mittels eines Hilfsmoleküls von dem Nachweismolekül abgespalten wird und eine 3'-terminale OH-Gruppe generiert wird.

Es ist außerdem bevorzugt, dass das erfindungsgemäße Verfahren für eine Multiplex-Analyse verwendet wird. Eine Multiplex-Analyse beschreibt insbesondere einen parallelen Nachweis mehrerer Zielmoleküle in einem Reaktionsansatz.

Mit einem standardisierten Array-Layout und Adaptierung der Mediator-Sonden kann vorteilhafterweise besonders kostengünstig eine Vielzahl an verschiedenen Zielmolekülen detektiert werden, die den Multiplexing-Grad einer im Stand der Technik beschriebenen PCR-Reaktion potentiell um Größenordnungen übersteigt. Der Multiplex-Grad dieses Verfahrens wird in der Praxis nicht ausgeschöpft werden. Eine Mediator-Region, beispielsweise bestehend aus einem Nukleinsäuremolekül mit einer Länge von 20 Nukleotiden kann rechnerisch 4^20 (~ 1*10^12) mögliche verschiedene Nukleotidabfolgen annehmen.

Bei direkten Interaktionen zwischen Zielmolekül und/oder Vorlagemolekül und Mediator-Sonde erzeugt die Anwesenheit des Zielmoleküls und/oder Vorlagemoleküls aus der Mediator-Sonde die aktivierte Mediator-Region. Bei indirekten Interaktionen induziert das Zielmolekül und/oder Vorlagemolekül, insbesondere eine Nukleinsäure, eine Wechselwirkung mit der Mediator-Sonde und einem Hilfsmolekül, insbesondere einem Oligonukleotid, welches strukturell nicht mit der Mediator-Sonde interagiert. Das beschriebene Hilfsmolekül fungiert als Primer und wird dabei durch ein andersartiges Hilfsmolekül, insbesondere eine geeignete Polymerase, verlängert, worauf die Mediator-Sonde nach struktureller Interaktion mit der Polymerase in Mediator-Region und Sonden-Region gespalten wird. Die Mediator-Region entsteht aus der Mediator-Sonde somit in Anwesenheit des Zielmoleküls. Diese Hilfsmoleküle sind vorzugsweise ausgewählt aus der Gruppe umfassen Katalysatoren, Proteine, Nukleinsäuren, Naturstoffe, Enzyme, Enzym-Systeme, Zell-Lysate, Zell-Bestandteile, daraus abgeleitete Derivate oder synthetische Moleküle oder eine Mischung hieraus.

Es war überraschend, dass die Spaltung der Mediator-Sonde eine Veränderung der physikalischen und/oder chemischen Eigenschaften mindestens einer Region der Mediator-Sonde bewirkt, ausgewählt aus der Gruppe umfassend molekulare Masse (nach Spaltung der Mediator-Sonde unterscheiden sich die Spaltfragmente von der Mediator-Sonde), enzymatische Aktivität (die Mediator-Sonde ändert vorteilhafterweise ihre Eigenschaft eine Nachweisreaktion initiieren zu können, durch das neue Vorhandensein eines freien 3' Endes), Bindungseigenschaften umfassend Affinität oder Avidität, chemische Reaktivität (nach Spaltung der Mediator-Sonde besitzt das Spaltfragment, welches die Mediator-Region der Mediator-Sonde enthält, eine Hydroxyl-Gruppe am 3'-Ende, welches durch ein Hilfsmolekül (zum Beispiel eine Polymerase) verlängert werden kann. Dies ist für die ungespaltene Sonde nicht möglich), Vorhandensein chemischer Gruppen, elektrischer Eigenschaft umfassend Leitfähigkeit, Polarisierbarkeit oder Ladung und/oder optischer Eigenschaft umfassend Absorption und Emission von Licht (sofern die Mediator-Sonde mit mindestens einem Fluoreszenzfarbstoff markiert ist, kann bevorzugt nach der Spaltung ein verändertes Fluoreszenzsignal detektiert werden).

In einer bevorzugten Ausführung erfolgt eine serielle Interaktion der Mediator-Region mit mehreren gleichartigen Nachweismolekülen, womit eine Signalverstärkung erfolgt. Dies erhöht die Sensitivität der Nachweisreaktion. Bei dieser Ausführung werden vorteilhafterweise modifizierte Nukleotide für die Synthese des Nachweismoleküls verwendet, die nur einen begrenzten 5'-Terminusabbau während der Nachweisreaktion erlauben. Dies kann durch den Einbau einer oder mehrerer Phosphothioat-Modifikationen (PTO) beispielsweise am vorletzten Nukleotid des 5'-Terminus während der Synthese des Moleküls erfolgen. Die Position der PTO-Modifikation befindet sich bevorzugt in Region 1, besonders bevorzugt zwischen Fluoreszenzdonor F und Fluoreszenzakzeptor Q. Eine Darstellung bevorzugter Positionen modifizierter Nukleotide ist in Fig. 6 dargestellt. Bevorzugte Nachweishilfsmoleküle können PTO-Verbindungen nicht oder nur mit geringer Effizienz spalten, worauf der 5'-Terminus (Region 1) nicht über dieses Nukleotid hinaus abgebaut wird. Folglich wird auch das Mediator-Molekül nicht weiter verlängert. Durch geeignetes Einstellen der Reaktionsbedingung können das Nachweishilfsmolekül und das Mediator-Molekül vom Nachweismolekül dissoziieren und für die Aktivierung eines weiteren Nachweismoleküls wieder zur Verfügung stehen.

In einer besonders bevorzugten Ausführung kann ein Mediator-Molekül in jedem Reaktionszyklus beziehungsweise zeitlichen Abschnitt der Reaktion mit mehreren identischen spezifischen Nachweismolekülen interagieren und eine Signal-generierende Nachweisreaktion auslösen. Da ein akkumulativer Effekt durch Amplifizierung des Zielmoleküls erfolgt, wird die Sensitivität der Reaktion deutlich.

Es ist außerdem bevorzugt, dass die Detektion unter Verwendung geeigneter Vorrichtungen in Echtzeit erfolgen kann, beziehungsweise eine Endpunkt-Messung ermittelt werden kann. In beiden Fällen benötigt die erfindungsgemäße Ausführung vorteilhafterweise keine Nachprozessierung, wie zum Beispiel Wasch- oder Inkubationsschritte.

In einer weiteren bevorzugten Ausführung, die mit der oben beschriebenen PTO-Modifikation kompatibel, aber nicht auf eine kombinierte Anwendung beschränkt ist, wird in Region c ein abasisches Nukleotid (Tetrahydrofuran-Modifkation, THF) eingebaut, welches keine Wasserstoffbrücken mit einem komplementären Nukleotid ausbilden kann. Dadurch wird die Verlängerungsreaktion der Mediator-Region an der gegenüberliegenden Position des abasischen Nukleotids unterbunden, worauf das Nachweishilfsmolekül und die verlängerte Mediator-Region vom Nachweismolekül dissoziieren (siehe Fig. 7). Die Verwendung einer der bevorzugten Modifikationen ermöglicht die serielle Interaktion eines Mediator-Moleküls mit mehreren Nachweismolekülen, was im Sinne der Erfindung insbesondere als Mediator-Wiederverwendung bezeichnet werden kann. Die Mediator-Region wird bei der ersten Interaktion mit einem Nachweismolekül und erfolgter Prozessierung mittels eines Nachweishilfsmoleküls verlängert und kann auch in diesem verlängerten Zustand mit weiteren Nachweismolekülen interagieren und eine Degradation des 5'-Terminus -und somit auch des Fluoreszenzakzeptors Q - mit Hilfe eines geeigneten Nachweishilfsmoleküls (zum Beispiel Polymerase mit 5'-Nukleaseaktivität) ermöglichen. Dieser Reaktionsmechanismus erlaubt eine Signalamplifikation, da unabhängig von einer Zielmolekülamplifikation ein Signal generiert und verstärkt wird. Wird das Zielmolekül mithilfe geeigneter Hilfsmoleküle amplifiziert und dieser Vorgang an eine Mediator-Sonden-Spaltung gekoppelt, tritt eine Mediator-Akkumulation auf, die eine Zielmolekül-Amplifikation mit einer Signalverstärkung kombiniert und die Nachweisgrenze der Detektionsreaktion um mehrere Größenordnungen verringert. Dies war völlig überraschend und ist nicht im Stand der Technik beschrieben. Außerdem stellt es eine Abkehr vom technisch Üblichen dar. Die Reaktionsbedingungen können vorteilhafterweise nach Ablauf dieses Prozesses, zum Beispiel durch Erhöhen der Reaktionstemperatur, geändert werden, so dass das Enzym, zum Beispiel die Polymerase und die verlängerte Mediator-Region vom Nachweismolekül dissoziiert. Dem Fachmann ist bekannt, dass die Reaktionsbedingungen zyklisch verändert werden können, so dass ein Mediator-Molekül in einer besonders bevorzugten Ausgestaltung der Erfindung mit mehreren gleichartigen Nachweismolekülen interagieren kann. Dadurch entsteht überraschenderweise eine Signalamplifizierung, welche die Sensitivität der Reaktion deutlich erhöht.

Die Nachweisreaktion ist bevorzugt derartig ausgelegt, dass im Gegensatz zur Mediator-Region die ungespaltene Mediator-Sonde keine Signal-generierende Reaktion auslöst und somit keine falsch-positiven Ergebnisse erzeugt werden. Die Mediator-Region, welche aus der Mediator-Sonden-Spaltung hervorgeht, besitzt einen 3'-OH-Terminus, der für eine Polymerase-vermittelte Verlängerungsreaktion besonders vorteilhaft ist. Tritt diese Spaltung nicht auf, ist eine Mediator-Elongation nicht möglich, da die Mediator-Sequenz kovalent mit der Hybridisierungssequenz der Mediator-Sonde verknüpft ist (siehe Fig. 8). Zusätzlich kann eine unspezifische Verlängerung des 3'-Terminus der Mediator-Sonde beispielsweise mit einer Phosphatgruppe oder anderen chemischen Gruppen verhindert werden.

Durch das Interaktionsereignis der Mediator-Region mit dem Nachweismolekül entsteht bevorzugt ein lokales, detektierbares (Fluoreszenz-) Signal. Werden ausreichend viele Nachweismoleküle durch die bevorzugte Mediatorverlängerung mit resultierender Abspaltung des 5'-Terminus aktiviert, wird das Signal verstärkt und kann mittels geeigneter (insbesondere optischer) Detektionsvorrichtungen nachgewiesen werden. Dies ermöglicht die Detektion in Anwesenheit des Reaktionsgemisches und bedarf keiner Prozessierungsschritte. Damit besitzt die bevorzugte Ausführungsform die vorteilhafte Eigenschaft, innerhalb eines geeigneten Reaktionsraumes eine Detektion zu erlauben, ohne dieses nach durchgeführter Reaktion öffnen zu müssen. Dies vermeidet die im Stand der Technik beschriebenen Probleme von Mikroarray-Analysen durch Hybridisierungs-, Färbe-, und/oder Waschschritten, welche zusätzlichen Arbeitsaufwand und hohes Kontaminationsrisiko darstellen. Demnach kann die bevorzugte Ausführungsform als Abkehr vom technischen Üblichen bezeichnet werden, da sie ein neues technisches Feld eröffnet und ein im Stand der Technik lang bestehendes Problem löst.

Ergänzend zu den beschriebenen Vorteilen ermöglicht die bevorzugte Ausführungsform das Auslesen der entstehenden Signale zu beliebigen Zeitpunkten der Reaktion. Somit kann eine Echtzeitüberwachung der Reaktion erfolgen, welche zum Beispiel für die Quantifizierung von Nukleinsäureamplifikationen oder Ermittlung der Bindekinetik von Protein-Interaktionen benötigt wird. Damit hebt sich die bevorzugte Ausführungsform vom Stand der Technik ab, in dem typischerweise nur Endpunkt-Bestimmungen erfolgen und daher eine Signaldetektion zu beliebigen Zeitpunkten nicht durchführbar ist.

Multiplex-Analysen bedingen die Detektion mehrerer verschiedener Analyte in einem Reaktionsgemisch. Für die Erhöhung des Multiplexgrads der bevorzugten Reaktion ist die Verwendung von "n" verschiedenartiger Mediator-Sonden für "n" unterschiedliche Zielmoleküle bevorzugt. Jedem zu detektierenden Zielmolekül kann in einer bevorzugten Ausführungsform eine Mediator-Sonde zugeordnet werden, deren Region 1 spezifisch mit dem Zielmolekül interagiert. Die Region 2 der jeweiligen Mediator-Sonde, welche nach erfolgter Spaltung den Mediator darstellt, ist nicht affin beziehungsweise komplementär zum Zielmolekül, stellt aber einen spezifischen Interaktionspartner für ein definiertes Nachweismolekül dar. Dadurch ist jedem Zielmolekül indirekt ein Nachweismolekül zugewiesen, dessen Zuordnung durch die Mediator-Sonde erfolgt. Die Detektion verschiedener Zielmoleküle bedingt verschiedenartige Nachweismoleküle. Aufgrund des bevorzugten Aufbaus ist es ausreichend, wenn sich diese Moleküle ausschließlich in Region 5 unterscheiden. Aufgrund beliebiger Sequenzabfolgen innerhalb dieser Region ist der Parallelitätsgrad nahezu beliebig hoch und somit ein Detektionsverfahren für eine Multiparameter-Analyse gegeben. Es ist somit bevorzugt, das System als Detektionsverfahren für eine Multiparameter-Analyse zu verwenden. Das System kann bevorzugt zur Detektion von einem oder mehreren gleichartigen oder verschiedenen Biomolekülen in einem Gemisch verwendet werden. Außerdem kann das System vorteilhafterweise zur Amplifikation von mindestens einem oder insbesondere mehreren Zielmolekülen verwendet werden, wobei es sich vorteilhafterweise um nicht identische Zielmoleküle handelt. Es kann weiterhin bevorzugt sein, das System unter Verwendung der Aktivität eines Restriktionsenzyms zu verwenden, wobei das Nachweismolekül über eine chemische Schutzgruppe am 3'-terminalen Bereich verfügt, die nach der Reaktion mit der Mediator-Region mittels eines Hilfsmoleküls von dem Nachweismolekül abgespalten wird und eine 3'-terminale OH-Gruppe generiert wird.

In einer erfindungsgemäßen Problemlösung wird die Mediator-Sonde mit der zu untersuchenden Probe zu den in einer Einweg-Reaktionskartusche immobilisierten Nachweismolekülen appliziert, in geeigneter Weise prozessiert und detektiert. Die Reaktionskartusche kann anschließend kontaminationsfrei entsorgt werden. Des Weiteren kann ein Amplifikationsschritt im Reaktionsgefäß vorgeschaltet beziehungsweise parallel zur Detektion durchgeführt werden, womit gegebenenfalls eine spezifische Anreicherung des Zielmoleküls erfolgen kann. Dadurch wird das Transferieren von Reaktionsansätzen mit hoher Zielmolekül-Konzentration (zum Beispiel post PCR-Ansätze) aus einem Amplifikationsin einen Detektionsbereich und den damit entstehenden Arbeitsschritten und Kontaminationsrisiken überflüssig.

Vorliegend offenbart wird ferner ein Prozessierungsgerät und ein mikrofluidische Reaktionskartusche zur Verwendung des Verfahrens oder des Systems. Die Kartusche weist mindestens eine Reaktionskammer auf, in der insbesondere ein universelles Detektionsarray vorliegt, welches vorzugsweise aus einer örtlich aufgelösten Anordnung eines oder mehreren Nachweismolekülen besteht.

Das Prozessierungsgerät unterzieht bevorzugt die Kartusche und Reaktionsflüssigkeit bei einer konstanten Temperatur beziehungsweise einem definierten Temperaturverlauf (Heizen und/oder Kühlen). Das Prozessierungsgerät kann die Änderung des Nachweismoleküls durch ein bevorzugtes System oder Verfahren detektieren. Hierfür kann beispielsweise ein Fluoreszenzsignal verwendet werden. Zusätzlich kann das Gerät mittels aktiver oder passiver Elemente einen Flüssigkeitstransport innerhalb der Reaktionskartusche ermöglichen. Die oben dargelegten Ausführungen sind auf die Mediator-Sonde, das System und das Verfahren zu beziehen.

Die Kosten für die Durchführung eines erfindungsgemäßen Verfahrens können reduziert werden, da in einer möglichen Ausführungsform die Applizierung der zu untersuchenden Probe und der benötigten Reagenzien in eine Einweg-Kartusche automatisiert durchgeführt werden kann. Somit entfällt die Notwendigkeit geschultes Personal für diese Arbeiten einzusetzen.

Zudem wird vorliegend offenbart ein Kit umfassend mindestens ein Nachweismolekül im Sinne der Erfindung, Polymerasen und dNTPs. Das so bereitgestellte Kit kann in jeder beliebigen Nachweis- oder Detektionsreaktion eingesetzt werden und ist daher vorteilhafterweise ein universelles Nachweiskit.

**Beispiele**

Im Folgenden soll die Erfindung sowie der Stand der Technik anhand von Figuren und Ausführungsbeispielen erläutert werden, ohne jedoch hierauf beschränkt zu sein. Es zeigen:

| | |
|---|---|
| Fig. 1(A-D) | Verschiedene Festphasen-basierte Detektionsverfahren nach invasiver Spaltung einer immobilisierten Sonde |
| Fig. 2 | Bevorzugten Aufbau einer Mediator-Sonde |
| Fig. 3A, B | Bevorzugte Interaktion der Mediator-Sonde mit dem Zielmolekül und Mediator-Sonden-Spaltung |
| Fig. 4A, B | Darstellung eines bevorzugten Nachweismoleküls |
| Fig. 5i).vi) | Schematische Darstellung einer bevorzugten enzymatischen Mediatorverlängerung |
| Fig. 6 | Schematische Darstellung bevorzugter Position chemischer Modifikation innerhalb des Nachweismoleküls |
| Fig. 7 | Bevorzugte Detektion des Mediators mit Hilfe eines immobilisierten Nachweismoleküls |
| Fig. 8 | Bevorzugte Interaktion von Mediator-Sonde und Nachweismolekül |
| Fig. 9 | Schematische Darstellung der bevorzugten Anwendungsbereiche der Mediator-Sonden-Technologie |
| Fig. 10 | Normalisierter Fluoreszenz-Plot einer PCR unter Verwendung einer bevorzugten Mediator-Sonden und im Reaktionsgefäß immobilisierter Nachweismoleküle |
| Fig. 11 | Schematische Darstellung eines bevorzugten PCR-Verfahrens |

Fig. 12 Vergleich der Charakteristiken von Mediator-Sonden-PCR und Hydrolyse-Sonden-PCR

Fig. 13 Amplifizierung von verschiedenen Targets mit Mediator-Sonden-PCR und Hydrolyse-Sonden-PCR

Fig. 1 (A-D) zeigt verschiedene Festphasen-basierte Detektionsverfahren nach invasiver Spaltung einer immobilisierten Sonde. (A) Direkte, Fluoreszenz-basierte invasive Spaltungsdetektion. Möglichkeit 1: Die Sonde liegt immobilisiert an der Substratoberfläche vor. Das Invader-Oligonukleotid (upstream oligonucleotide) und die Zielsequenz (target) werden zu der Reaktionslösung hinzugegeben (siehe Abbildung 1). Möglichkeit 2: Die Sonde und das Invader-Oligonukleotid sind auf der Oberfläche immobilisiert. Die Zielsequenz wird zu dem Reaktionsmix hinzugegeben (siehe Abbildung 2). In beiden Fällen erfolgt eine Spaltung des Sondenmoleküls, woraus die Änderung eines Fluoreszenzsignals resultiert. Quelle: (Lu,M.C. et al, 2002, A surface invasive cleavage assay for highly parallel SNP analysis, Hum. Mutat., 19, 416-422.).

(B) Indirekte Spaltungsdetektion. Eine Dabcyl-modifizierte Sonde liegt immobilisiert an einer Festphase vor. Nach erfolgter invasiver Spaltung erfolgt die Ligation eines Biotin-markierten Linkers, an welchen Streptavidin-beschichtete Goldpartikel gebunden werden. Quelle: (Nie,B. et al, 2006, Quantitative detection of individual cleaved DNA molecules on surfaces using gold nanoparticles and scanning electron microscope imaging, Anal. Chem., 78, 1528-1534.).

(C) Indirekte Spaltungsdetektion durch anschließende Rolling Circle Amplification. Nach invasiver Spaltung einer immobilisierten, 5'-markierten Sonde erfolgt ein Ligationsschritt mit anschließender Rolling Ciricle Amplification. Es werden zwei unterschiedliche Strategien dargestellt, bei welchen nur die Sonde (a) beziehungsweise die Sonde und das Invader-Oligonukleotid (b) immobilisiert vorliegen. Quelle: (Chen,Y. et al, 2004, Surface amplification of invasive cleavage products, J. Amer. Chem. Soc., 126, 3016-3017.).

(D) Indirekte, Fluoreszenz-basierte Spaltungsdetektion. An eine Fluoreszenz-markierte Sonde wird eine markierte Detektionssonde hybridisiert und das Fluoreszenzsignal detektiert. Nach erfolgten Waschschritten und durchgeführter invasiven Spaltung erfolgt ein weiterer Hybridisierungsschritt mit der beschriebenen Detektionssonde. Die anschließende Fluoreszenzmessung erlaubt einen Rückschluss auf die Anwesenheit der Zielsequenz im Reaktionsansatz. Quelle: (Lockett,M.R. et al, 2007, Molecular beacon-style hybridization assay for quantitative analysis of surface invasive cleavage reactions, Anal. Chem., 79, 6031-6036.).

Fig. 2 zeigt einen bevorzugten Aufbau einer Mediator-Sonde. Die Mediator-Sonde besteht insbesondere aus einem Oligonukleotid und ist in zwei funktionelle Regionen unterteilt. Region 1 ist affin beziehungsweise komplementär zum Vorlage und/oder Zielmolekül, Region 2 interagiert ausschließlich mit einem spezifischen Nachweismolekül. Zwischen den Regionen befindet sich eine potentielle Spaltstelle.

Fig. 3 zeigt eine bevorzugte Interaktion der Mediator-Sonde mit dem Vorlagemolekül und/oder Zielmolekül und Mediator-Sonden-Spaltung. Die Mediator-Sonde, Hilfsmolekül 1 (hier: Primer) und Hilfsmolekül 2 (hier: Enzym mit Polymerisations- und Nuklease-Aktivität (Polymerase)) interagieren mit dem Vorlagemolekül und/oder Zielmolekül (hier: Nukleinsäuresequenz) (A). Unter geeigneten Reaktionsbedingungen wird der Primer durch die Polymerase verlängert und die Mediator-Sonde gespalten, wobei eine Mediator-Region freigesetzt wird (B).

Fig. 4A, B zeigt eine Darstellung eines bevorzugten Nachweismoleküls. Lineare Darstellung (A). Darstellung eines 3'-immobilisierten Nachweismoleküls unter Ausbildung der Sekundärstruktur (B). Die revers-komplementären Sequenzabschnitte, durch deren Interaktion die Sekundärstruktur des Nachweismoleküls entsteht, sind als schwarze Bereiche, die Mediator-Hybridisierungssequenz als diagonal-gestreifter Bereich dargestellt. Die Region a kann dabei mit oder ohne PTO-Modifikationen vorliegen.

Ein Nachweismolekül besteht in einer bevorzugten Ausführungsform aus einem Oligonukleotid, welches in mehrere Regionen unterteilt ist (vergleiche bevorzugt Fig. 4).

Region a (= erste Region) umfasst den 5'-Terminus des Nachweismoleküls, welcher in einer bevorzugten Ausführungsform aus einem Sequenzabschnitt und einem Fluoreszenzakzeptor Q besteht. Region c ist eine revers-komplementäre Sequenzabfolge von Region a und wird durch Region b davon abgetrennt. Region d (=dritte Region) trennt Region c und Region e (=zweite Region), welche spezifisch mit einem Mediator-Molekül interagieren kann. Region f (=vierte Region) umfasst den 3'-terminalen Sequenzbereich, welcher bevorzugt über eine chemische Modifikation verfügt und somit eine gerichtete Immobilisierung des Oligonukleotids ermöglicht. Ein Fluoreszenzdonor F ist in dem Fachmann bekannter Weise an einen Bereich der Region b bis Region f, beispielsweise Region d, assoziiert. Es ist bevorzugt, dass das Nachweismolekül eine Haarnadel-Struktur aufweist. Region a und Region c des Nachweismoleküls bilden unter Reaktionsbedingungen eine definierte Sekundärstruktur (im Sinne der Erfindung als Haarnadel-Struktur bezeichnet), bei welcher der 5'-Terminus mit einem internen Sequenzabschnitt hybridisiert.

Fig. 5 i)-vi) stellt eine schematische Darstellung einer bevorzugten enzymatischen Mediatorverlängerung dar. i) Ein Nachweismolekül liegt immobilisiert an einer Festphase vor und nimmt unter Reaktionsbedingungen eine definierte Sekundärstruktur an. Zwei geeignete Fluoreszenz-Modifikationen F und Q interagieren miteinander, wobei das Fluoreszenzsignal von F unterdrückt wird. ii) Der Mediator kann mit dem Nachweismolekül an definierter Position (Mediator-Hybridisierungssequenz, Region 5) interagieren iii) - iv) und wird dabei von einem Nachweishilfsmolekül (hier: Polymerase) enzymatisch verlängert. Dabei wird das Fluoreszenzakzeptormolekül Q vom Nachweismolekül abgespalten, womit die Fluoreszenzintensität des Fluoreszenzfarbstoffes F wiederhergestellt wird. vi) Nach Abspaltung von Region 1 nimmt das Nachweismolekül eine lineare Konformation an, wodurch eine weitere Verlängerung des Mediators erfolgen kann. Der in Fig. 5 gezeigte Mechanismus findet auch statt, wenn PTO-Modifikationen vorhanden sind.

Fig. 6 zeigt eine schematische Darstellung bevorzugter Position chemischer Modifikation innerhalb des Nachweismoleküls. An geeigneten Sequenzpositionen innerhalb der Region 1 und/oder Region 2 sind modifizierte Nukleotide eingebaut, welche eine potentielle Mediator-Elongation an definierter Position terminieren.

Fig. 7 stellt eine bevorzugte Detektion des Mediators mit Hilfe eines immobilisierten Nachweismoleküls dar. i) Ein Nachweismolekül liegt immobilisiert an einer Festphase vor und nimmt unter Reaktionsbedingungen eine definierte Sekundärstruktur an. Zwei geeignete Fluoreszenz-Modifikationen F und Q interagieren miteinander, wobei das Fluoreszenzsignal von F unterdrückt wird. Der 3'-terminale Sequenzbereich liegt ungepaart vor und dient als potentielle Mediator-Hybridisierungssequenz (diagonal gestreifter Bereich). ii) An dieser definierten Position kann der Mediator mit dem Nachweismolekül interagieren iii, iv) und wird dabei von einem Nachweishilfsmolekül (hier: Polymerase) enzymatisch verlängert. v) Dabei wird das Fluoreszenzakzeptormolekül Q vom Nachweismolekül abgespalten, womit die Fluoreszenzintensität des Fluoreszenzfarbstoffes F wiederhergestellt wird. Nach einer geeigneter Dauer werden die Reaktionsbedingungen durch Erhitzen der Reaktionslösung geändert, so dass die Polymerase und der verlängerte Mediator vom Nachweismolekül abdissoziiert.

Fig. 8 zeigt eine bevorzugte Interaktion von Mediator-Sonde und Nachweismolekül. Falls die ungespaltene Mediator-Sonde mit dem Nachweismolekül interagiert, erfolgt selbst in Anwesenheit geeigneter Nachweishilfsmoleküle keine enzymatische Verlängerungsreaktion, da diese einen 3'-OH-Terminus der Mediator-Sequenz benötigt. Somit wird die Generierung falsch-positiver Signale verhindert. Zusätzlich kann eine 3'-terminale Modifizierung vorhanden sein, um eine unspezifische Elongation zu unterbinden.

Fig. 9 stellt eine schematische Darstellung der bevorzugten Anwendungsbereiche der Mediator-Sonden-Technologie dar. Die Mediator-Sonden-Technologie kann DNA, in cDNA umgeschriebene RNA oder an Protein-assoziierte Aptamere detektieren. Die Prozessierung der Medaitor-Sonde kann gegebenenfalls an einen Amplifikationsschritt (A) des Zielmoleküls integriert werden. Dargestellt ist die Detektion mittels eines immobilisierten, Mediatorspezifischen Nachweismoleküls. Durch Interaktion mit einem Hilfsmolekül (hier: Polymerase) wird durch eine Mediator-vermittelte Reaktion eine Zustandsänderung des Nachweismoleküls generiert (hier: Fluoreszenzänderung).

Fig. 10 zeigt einen normalisierten Fluoreszenz-Plot einer PCR unter Verwendung einer bevorzugten Mediator-Sonde und im Reaktionsgefäß immobilisierter Nachweismoleküle. Die Reagenzien, welche unter anderem Zielsequenz-spezifische Primer sowie die Mediator-Sonde und verschiedene Staphylococcus aureus-Genomäquivalente beinhalten, werden in ein geeignetes Reaktionsgefäß mit immobilisierten Nachweismolekülen pipettiert und mit einer geeigneten Versiegelungsfolie verschlossen. Die Reaktion wurde in einem Thermocycler durchgeführt, wobei die Messung der Fluoreszenz zu den angegebenen Zyklen in einem gesonderten Gerät vorgenommen wurde. In jedem PCR-Zyklus wird der zu amplifizierende Sequenzabschnitt verdoppelt, wobei bei jeder Duplikation ein Mediator aus der Spaltung einer Mediator-Sonde hervorgeht. Der freigesetzte Mediator interagiert mit dem Nachweismolekül in geeigneter Weise, wobei ein detektierbares Fluoreszenzsignal entsteht. Der Plot zeigt eine Korrelation der DNA-Menge und dem Fluoreszenzverlauf. Die Fluoreszenzintensitäten wurden auf den Wert von Zyklus 1 normiert (Der Messwert von Zyklus 37 wurde durch Kondensat an der Deckelfolie verfälscht und daher nicht berücksichtigt).

Fig.11 zeigt eine schematische Darstellung eines bevorzugten Verfahrens. Die Schritte A bis H zeigen dabei die Amplifikationsreaktion und die Detektion. Ein Nukleinsäure-Target ist im gezeigten Fall gleichzeitig Ziel- und Vorlagemolekül. Nach Denaturierung (B) lagern sich die Mediator-Sonde (=Mediator-Probe), die Primer und Polymerase an (C). In Schritt D wird die Verlängerung der Primer gezeigt sowie die Spaltung der Mediator-Sonde und der Abbau der Sonden-Region. Die Mediator-Region wird in diesem Schritt freigesetzt. Die Mediator-Region lagert sich anschließend (E) an ein Nachweismolekül (=Universal Reporter) an. In Schritt F wird die Mediator-Region durch eine Polymerase verlängert. Das Dequenching kann über zwei Wege erfolgen. Entweder über den sequenziellen Abbau des 5'-Endes des Nachweismoleküls und der Freisetzung des Quencher-Rests (G) oder über Displacement des 5'-Endes und Entfaltung der Haarnadelstruktur (H). Dabei finden alle Schritte in einem Thermozyklus statt.

Fig. 12 zeigt einen Vergleich zwischen verschiedenen Fig. 12 Charakteristiken von Mediator-Sonden-PCR und Hydrolyse-Sonden-PCR. Dabei wurden verschiedene Konzentrationen von HAPV18DNA amplifiziert und Kontrollen ohne Vorlage-DNA verwendet. Auf der x-Achse wurden die berechneten Kopiezahlen der Mediator-Sonden-PCR aufgetragen. Auf der γ-Achse die der Hydrolyse-Sonden-PCR (B) bis (D). (A) zeigt die LOD, das Limit der Detektion, für die HPV18 Detektion. Die Wahrscheinlichkeit für eine erfolgreiche Amplifikation (x-Achse) für eine bestimmte Input-Kopienzahl (y-Achse) wurde mit Probit-Analysen bestimmt. Die Mediator-Sonde resultierte in den schwarzen Linien, die Hydrolysen-Sonde in den grauen. Die oberen und unteren gestrichelten Linien zeigen 95% CI. In (B) wird die Intraassay-Varianz für fünf verschiedene DNA-Konzentrationen gezeigt. In (C) wird die Interassay-Varianz für fünf DNA Konzentrationen in fünf verschiedenen PCR Durchgängen gezeigt. In (D) zeigt die Abbildung die Ergebnisse der Duplex-PCR.

Fig. 13 zeigt die Ergebnisse der Amplifizierung von verschiedenen Targets mit Mediator-Sonden-PCR und Hydrolyse-Sonden-PCR. (A):HPV18, (B):E.coli, (C):S.aureus, (D): humanes Beta Aktin.

**Ausführungsbeispiel i)**

Die Ausführungsbeispiele sind außerdem schematisch in Fig. 9 dargestellt. Eine einfache Demonstration eines bevorzugten Nukleinsäurenachweises kann wie folgt durchgeführt werden: Zum Nachweis bakterieller DNA in einer zu untersuchenden Probe wird ein Nachweismolekül in einem geeignetem Reaktionsgefäß immobilisiert. Das Nachweismolekül kann aber auch in Lösung vorliegen. Anschließend werden die Probe und die benötigten Reagenzien dem Reaktionsgefäß zugegeben und das Gemisch nach einer initialen Temperaturhalteschritt bei 95 °C zyklisch erhitzt und abgekühlt. Während diesem Prozess wird an definierten Zeitpunkten des Zyklus' die Fluoreszenz im Reaktionsgefäß detektiert. Im Folgenden wird das Ausführungsbeispiel im Detail beschrieben:

In "NucleoLink-Strips" (NUNC, Langenselbold, Deutschland, Cat.-No. 248650) werden 25 μL einer 100 nM Lösung eines Nachweismoleküls der Sequenz 5'-DABCYL-CCGCAG*A*A*GATGAGATC(dT-FAM)GCGGTGTTGGTCGTAGAGCCCAGAACGATTTTTTTTTTTTTTTTTTTTT-[C6NH2]-3' (* = Phosphothioat) (IBA, Göttingen, Deutschland) in Kopplungspuffer (10 mM 1-Methyl-Imidazole (1-Melm) (pH 7,0) (Sigma-Aldrich, Steinheim, Deutschland) und 10 mM 1-Ethyl-3-(3-Dimethylaminopropyl)-Carbodiimide (EDC) (Sigma-Aldrich, Steinheim, Deutschland)) pipettiert, mit ViewSEALTM-Deckelfolie (Greiner-BioOne, Frickenhausen, Germany, Cat.-No.676070) verschlossen und über Nacht bei 50°C inkubiert. Der Überstand wird verworfen und die Mikroreaktionsgefäße werden anschließend mit 100 μL Waschpuffer (100 mM Tris-HCl (pH 7.5), 150 mM NaCl, 0.1% Tween 20 (Carl Roth, Karlsruhe, Deutschland)) gewaschen und mit 25 μL einer 1 M Glycin-Lösung (Carl Roth, Karlsruhe, Deutschland) in Kopplungspuffer 1 Stunde bei 50 °C inkubiert und erneut gewaschen.

Die Reaktionsgefäße werden mit 25 μL PCR-Reaktionsmix (1x Finnzymes DyNamo Flash Probe (Finnzymes, Cat.-No. F-455), Primer-Moleküle der Sequenzen 5'-GAGGTAGGAAACTCTGGATCAGGTAT-3' (300 nM) (biomers.net, Ulm, Deutschland), 5'-TCTATTGAAAAACACTCCTATTGGAAGA-3' (300 nM) (biomers.net, Ulm, Deutschland), eine Mediator-Sonde der Sequenz 5'-TCTGGGCTCTACGACCAACAGGTATTCACAGTGGTAAAGGCGGACAACAAGAGCCCAG A-[Phosphat]-3' (200 nM) (biomers.net, Ulm, Deutschland)) sowie unterschiedlichen Konzentrationen einer Staphylococcus aureus DNA, die den Lokus Exfoliatin-Toxin B (NCBI Accession-Number M17348) enthält, befüllt. Je DNA-Konzentration werden vier Mikroreaktionsgefäße verwendet. Die Reaktionsgefäße werden mit ViewSEALTM-Deckelfolie verschlossen und in einen GeneAmp® 9700 Thermocycler (Perkin-Elmer, Massachusetts) überführt. Nach einer Inkubationsphase (7 min bei 95 °C) wird das zyklische Temperaturprotokoll (30 s bei 95 °C, 3 min bei 58 °C) durchgeführt und die Reaktionsgefäße nach definierten Zyklen aus dem Thermocycler entnommen und mit Hilfe des Mikrotiterplatten-Lesegeäts Victor$^2$ 1420 Multilabel Counter (Perkin-Elmer, Massachusetts) das Fluorescein-Signal gemessen. Anschließend werden die Reaktionsgefäße wieder in den Thermocycler überführt. Die Fluoreszenzwerte der einzelnen Mikroreaktionsgefäße werden auf den jeweiligen Wert des ersten Zyklus normalisiert, so dass für jedes Reaktionsgefäß ein Amplifikationsfaktor in Abhängigkeit des Prozessierungszyklus erstellt werden kann (siehe Fig. 10).

**Ausführungsbeispiel ii)**

In einem zweiten Beispiel wurde als Zielmolekül RNA verwendet. Die RNA wird mittels einer reversen Transkription oder durch ein anderes geeignetes enzymatisches System in cDNA umgeschrieben. Dabei wird dieser Schritt in einem gesonderten Reaktionsgefäß durchgeführt und ein Aliquot zu einer Nachweisreaktion gemäß Ausführungsbeispiel i) hinzugegeben. Alternativ kann die reverse Transkription und anschließende Amplifikation gemäß Ausführungsbeispiel i) im selben Reaktionsgefäß stattfinden. Als Versuchsziel steht bei diesem Beispiel die Expressionsanalyse eines oder mehrerer Gene im Vordergrund.

**Ausführungsbeispiel iii)**

Der parallele Nachweis von DNA und RNA in einer Probe kann durch Kombination geeigneter Enzymsysteme durchgeführt werden. Dabei wird die nachzuweisende RNA mit Hilfe von Primern mit definiertem 5'-Sequenzüberhang amplifiziert (Siehe Fig. 9). Die für diesen Nachweis verwendete Mediator-Sonde ist derartig ausgelegt, dass sie teilweise an den Sequenzüberhang, den Primer sowie einen Abschnitt des verlängerten Primers bindet. Durch diesen definierten Lokus ist sichergestellt, dass nur aus RNA generierte cDNA durch eine spezifische Mediator-Sonde detektiert wird, jedoch nicht der genomische Lokus, von dem die RNA transkribiert wurde. Zur Detektion von genomischer DNA im Reaktionsansatz werden Mediator-Sonden verwendet, die ausschließlich komplementär zu dieser Sequenz sind. In der gegebenenfalls durchgeführten Amplifikationsreaktion wird die generierte cDNA und spezifische Abschnitte der genomischen DNA amplifiziert, wobei lokus-spezifische Mediator-Sonden gespalten und der Mediator durch geeignete Nachweisverfahren an einem örtlich aufgelösten immobilisierten Nachweismolekül detektiert werden kann.

**Ausführungsbeispiel iv)**

In ein geeignetes Reaktionsgefäß werden Reagenzien, welche Zielmolekül-spezifische Aptamere umfassen und die zu untersuchende Probe gegeben, wobei die Nachweismoleküle örtlich aufgelöst immobilisiert (siehe Fig. 9) oder in Lösung vorliegen. Das nachzuweisende Zielmolekül kann beispielsweise ein Protein oder Peptid sein ist aber nicht darauf beschränkt. Ein Aptamer bindet an das Zielmolekül und verändert seine Struktur, so dass sich nach erfolgter Interaktion eine Aptamer-spezifische Mediator-Sonde und Primer anlagern können. Durch Prozessierung mit einem geeigneten Enzymsystem kann der angelagerte Primer verlängert werden, wobei eine Spaltung der Mediator-Sonde erfolgt. Der freigesetzte Mediator kann mit Hilfe eines spezifischen Nachweismoleküls detektiert werden. Der enzymatische Amplifikationsprozess kann isotherme Verfahren beinhalten, ist aber nicht darauf beschränkt.

**Ausführungsbeispiel v)**

In einer besonderen Ausführungsvariante werden DNA, RNA und Peptide beziehungsweise Proteine oder eine andere Kombination der genannten Stoffklassen durch die beschriebenen Verfahren i)-iv) in einem Ansatz parallel detektiert. Das Verfahren beinhaltet isotherme Amplifiaktionsverfahren, ist aber nicht auf diese beschränkt. Diese Ausführung wird in Fig. 9 illustriert.

**Ausführungsbeispiel vi)**

Unabhängig von der Art des Nachweises kann das Reaktionsgefäß beispielsweise das etablierte und weit verbreitete Mikrotiterplattenformat (96-well plate) besitzen, womit handelsübliche Temperierungs- und Auslesegeräte, beziehungsweise Geräte, die beide Funktionen kombinieren, verwendet werden können. In allen Fällen können die Nachweismoleküle örtlich aufgelöst immobilisiert (Array) vorliegen. Als mögliches Reaktionsgefäß kann auch eine Flusszelle betrachtet werden, die nach durchgeführter Analyse gegebenenfalls gereinigt und wiederverwendet werden kann. In einer besonderen Ausführungsform kann das Reaktionsgefäß eine Kartusche darstellen, in welcher die Nachweismoleküle immobilsiert vorliegen können. Der Reaktionsraum kann auch durch die Verwendung eines modifizierten Mikroskop-Objektträger und eines geeigneten Rahmens definiert werden. Diese Ausführung hat die vorteilhafte Eigenschaft, dass die Immobilisierung der Nachweismoleküle auf geeigneten Materialien im Stand der Technik beschrieben ist und passende Kleberahmen (Peqlab in-situ Kleberahmen, Peqlab Biotechnologie, Erlangen, Deutschland) sowie temperierbare Prozessierungsgeräte (Peqlab PeqStar in-situ, Peqlab Biotechnologie, Erlangen, Deutschland) und Auslesegeräte (BioAnalyzer, LaVision BioTec GmbH, Bielefeld, Deutschland) kommerziell erhältlich sind. Das Format der Kartusche ist nicht festgelegt und kann gegebenenfalls nach den Wünschen des Benutzers erstellt sein. Die Kartusche kann mit Hilfe von integrierten Prismen einen eingekoppelten Lichtstrahl zur Anregung oberflächennaher Fluorophore mittels TIRF durch einen Auslesebereich leiten. Ferner kann diese Kartusche in Kombination mit einem Gerät verwendet werden, welches über eine Temperierungsvorrichtung und optischen Komponenten zur Anregung und Detektion von Fluoreszenzsignalen verfügt. Die Kartusche kann gegebenenfalls über mikrofluidische Strukturen, zum Beispiel Einfüll- und Belüftungsöffnungen, Konnektierungen für aktive Elemente, Misch-, Mess- und Aliquotierungskammern, -kanäle oder -strukturen, oder Strukturen, die für anderweitige Zwecke verwendet werden können, verfügen. Das System kann über Pumpen oder andere Aktoren verfügen, mit deren Hilfe die Flüssigkeit prozessiert werden kann. Zusätzlich können weitere Reagenzien vorgelagert sein.

**Ausführungsbeispiel vii)**

Es wird eine PCR unter Verwendung einer bevorzugten Mediator-Sonde der Erfindung durchgeführt (vergleiche auch Fig. 11). Für die Amplifikationsreaktion werden gewöhnliche Oligonukleotid-Primer und eine Taq-Polymerase eingesetzt. Die Mediator-Sonde im Sinne der Erfindung ist dabei ein bifunktionelles Oligonukleotid, welche die Echtzeitdetektion der PCR-Reaktion ermöglicht. Um die Erfindung mit dem Stand der Technik zu vergleichen, werden die Versuche parallel mit einer Hydrolyse-Sonde aus dem Stand der Technik durchgeführt.

*Probenmaterial*

Für den Versuch wurden *Staphylococcus aureus* DNA Proben (Genomic Research Laboratory; Prof. Jacques Schrenzel, Genf, Schweiz) verwendet. Die Proben enthielten den genomische Locus des exfoliativen Toxin B (GenBank accession number AP003088). Das pBR322 Plasmid enthält das humane Papillomavirus 18 (HPV18) Genom und wurde von GenoID (Budapest, Ungarn) bereit gestellt. *Escherichia coli* K12 DH5_Z1 DNA, welche den genomischen Locus des Peptidoglycan assoziierte Lipoprotein (GenBank accession _65796) enthält, wurde mit einem magnetischen Bead basierten DNA-Isolierungs-Kit isoliert. Humane genomische DNA wurde mit QIAamp DNA Blood mini kit (Qiagen) aus Vollblut isoliert. Für die duplex PCR Reaktionen wurden kommerziell erhältliche humane DNA (Roche Diagnostics) verwendet. Die DNA Proben wurden in 0, 2 X Tris-EDTA Puffer verdünnt. Es wurden 10 ng/μL Lachsspermien-DNA (Invitrogen) hinzugegeben, um unspezifische Adsorption der DNA-Targets an den Reaktionsgefäßen zu verhindern.

*Oligonukleotide*

Es wurden die folgenden Oligonukleotide verwendet:

Nachweismolekül 01:

CCGCAG*A*A*GATGAGATC(dT-FAM)GCGGTGTTGGTCGTAGAGCCCAGAACGATTTTTTTTTTTTTTTTTTTTT

Modifikationen: 5': DABCYL; 3': $C_6NH_2$

*=Phosphothioate

Nachweismolekül 02:

CCGCAG*A*A*GATGAGATC(dT-Cy5)GCGGTGTTCACTGACCGAACTGGAGCATTTTTTTTTTTTTTTTTTTTT

Modifikationen: 5': BHQ-2; 3': $C_6NH_2$

Target: Escherichia coli K12 Peptidoglycan assoziierte Lipoprotein (pal Gen), GenBank accession number X05123

Forward Primer: GGCAATTGCGGCATGTTCTTCC

Reverse Primer: TGTTGCATTTGCAGACGAGCCT

Hydrolyse-Sonde: ATGCGAACGGCGGCAACGGCAACATGT
Modifikation: 5': 6-FAM 3': BHQ-1

Mediator-Sonde:
AAATCGTTCTGGGCTCTACGCGAACGGCGGCAACGGCAACATGT
Modifikation: 3': PH

Target: Staphylococcus aureus exfoliatives Toxin B

Forward primer: AGATGCACGTACTGCTGAAATGAG
Reverse primer: AATAAAGTACGGATCAACAGCTAAAC
Hydrolyse-Sonde: CCGCCTACTCCTGGACCAGG
Modifikation: 5': 6-FAM; 3': BBQ
Mediator-Sonde:
AAATCGTTCTGGGCTCTACGGTATTCACAGTGGTAAAGGCGGACAACA
Modifikation: 3': PH

Target: HPV18 GenBank accession no. NC_001357.1

Forward primer: GCTGGCAGCTCTAGATTATTAACTG
Reverse primer: GGTCAGGTAACTGCACCCTAA

Hydrolyse-Sonde: GGTTCCTGCAGGTGGTGGCA
Modifikationen: 5': 6-FAM; 3': BHQ-1
Mediator-Sonde: AAATCGTTCTGGGCTCTACGGTTCCTGCAGGTGGTGGCA
Modifikationen: 3 - PH

Target: H. sapiens ACTB GenBank accession no. AC_000068.1/ HGNC:132

Forward primer: TCACCCACACTGTGCCCATCTACGA
Reverse primer: CAGCGGAACCGCTCATTGCCAATGG
Hydrolyse-Sonde 01: ATGCCCTCCCCCATGCCATCCTGCGT
Modifikation: 5': 6-FAM; 3': DDQ-1
Hydrolyse-Sonde 02: ATGCCCTCCCCCATGCCATCCTGCGT
Modifikation: 5' Cy5; 3': DDQ-2
Mediator-Sonde 01:
AAATCGTTCTGGGCTCTACGCCCTCCCCCATGCCATCCTGCGT
Modifikation: 3': PH
Mediator-Sonde 02:
ATGCTCCAGTTCGGTCAGTGCCCTCCCCCATGCCATCCTGCGT
Modifikation: 3': PH

Dabei wurden alle modifizierten Oligonukleotide mit HPLC aufgereinigt.

Die Mediator-Sonden wurden in einem zweistufigen Prozess designt. Die Sonden-Region und die Mediator-Region überlappen dabei mit einem Nukleotid. Daher muss das 5'-Ende der Sonden-Region mit dem 3'-Ende der Mediator-Region übereinstimmen. In dem vorliegenden Versuch wurde ein Guanosin-Nukleotid hierfür verwendet. Die Sonden-Region wurde nach den Richtlinien für die Entwicklung einer Hydrolyse-Sonde designt /Länge 25 - 30 Nukleotide, Sonden-Schmelztemperatur: 5-10° C und > als die Primer-Schmelztemperatur). Die Mediator-Region wurde so gestaltet, dass diese Region keinerlei Homologien zu dem Target aufweist. Der 3'-Terminus wurde mit einer Phosphatgruppe blockiert, um eine Verlängerung der Mediator-Sonde zu verhindern.

Das Design für die Nachweismoleküle wurden in silico erstellt, um eine Haarnadelstruktur mit einem ungepaarten einzelsträngigen 3'-Stamm zu erhalten. Vorhersagen über die Sekundärstruktur wurden unter Verwendung von RNAfold gemacht und die Schmelztemperatur wurde mit VisOMP (Visual Oligonucleotide Modeling Program) berechnet. Für die Sekundärstruktur wurden die Einstellungen "no dangling end energies," "DNA settings,""60 °C" in der "advanced folding" Option gewählt. Die Schmelztemperatur des Stamms (GC-Gehalt 71%) ist 71,4 °C und erlaubt eine Rückfaltung während des Kühlungsschritts (60 °C) während jedes Thermozykluses. Die gefaltete Struktur stellt ein FRET-Paar bereit, wobei das Paar an beiden Strängen in enger räumlicher Nähe zueinander angeordnet ist. Das FRET-Paar umfasst einen 5'-terminalen Quencher und ein inneres Fluorophor ist ausgewählt, um eine hohe Quenching-Effizienz zu erzielen. Der 3' ungepaarte Stamm umfasst die Bindestelle für die Mediator-Region, welche revers-komplementär zu der Mediator-Region ist. Um eine Verlängerung des Nachweismoleküls zu verhindern, wurde der 3'Terminus mit einer Amino-Gruppe blockiert. Für die Duplex PCR-Versuche wurde ein zweites Nachweismolekül designt, wobei dies eine identische Sequenz wie das erste Nachweismolekül aufweist, nur dass eine veränderte Mediator-Bindestelle und ein anderes FRET-Paar eingesetzt wurden.

*Effizienz des Quenchings*

Die Auswahl von geeigneten Fluorophor-Farbstoffen und Quencher-Resten war besonders wichtig, um eine hohe Quenching-Effizienz und analytische Sensitivität für die Detektion von besonders geringen Mengen von Nukleinsäuren zu ermöglichen. Um die Effizienz des Quenching zu bestimmen, wurde für jede dual-gelabelte Hydrolyse-Sonde und Nachweismolekül die Fluoreszenzemission bestimmt, mit und ohne DNase I Behandlung. Die Quenching-Effizienz (Eq) bestimmt sich wie folgt:

$$Eq = 1 - (I_{unverdaut}/I_{verdaut}) \times 100,$$

wobei $I_{unverdaut}$ die Fluoreszenzemission von unverdauter Probe und $I_{verdaut}$ die Fluoreszenzemission von mit DNase I-behandelten Proben ist.

*Mediator-Sonden-PCR und Hydrolysen-Sonden-PCR Versuche*

Der Mediator-Sonden-PCR Reaktionsansatz umfasst 1X PCR Puffer (GenoID, Budapest, Hungary), 0,1 U/μL Hot StarTaq plus Polymerase (Qiagen), 200 μmol/L Deoxyribonukleotide (Qiagen), 300 nmol/L Nachweismolekül (synthetisiert durch IBA), 300 nmol/L Targetspezifische Primerpaare und 200 nmol/L Mediator-Spnde (synthetisiert von biomers.net). Der Hydrolyse-Sonden-PCR Reaktionsansatz enthält die gleichen Mengen Reagenzien außer der Mediator-Sonde, welche durch die Hydrolyse-Sonde ersetzt wurde (200 nmol/L; synthetisiert von biomers.net). Außerdem wurden keine Nachweismoleküle hinzugegeben. Anschließend wurden in beide Ansätze DNA-Vorlagemoleküle hinzugegeben (bei den Negativkontrollen wurde stattdessen die gleiche Menge an $H_2O$ hinzugegeben). Das Reaktionsvolumen betrug 10 μL.

Alle real-time PCR Reaktionen wurden in einem Corbett Rotor-Gene 6000 (Corbett Research Pty., now Qiagen GmbH) mit folgendem universal Thermocycling Profil durchgeführt:

initial polymerase activation: 95 °C für 5 min

45 Zyklen mit Denaturierung bei 95 °C für 15 s und

einem kombinierten Annealing- und Elongationsschritt bei 60 °C für 45 s.

Fluoreszenzsignale erfolgten am Ende jedes Elongationsschrittes. Datenanalyse wurden mit der Rotor-Gene 6000 Software (version 1.7.87) vorgenommen.

*Statistische Analyse*

Das Limit der Detektion (LOD) für HPV18 Detektion wurde wie folgt bestimmt:
Es wurden verschiedenen DNA-Konzentrationen amplifiziert ($10^4$, $10^3$, $5x10^2$, $10^2$, $5x10^1$, $10^1$, 10°, und $10^{-1}$ Kopien pro Reaktion). Der Anteil an positiven Amplifikationen pro DNA-Konzentration wurde bestimmt. Probit-Analysen mit SPSS (Statistical Package for Social Sciences, version 19; IBM) erlauben eine Vorhersage der Kopienzahl pro Reaktion, die zu einer mit 95%iger Wahrscheinlichkeit positiven Amplifikation führt.

*Ergebnisse*

*Effizienz des Quenchings*

Fluoreszernzemission aller fluorogenen Moleküle stieg mit Abbau im Vergleich zu unverdauten Sonden. Die beobachteten Eq-Werte der spezifischen Hydrolyse-Sonden lagen zwischen 54,5% (3,1%) [Cy5/2,3-dichloro-5,6-dicyano-1,4-benzoquinone 2 (DDQ-2)] und 92,7% (0,5%) [FAM/di-tert-butylhydroquinone 1 (BHQ-1)]. Die Quenchingeffizienz der Nachweismoleküle lag hingegen zwischen 83,7% (1.4%) (Cy5/BHQ-2) und 90,9% (0,4%) (FAM/Dabcyl). Diese Ergebnisse stimmen mit den bekannten Eq-Werten für FAM/Dabcyl (80%-91%), FAM/BHQ-1 (88%-93%) und Cy5/BHQ-2 (91%-96%) bei optimierten Bedingungen überein.

*Mediator-Sonden-PCR vs. Hydrolyse-Sonden-PCR*

In den vorliegenden Versuchen wurden die Mediator-Sonden-PCR mit der Hydrolysen-Sonden-PCR verglichen. Zunächst wurden die Reaktionseffizienz, die LOD, die Interassay-Variation, die Intraassay-Variation, und die Duplexing Eigenschaften analysiert. Für diese Experimente wurden unterschiedliche Konzentrationen von HPV18-DNA ($10^2$, $10^3$, $10^4$, $10^5$, und $10^6$ Kopien pro Reaktion, wenn nicht anders beschrieben) amplifiziert, unter der parallelen Verwendung von beiden Techniken. Als zweites wurden unter der parallelen Verwendung von beiden Techniken unterschiedliche Targets amplifiziert.

*LOD (LIMIT OF DETECTION)*

Die LOD wurde als DNA-Konzentration resultierend in einer mit 95% wahrscheinlich positiven Amplifikation bestimmt. Probit-Analysen ergaben eine analytische Sensitivität von 78,3 Kopien pro Reaktion (95% CI: 47,0 -372,5 Kopien pro Reaktion) für die Mediator-Sonden-PCR und 85,1 Kopien pro Reaktion (95% CI: 55,7 - 209,4 Kopien pro Reaktion) für die Hydrolysen-Sonden-PCR (Fig.12A).

*Intraassy Varianz*

Fünf Konzentrationen der HPV18 DNA Verdünnungsreihe ($10^2$, $10^3$, $10^4$, $10^5$, und $10^6$ Kopien pro Reaktion) wurden in 8 Wiederholungen amplifiziert. Die $R^2$ Werte 0,975 (Mediator-Sonden-PCR) und 0,983 (Hydrolyse-Sonde-PCR) zeigen exzellente Linearität (Fig. 12B). Prozentuale CVs für die Amplifikationen von $10^2$-$10^6$ Kopien pro Reaktion waren 55,1%-9,9% (Mediator-Sonden-PCR) und 38,3%-10,7% (Hydrolyse-Sonden-PCR). Die Genauigkeit reicht von +21,6% bis - 8,1% (Mediator-Sonden-PCR) und von +19,4% bis - 9.8% (Hydrolyse-Sonden-PCR).

*Interassay Varianz*

Fünf separate vorbereitete Ansätze wurden für die Amplifikation von fünf Konzentrationen von HPV18 DNA Verdünnungsreihen ($10^2$, $10^3$, $10^4$, $10^5$, und $10^6$ Kopien pro Reaktion) verwendet. Jede Konzentration wurde dreifach angesetzt. Die $R^2$ Werte 0,940 (Mediator-Sonden-PCR) und 0,954 (Hydrolyse-Sonde-PCR) zeigen die Linearität der Amplifikation (Fig. 12C). Die Interassay-Varianz für Kopienzahlen von $10^2$-$10^6$ pro Reaktion lag zwischen 25% bis 8,7% (Mediator-Sonden-PCR) und zwischen 34,7% und 12,7% (Hydrolysen-Sonden-PCR). Die Genauigkeit reicht von +3,4% bis - 7% (Mediator-Sonden-PCR) und von - 2% bis - 12,4% (Hydrolyse-Sonden-PCR).

*Duplex Amplifikation*

Ein Fragment eines Plasmids, enthaltend HPV18 DNA ($10^2$, $10^3$, $10^4$, $10^5$, und $10^6$ ursprüngliche Kopien) wurde koamplifiziert mit 300 Kopien des Homo sapiens Genom. Die jeweiligen Reaktionen wurden im dreifachen Ansatz durchgeführt. Die Hydrolyse-Sonde für HPV18 wurde mit FAM/BHQ-1 und die Sonde für Actin, Beta(ACTB) mit Cy5/DDQ-2 gelabelt. Für die Duplex-PCR wurde das Nachweismolekül 01 mit FAM/Dabcyl und das Nachweismolekül 02 mit Cy5/BHQ-2 gelabelt. Fig. 12D zeigt die Liniarität der HPV18 Amplifikation für die unterschiedlichen DNA-Konzentrationen für die Mediator-Sonden-PCR ($R^2$ = 0,998) und für die Hydrolyse-Sonden-PCR ($R^2$ = 0,988). Die Berechnung der ACTB-Werte konnte nicht gezählt werden, da nur eine Konzentration in dem Duplex-Versuch eingesetzt wurde. Die Zykluswerte (cycle of quantification; Cq) wurden mit einem Schwellenwert von 0,02 im roten Kanal für die Mediator-Sonden-PCR und Hydrolysen-Sonden-PCR bestimmt. Die mittelern Cq Werte für koamplifizierte ACTB und HPV18-DNA-Proben waren 33,0 (0,5) und 31,8 (0,4) für die Mediator-Sonden-PCR und Hydrolysen-Sonden-PCR.

*Verschiedene Targets*

Die universelle Natur der Mediator-Sonden-PCR wurde durch Tests mit vier klinisch relevanten Targets verdeutlicht. Für den Vergleich wurde für jedes Target die Hydrolysen-Sonden-PCR parallel durchgeführt. Die Linearität zwischen Input und berechneter Output Kopienzahl wurde für jedes Target und jede Amplifikationstechnik bestimmt (Fig.13). Die Ergebnisse für die Detektion der Verdünnungsreihe des HPV-18 L1 Gens (Mediator-Sonden-PCR $R^2$ = 0,999/ Hydrolysen-Sonden-PCR $R^2$ = 0.975), S. aureus exfoliative Toxin B Gen (0,991/0,988), E. coli Peptidoglycan assoziierte Lipoprotein (E. coli pal) Gen (0,996/0,988), und Human β-Actin Gen (0,991/0,993) zeigen eine hohe Übereinstimmung zwischen den beiden PCRs:

| Target | Input copy number, n | Mediator probe PCR | | | Hydrolysis probe PCR | | |
|---|---|---|---|---|---|---|---|
| | | Output, n | SD | % CV | Output, n | SD | % CV |
| *HPV18 L1* | $1.0 \times 10^5$ | $1.1 \times 10^5$ | $4.2 \times 10^3$ | 4.0 | $1.1 \times 10^5$ | $4.1 \times 10^3$ | 3.8 |
| | $1.0 \times 10^4$ | $9.1 \times 10^3$ | $3.6 \times 10^2$ | 4.0 | $1.0 \times 10^4$ | $1.5 \times 10^3$ | 14.6 |
| | $1.0 \times 10^3$ | $1.0 \times 10^3$ | $5.9 \times 10^2$ | 5.8 | $8.7 \times 10^2$ | $4.4 \times 10^2$ | 50.9 |
| | $1.0 \times 10^2$ | $1.0 \times 10^2$ | $1.4 \times 10^1$ | 13.2 | $1.3 \times 10^2$ | $5.1 \times 10^1$ | 39.0 |
| *E. coli pal* | $6.3 \times 10^4$ | $5.5 \times 10^4$ | $1.1 \times 10^3$ | 1.9 | $6.4 \times 10^4$ | $5.6 \times 10^3$ | 8.9 |
| | $6.3 \times 10^3$ | $7.1 \times 10^3$ | $5.3 \times 10^2$ | 7.5 | $7.3 \times 10^3$ | $3.2 \times 10^2$ | 4.3 |
| | $6.3 \times 10^2$ | $6.7 \times 10^2$ | $40.7 \times 10^1$ | 6.1 | $5.9 \times 10^2$ | $1.4 \times 10^2$ | 23.2 |
| | $6.3 \times 10^1$ | $7.1 \times 10^1$ | $20.7 \times 10^1$ | 29.2 | $5.1 \times 10^1$ | $20.5 \times 10^1$ | 40.2 |
| *S. aureus ExfB* | $3.0 \times 10^4$ | $2.9 \times 10^4$ | $2.6 \times 10^3$ | 0.9 | $3.0 \times 10^4$ | $6.8 \times 10^3$ | 0.9 |
| | $3.0 \times 10^3$ | $4.7 \times 10^3$ | $3.9 \times 10^3$ | 8.4 | $3.8 \times 10^3$ | $2.5 \times 10^2$ | 6.7 |
| | $3.0 \times 10^2$ | $3.3 \times 10^2$ | $3.1 \times 10^1$ | 9.4 | $4.0 \times 10^2$ | $20.8 \times 10^1$ | 5.2 |
| | $3.0 \times 10^1$ | $3.8 \times 10^1$ | $2.4 \times 10^0$ | 6.3 | $4.0 \times 10^1$ | $3.1 \times 10^0$ | 7.8 |
| | $3.0 \times 10^0$ | $3.2 \times 10^0$ | $2.0 \times 10^0$ | 62.5 | $2.9 \times 10^0$ | $2.6 \times 10^0$ | 89.7 |
| *H. sapiens ACTB* | $4.0 \times 10^3$ | $2.9 \times 10^3$ | $1.6 \times 10^2$ | 5.4 | $3.6 \times 10^3$ | $3.4 \times 10^2$ | 9.4 |
| | $4.0 \times 10^2$ | $4.9 \times 10^2$ | $7.8 \times 10^1$ | 15.8 | $4.8 \times 10^2$ | $1.2 \times 10^2$ | 25.0 |
| | $4.0 \times 10^1$ | $4.3 \times 10^1$ | $5.2 \times 10^0$ | 12.1 | $2.8 \times 10^1$ | $1.6 \times 10^0$ | 5.7 |
| | $4.0 \times 10^0$ | $4.1 \times 10^0$ | $1.1 \times 10^0$ | 26.8 | $4.6 \times 10^1$ | $1.2 \times 10^0$ | 26.1 |
| Coamplification | | | | | | | |
| HPV18 L1 | $1.0 \times 10^6$ | $1.1 \times 10^6$ | $3.5 \times 10^4$ | 3.4 | $1.0 \times 10^6$ | $9.3 \times 10^4$ | 9.1 |
| | $1.0 \times 10^5$ | $8.1 \times 10^4$ | $6.9 \times 10^3$ | 8.5 | $1.2 \times 10^5$ | $2.2 \times 10^4$ | 18.9 |
| | $1.0 \times 10^4$ | $1.2 \times 10^4$ | $1.7 \times 10^3$ | 15.1 | $7.9 \times 10^3$ | $6.1 \times 10^2$ | 7.8 |
| | $1.0 \times 10^3$ | $1.1 \times 10^3$ | $7.7 \times 10^1$ | 6.7 | $1.0 \times 10^3$ | $3.1 \times 10^2$ | 30.3 |
| | $1.0 \times 10^2$ | $9.6 \times 10^1$ | $3.5 \times 10^1$ | 36.8 | $1.2 \times 10^2$ | $5.6 \times 10^1$ | 45.5 |
| *H. sapiens* ACTB[b] | $3.0 \times 10^2$ | $C_q$: 33.0 | ±0.5 | | $C_q$: 31.8 | ±0.4 | |

[a] Calculated copy numbers (no. output) of 4 targets amplified with mediator probe PCR and hydrolysis probe PCR. SD and imprecision (% CV) were calculated for each target and copy number.
[b] Quantification of copy number is not feasible. The threshold for ACTB was set to 0.02 and obtained $C_q$ values are presented.

*Diskussion:*

Das herausragende Merkmal der gezeigten Versuche ist die Entkopplung von Amplifikation und Fluoreszenz-Detektion, welche es erlaubt ein standardisiertes Nachweismolekül einzusetzen. Die Sequenz der Mediator-Region und des Nachweismoleküls wurden in silico designt und zeigen laut einer BLAST-Suche keinerlei Übereinstimmung mit den Targets. Das Nachweimolekül zeigt eine Haarnadel-Sekundärstruktur und stellt dadurch optimale FRET-Quenching Bedingungen bereit [>90% (FAM/Dabcyl),>80% (Cy5/BHQ-2)]. Die räumliche Nähe zwischen Fluorophor und Quencher innerhalb der Haarnadelstruktur resultieren in einer hohen und konstanten Quenching-Effizienz. Im Gegensatz zu den hier gezeigten Ergebnissen erzielen FAM-gelabelte Hydrolyse-Sonden aus dem Stand der Technik aufgrund der unterschiedlichen Quenching-Reste und Entfernungen von Donor und Akzeptor regelmäßig Quenching-Effizienzen von 60% bis 93%. Die Cy5/DDQ-2 gelabelte Hydrolyse-Sonde zeigte einen niedrigen Eq Wert von nur 55%.

Die Amplifizierung von HPV18 DNA wurde als Model-Assay ausgewählt um die neue Mediator-Sonde und die Hydrolyse-Sonde, den Goldstandard aus dem Stand der Technik mit einander zu vergleichen.

Die LOD wurde für beide Sonden mit Probit Analysen bestimmt (Mediatos-Sonde: 78,3; Hydrolyse-Sonde: 85,1 Kopien pro Reaktion). Inter- und Intraassay Varianz lagen für $10^2$ bis $10^6$ Kopien pro Reaktion in dergleichen Größenordnung (Mediator-Sonde 25%-8,7%, 55,1%-9,9%; Hydrolyse-Sonde 34,7%-12,7%, 38,3%-10,7%).

Eine Reduktion der Elongationszeit in verschiedenen PCR-Versuchen von 50 s auf 6 s hat die Quantifizierung nicht beeinträchtigt. Diese Ergebnisse zeigen, dass die Mediator-Sonden-PCR für rapid cycling Protokolle, welche mit aktuellsten real-time Thermocyclern durchgeführt werden können, geeignet ist

Es wurden zwei unterschiedliche Nachweismoleküle mit unterschiedlichen Hybridisierungssequenzen und FRET Modifikationen designt. Diese Reportersysteme sind in der Lage jede Target-Gen-Kombination zu detektieren, wobei das System Kosten spart und als Routine-Diagnose-Test eingesetzt werden kann. So konnte die Koamplifizierung von unterschiedlichen Mengen an HPV18-DNA und einer Konstanten Kopienzahl von ACTB erfolgreich gezeigt werden.

**Patentansprüche**

1. Verfahren zur Detektion von mindestens einem Zielmolekül, umfassend folgende Schritte:

   a) Bereitstellen einer Mediator-Sonde zur Detektion von mindestens einem Zielmolekül umfassend eine Sonden-Region und eine Mediator-Region, wobei die Mediator-Sonde ein Oligonukleotid ist und die Sonden-Region am 3'-Terminus und die Mediator-Region am 5'-Terminus des Oligonukleotides angeordnet ist, wobei zwischen den Regionen eine chemische, biologische und/oder physikalische Spaltstelle vorliegt und die Sonden-Region eine Affinität zu einem Vorlagemolekül und die Mediator-Region eine weitere Affinität zu einem Nachweismolekül aufweist,
   b) Bereitstellen eines Nachweismoleküls, wobei das Nachweismolekül ein Oligonukleotid ist und mindestens folgende Regionen aufweist:

      • eine erste Region an einem 5'-Terminus des Nachweismoleküls, die einen Fluoreszenzakzeptor oder einen Fluoreszenzdonor und/oder eine chemische Gruppe zur Bindung an eine Festphase und/oder eine chemische Schutzgruppe aufweist,
      • eine zweite Region, die mit der Mediator-Region interagiert und
      • eine dritte Region, die einen Fluoreszenzdonor oder einen Fluoreszenzakzeptor und/oder eine chemische Schutzgruppe aufweist,

   c) Bindung der Sonden-Region der Mediator-Sonde an eine Sequenz des Vorlagemoleküls und/oder des Zielmoleküls,
   d) Amplifikation des Vorlagemoleküls und/oder des Zielmoleküls mit Hilfe eines Hilfsmoleküls, bevorzugt durch einen Amplifikationsvorgang beinhaltend ein isothermales Verfahren und/oder Thermocycling-basiertes Verfahren und/oder durch eine PCR, besonders bevorzugt eine Real-time PCR,
   e) Spaltung der Mediator-Sonde an der Spaltstelle während Schritt d) durch das Hilfsmolekül aus Schritt d) oder weitere Hilfsmoleküle, und
   f) Bindung der abgespaltenen Mediator-Region der Mediator-Sonde an das Nachweismolekül,
   g) Signaldetektion.

2. Verfahren nach Anspruch 1, **dadurch gekennzeichnet, dass** das Zielmolekül das Vorlagemolekül ist und/oder das Zielmolekül an das Vorlagemolekül bindet und/oder die Sonden-Region und die Mediator-Region funktionell und/oder räumlich überlappen, bevorzugt mit einem Nukleotid und/oder an dem 3' Ende der Sonden-Region eine Lock-Region angeordnet ist, die eine komplementäre Sequenz zu der Mediator-Region aufweist und/oder die Mediator-Sonde am 3'-Terminus eine chemische Schutzgruppe aufweist.

3. Verfahren nach Anspruch 1 oder 2, **dadurch gekennzeichnet, dass** das Nachweismolekül eine vierte Region an einem 3'-Terminus des Nachweismoleküls, die eine chemische Gruppe zur Bindung an eine Festphase und/oder eine chemische Schutzgruppe aufweist.

4. Verfahren nach einem oder mehreren der vorhergehenden Ansprüche, **dadurch gekennzeichnet, dass** die Mediator-Sonde und/oder das Nachweismolekül fluoreszenzmarkierte Nukleotide aufweisen.

5. Verfahren nach einem oder mehreren der vorhergehenden Ansprüche, **dadurch gekennzeichnet, dass**

das Nachweismolekül ein einzelsträngiges Nukleinsäuremolekül oder Nukleinsäurederivat ist.

6. Verfahren nach einem oder mehreren der vorhergehenden Ansprüche, **dadurch gekennzeichnet, dass**

das Nachweismolekül eine Haarnadel-Struktur aufweist, und/oder
das Nachweismolekül mindestens eine Fluoreszenz-Modifikationen am 5'-Terminus und/oder innerhalb der Haarnadelstruktur aufweist.

7. Verfahren nach dem vorhergehenden Anspruch,
**dadurch gekennzeichnet, dass**
die Haarnadel-Struktur ausgebildet ist, indem der 5'-Terminus des Nachweismoleküls mit einem internen Sequenzabschnitt komplementär hybridisiert und der 3'-Terminus des Nachweismoleküls einen ungepaarten Sequenzabschnitt umfasst.

8. Verfahren nach einem oder mehreren der vorhergehenden Ansprüche ,
**dadurch gekennzeichnet, dass**
das Zielmolekül und/oder das Vorlagemolekül ein Biomolekül ist, ausgewählt aus der Gruppe umfassend DNA, RNA, Peptid, Protein, Aptamer und/oder einer Kombination hieraus, bevorzugt wobei
mindestens ein Zielmolekül eine RNA ist und die RNA in cDNA umgeschrieben wird und wobei die cDNA das Vorlagemolekül ist und/oder
mindestens ein Zielmolekül ein Peptid oder ein Protein ist und das Vorlagemolekül ein Aptamer ist und wobei das Aptamer an das Peptid oder das Protein bindet und dabei die Bindestelle für die Sonden-Region der Mediator-Sonde zugänglich wird.

9. Verfahren nach einem oder mehreren der vorhergehenden Ansprüche ,
**dadurch gekennzeichnet, dass**
ein Primer mit Sequenzüberhang für die Umschreibereaktion eingesetzt wird und die Sonden-Region der Mediator-Sonde an einen Bereich umfassend cDNA und Überhangsequenz bindet.

10. Verfahren nach einem oder mehreren der vorhergehenden Ansprüche, **dadurch gekennzeichnet, dass**
die Spaltung der Mediator-Sonde eine Veränderung der physikalischen und/oder chemischen Eigenschaften mindestens einer Region der Mediator-Sonde bewirkt, ausgewählt aus der Gruppe umfassend molekulare Masse, enzymatische Aktivität, Bindungseigenschaften umfassend Affinität oder Avidität, chemische Reaktivität, Vorhandensein chemischer Gruppen, elektrischer Eigenschaft umfassend Leitfähigkeit, Polarisierbarkeit oder Ladung und/oder optischer Eigenschaft umfassend Absorption und Emission von Licht.

11. Verfahren nach einem oder mehreren der vorhergehenden Ansprüche,
**dadurch gekennzeichnet, dass**
die Mediator-Region an die zweite Region des Nachweismoleküls bindet.

12. Verfahren nach einem oder mehreren der vorhergehenden Ansprüche,
**dadurch gekennzeichnet, dass**
die an die zweite Region des Nachweismoleküls gebundene Mediator-Region durch mindestens ein Hilfsmolekül enzymatisch verlängert wird, wobei das Hilfsmolekül bevorzugt an den 3'-Terminus der gebundenen Mediator-Region bindet und wobei das Hilfsmolekül bevorzugt ausgewählt ist aus der Gruppe umfassend Katalysatoren, Proteine, Nukleinsäuren, Naturstoffe, Enzyme, Enzym-Systeme, Zell-Lysate, Zell-Bestandteile, aus Zell-Bestandteilen abgeleitete Derivate und/oder synthetische Moleküle und/oder
ein Molekül aus einem Nukleinsäureamplifikationssystem und/oder einem Restriktionsenzymsystem ist.

13. Verfahren nach einem oder mehreren der vorhergehenden Ansprüche,
**dadurch gekennzeichnet, dass**
das Nachweismolekül in Folge einer direkten oder indirekten Interaktion mit der Mediator-Region verändert wird, umfassend Änderung der Sekundärstruktur, Änderung in Fluoreszenz, Phosphoreszenz, Masse, Absorption, Streuung des Lichts, elektrischen Leitfähigkeit, enzymatische Aktivität und/oder der Affinität und/oder wobei
durch eine indirekte oder direkte Interaktion der Mediator-Region mit der zweiten Region des Nachweismoleküls eine physikalisch oder chemisch messbare Veränderung des Nachweismoleküls erfolgt.

14. Verfahren nach einem oder mehreren der vorhergehenden Ansprüche,

**dadurch gekennzeichnet, dass**
sequenzspezifische oder -unspezifische, fluorogene und/oder chromogene Sonden oder Fluoreszenzfarbstoffe mit mindestens einer Region der Mediator-Sonde und/oder dem Nachweismolekül interagieren.

**15.** Verfahren nach einem oder mehreren der vorhergehenden Ansprüche,
**dadurch gekennzeichnet, dass**
das Nachweismolekül durch eine Interaktion mit der Mediator-Region durch mindestens ein Nachweishilfsmoleküle verändert wird, umfassend Spaltung, Verdau, Strangverdoppelung, interne Hybridisierung, Phosphorylierung, Dephosphorylierung, Amidierung, Anbindung oder Abspaltung einer chemischen Gruppe, Fluoreszenz-, Phosphoreszenz- oder Lumineszenzänderung, wobei bevorzugt das Nachweismolekül über mindestens eine Fluoreszenz-Modifikationen am 5'-terminalen Bereich und/oder innerhalb der Haarnadelstruktur verfügt und nach der Reaktion mit der Mediator-Region mittels eines Nachweishilfsmoleküls von dem Nachweismolekül die Fluoreszenz-Modifikationen abgespalten und/oder der 5'-Terminus der Haarnadelstruktur des Nachweismoleküls entfernt und/oder die Haarnadelstruktur entfaltet wird und eine Änderung des Fluoreszenzsignals am Nachweismolekül detektiert wird.

## Claims

**1.** A method for the detection of at least one target molecule, comprising the following steps:

a) providing a mediator probe for the detection of at least one target molecule comprising a probe region and a mediator region, wherein the mediator probe is an oligonucleotide and the probe region is located at the 3' terminus and the mediator region is located at the 5' terminus of the oligonucleotide, wherein there is a chemical, biological and/or physical cleavage site between the regions, and the probe region has affinity for a template molecule and the mediator region has another affinity for a detection molecule,
b) providing a detection molecule, wherein the detection molecule is an oligonucleotide and has at least the following regions:

• a first region at a 5' terminus of the detection molecule, said first region having a fluorescence acceptor or a fluorescence donor and/or a chemical group for binding to a solid phase and/or a chemical protecting group,
• a second region interacting with the mediator region, and
• a third region having a fluorescence donor or a fluorescence acceptor and/or a chemical protecting group,

c) binding the probe region of the mediator probe to a sequence of the template molecule and/or the target molecule,
d) amplifying the template molecule and/or the target molecule by means of a helper molecule, preferably by an amplification process including an isothermal method and/or thermocycling-based method and/or by a PCR, particularly preferably a real-time PCR,
e) cleaving the mediator probe at the cleavage site during step d) by the helper molecule from step d) or further helper molecules, and
f) binding the cleaved mediator region of the mediator probe to the detection molecule,
g) detecting a signal.

**2.** The method according to claim 1,
**characterized in that**
the target molecule is the template molecule and/or the target molecule binds to the template molecule and/or the probe region to the mediator region overlap functionally and/or spatially, preferably with a nucleotide and/or a lock region is arranged at the 3' end of the probe region, said lock region having a complementary sequence to the mediator region and/or the mediator probe has a chemical protecting group at the 3' terminus.

**3.** The method according to claim 1 or 2,
**characterized in that**
the detection molecule has a fourth region at a 3'-terminus of the detection molecule, said fourth region having a chemical group for binding to a solid phase and/or a chemical protecting group.

**4.** The method according to any one or more of the preceding claims,
**characterized in that**

the mediator probe and/or the detection molecule have fluorescently labeled nucleotides.

5. The method according to any one or more of the preceding claims,
**characterized in that**
the detection molecule is a single-stranded nucleic acid molecule or nucleic acid derivative.

6. The method according to any one or more of the preceding claims,
**characterized in that**
the detection molecule has a hairpin structure, and/or
the detection molecule has at least one fluorescence modification at the 5' terminus and/or within the hairpin structure.

7. The method according to the preceding claim, **characterized in that**
the hairpin structure is formed by complementarily hybridizing the 5' terminus of the detection molecule to an internal sequence segment, and the 3' terminus of the detection molecule comprising an unpaired sequence segment.

8. The method according to any one or more of the preceding claims,
**characterized in that**
the target molecule and/or the template molecule is a biomolecule selected from the group comprising DNA, RNA, peptide, protein, aptamer and/or a combination thereof, preferably where
at least one target molecule is an RNA and the RNA is transcribed into cDNA and wherein the cDNA is the template molecule and/or
at least one target molecule is a peptide or a protein, and the template molecule is an aptamer, and wherein the aptamer binds to the peptide or protein, thereby making the binding site accessible to the probe region of the mediator probe.

9. The method according to any one or more of the preceding claims,
**characterized in that**
a primer with sticky end sequence is used for the transcription reaction, and the probe region of the mediator probe binds to an area comprising cDNA and sticky end sequence.

10. The method according to any one or more of the preceding claims,
**characterized in that**
the cleavage of the mediator probe causes a change in the physical and/or chemical properties of at least one region of the mediator probe selected from the group comprising molecular mass, enzymatic activity, binding properties comprising affinity or avidity, chemical reactivity, presence of chemical groups, electrical property comprising conductivity, polarizability or charge and/or optical property comprising absorption and emission of light.

11. The method according to any one or more of the preceding claims,
**characterized in that**
the mediator region binds to the second region of the detection molecule.

12. The method according to any one or more of the preceding claims,
**characterized in that**
the mediator region bound to the second region of the detection molecule is enzymatically extended by at least one helper molecule, wherein the helper molecule preferably binds to the 3' terminus of the bound mediator region, and wherein the helper molecule is preferably selected from the group comprising catalysts, proteins, nucleic acids, natural products, enzymes, enzyme systems, cell lysates, cellular components, derivatives derived from cellular components and/or synthetic molecules, and/or
is a molecule from a nucleic acid amplification system and/or a restriction enzyme system.

13. The method according to any one or more of the preceding claims,
**characterized in that**
the detection molecule is changed as a result of direct or indirect interaction with the mediator region, comprising a change in the secondary structure, a change in fluorescence, phosphorescence, mass, absorption, scattering of light, electrical conductivity, enzymatic activity and/or affinity, and/or wherein
a physically or chemically measurable change of the detection molecule takes place by a direct or indirect interaction of the mediator region with the second region of the detection molecule.

**14.** The method according to any one or more of the preceding claims,
**characterized in that**
sequence-specific or -nonspecific, fluorogenic and/or chromogenic probes or fluorescent dyes interacting with at least one region of the mediator probe and/or the detection molecule.

**15.** The method according to any one or more of the preceding claims,
**characterized in that**
the detection molecule is changed by an interaction with the mediator region by at least one detection helper molecule, comprising cleavage, digestion, strand doubling, internal hybridization, phosphorylation, dephosphorylation, amidation, attachment or cleavage of a chemical group, fluorescence, phosphorescence or luminescence change, preferably wherein
the detection molecule has at least one fluorescence modification at the 5' terminal area and/or within the hairpin structure, and, after the reaction with the mediator region, the fluorescence modifications are cleaved off from the detection molecule by means of a detection helper molecule, and/or the 5' terminus of the hairpin structure of the detection molecule is removed, and/or the hairpin structure is unfolded, and a change of the fluorescence signal is detected at the detection molecule.

## Revendications

**1.** Procédé de détection d'au moins une molécule cible, comprenant les étapes suivantes :

a) mise à disposition d'une sonde médiatrice pour la détection d'au moins une molécule cible comprenant une région sonde et une région médiatrice, la sonde médiatrice étant un oligonucléotide et la région sonde étant agencée à l'extrémité 3' et la région médiatrice à l'extrémité 5' de l'oligonucléotide, dans lequel un site de clivage chimique, biologique et/ou physique est présent entre les régions et la région sonde présente une affinité avec une molécule de matrice et la région médiatrice présente une autre affinité avec une molécule de détection,
b) mise à disposition d'une molécule de détection, la molécule de détection étant un oligonucléotide et présente au moins les régions suivantes :

- une première région à une extrémité 5' de la molécule de détection, qui présente un accepteur de fluorescence ou un donneur de fluorescence et/ou un groupe chimique pour la liaison à une phase solide et/ou à un groupe protecteur chimique,
- une deuxième région, qui interagit avec la région médiatrice et
- une troisième région, qui présente un donneur de fluorescence ou un accepteur de fluorescence et/ou un groupe protecteur chimique,

c) liaison de la région de sonde de la sonde médiatrice à une séquence de la molécule de matrice et/ou de la molécule cible,
d) amplification de la molécule de matrice et/ou de la molécule cible à l'aide d'une molécule auxiliaire, de préférence par une opération d'amplification contenant un procédé isothermique et/ou un procédé basé sur le thermocyclage et/ou par une PCR, de manière particulièrement préférée une PCR en temps réel,
e) scission de la sonde médiatrice au niveau du site de clivage pendant l'étape d) par la molécule auxiliaire de l'étape d) ou d'autres molécules auxiliaires, et
f) liaison de la région médiatrice séparée de la sonde médiatrice à la molécule de détection,
g) détection de signal.

**2.** Procédé selon la revendication 1, **caractérisé en ce que** la molécule cible est la molécule de matrice et/ou la molécule cible se fixe à la molécule de matrice et/ou la région sonde et la région médiatrice se chevauchent de manière fonctionnelle et/ou spatiale, une région Lock est agencée de préférence avec un nucléotide et/ou à l'extrémité 3' de la région sonde, qui présente une séquence complémentaire à la région médiatrice et/ou la sonde médiatrice présente à l'extrémité 3' un groupe protecteur chimique.

**3.** Procédé selon la revendication 1 ou 2, **caractérisé en ce que** la molécule de détection présente une quatrième région à une extrémité 3' de la molécule de détection, qui présente un groupe chimique pour la liaison à une phase solide et/ou un groupe protecteur chimique.

**4.** Procédé selon une ou plusieurs des revendications précédentes,

**caractérisé en ce que**
la sonde médiatrice et/ou la molécule de détection présentent des nucléotides marqués par fluorescence.

5. Procédé selon une ou plusieurs des revendications précédentes,
**caractérisé en ce que**
la molécule de détection est une molécule d'acide nucléique monobrin ou un dérivé d'acide nucléique.

6. Procédé selon une ou plusieurs des revendications précédentes,
**caractérisé en ce que**
la molécule de détection présente une structure en épingle à cheveux, et/ou
la molécule de détection présente au moins une modification de fluorescence à l'extrémité 5' et/ou à l'intérieur de la structure en épingle à cheveux.

7. Procédé selon la revendication précédente,
**caractérisé en ce que**
la structure en épingle à cheveux est réalisée par le fait que l'extrémité 5' de la molécule de détection est hybridée de manière complémentaire avec un segment de séquence interne et l'extrémité 3' de la molécule de détection comprend un segment de séquence non apparié.

8. Procédé selon une ou plusieurs des revendications précédentes,
**caractérisé en ce que**
la molécule cible et/ou la molécule de matrice est une biomolécule, choisie dans le groupe comprenant un ADN, un ARN, un peptide, une protéine, un aptamère et/ou une combinaison de ceux-ci, de préférence dans lequel
au moins une molécule cible est un ARN et l'ARN est transcrit en ADNc et dans lequel l'ADNc est la molécule de matrice et/ou au moins une molécule cible est un peptide ou une protéine et la molécule de matrice est un aptamère et dans lequel l'aptamère se fixe au peptide ou à la protéine et le site de liaison est accessible ce faisant à la région sonde de la sonde médiatrice.

9. Procédé selon une ou plusieurs des revendications précédentes,
**caractérisé en ce que**
un primaire avec surplomb de séquence est utilisé pour la réaction de transcription et la région sonde de la sonde médiatrice se fixe à une zone comprenant un ADNc et une séquence de surplomb.

10. Procédé selon une ou plusieurs des revendications précédentes, **caractérisé en ce que**
la scission de la sonde médiatrice entraîne une modification des propriétés physiques et/ou chimiques d'au moins une région de la sonde médiatrice, choisie(s) dans le groupe comprenant la masse moléculaire, l'activité enzymatique, des propriétés de liaison comprenant l'affinité ou l'avidité, la réactivité chimique, la présence de groupes chimiques, d'une propriété électrique comprenant la conductibilité, la polarisabilité ou la charge et/ou d'une propriété optique comprenant l'absorption et l'émission de lumière.

11. Procédé selon une ou plusieurs des revendications précédentes,
**caractérisé en ce que**
la région médiatrice se fixe à la deuxième région de la molécule de détection.

12. Procédé selon une ou plusieurs des revendications précédentes,
**caractérisé en ce que**
la région médiatrice liée à la deuxième région de la molécule de détection est prolongée du point de vue enzymatique par au moins une molécule auxiliaire, dans lequel la molécule auxiliaire se fixe de préférence à l'extrémité 3' de la région médiatrice liée et dans lequel la molécule auxiliaire est sélectionnée de préférence dans le groupe comprenant des catalyseurs, des protéines, des acides nucléiques, des substances naturelles, des enzymes, des systèmes enzymatiques, des lysats de cellules, des composants de cellules, des dérivés de composants de cellules et/ou des molécules synthétiques et/ou
une molécule d'un système d'amplification d'acide nucléique et/ou d'un système enzymatique de restriction.

13. Procédé selon une ou plusieurs des revendications précédentes,
**caractérisé en ce que**
la molécule de détection est modifiée à la suite d'une interaction directe ou indirecte avec la région médiatrice, comprenant une modification de la structure secondaire, une modification de la fluorescence, de la phosphorescence, de la masse, de l'absorption, de la dispersion de la lumière, de la conductibilité électrique, de l'activité enzymatique et/ou de l'affinité et/ou dans lequel
une modification mesurable du point de vue physique ou chimique de la molécule de détection a lieu par une interaction directe ou indirecte de la région médiatrice avec la deuxième région de la molécule de détection.

**14.** Procédé selon une ou plusieurs des revendications précédentes,
**caractérisé en ce que**
des sondes spécifiques ou non spécifiques d'une séquence , fluorogènes et/ou chromogènes ou des colorants fluorescents interagissent avec au moins une région de la sonde médiatrice et/ou la molécule de détection.

**15.** Procédé selon une ou plusieurs des revendications précédentes,
**caractérisé en ce que**
la molécule de détection est modifiée par une interaction avec la région médiatrice par au moins une molécule auxiliaire de détection, comprenant la scission, la digestion, la duplication de brin, l'hybridation interne, la phosphorylation, la déphosphorylation, l'amidification, la liaison ou la séparation d'un groupe chimique, une modification de la fluorescence, de la phosphorescence ou de la luminescence, dans lequel de préférence
la molécule de détection dispose d'au moins une modification de fluorescence au niveau de la zone d'extrémité 5' et/ou à l'intérieur de la structure en épingle à cheveux et après la réaction avec la région médiatrice, les modifications de fluorescence sont séparées de la molécule de détection au moyen d'une molécule auxiliaire de détection et/ou l'extrémité 5' de la structure en épingle à cheveux de la molécule de détection est retirée et/ou la structure en épingle à cheveux est dépliée et une modification du signal de fluorescence est détectée au niveau de la molécule de détection.

Fig. 1

(A)

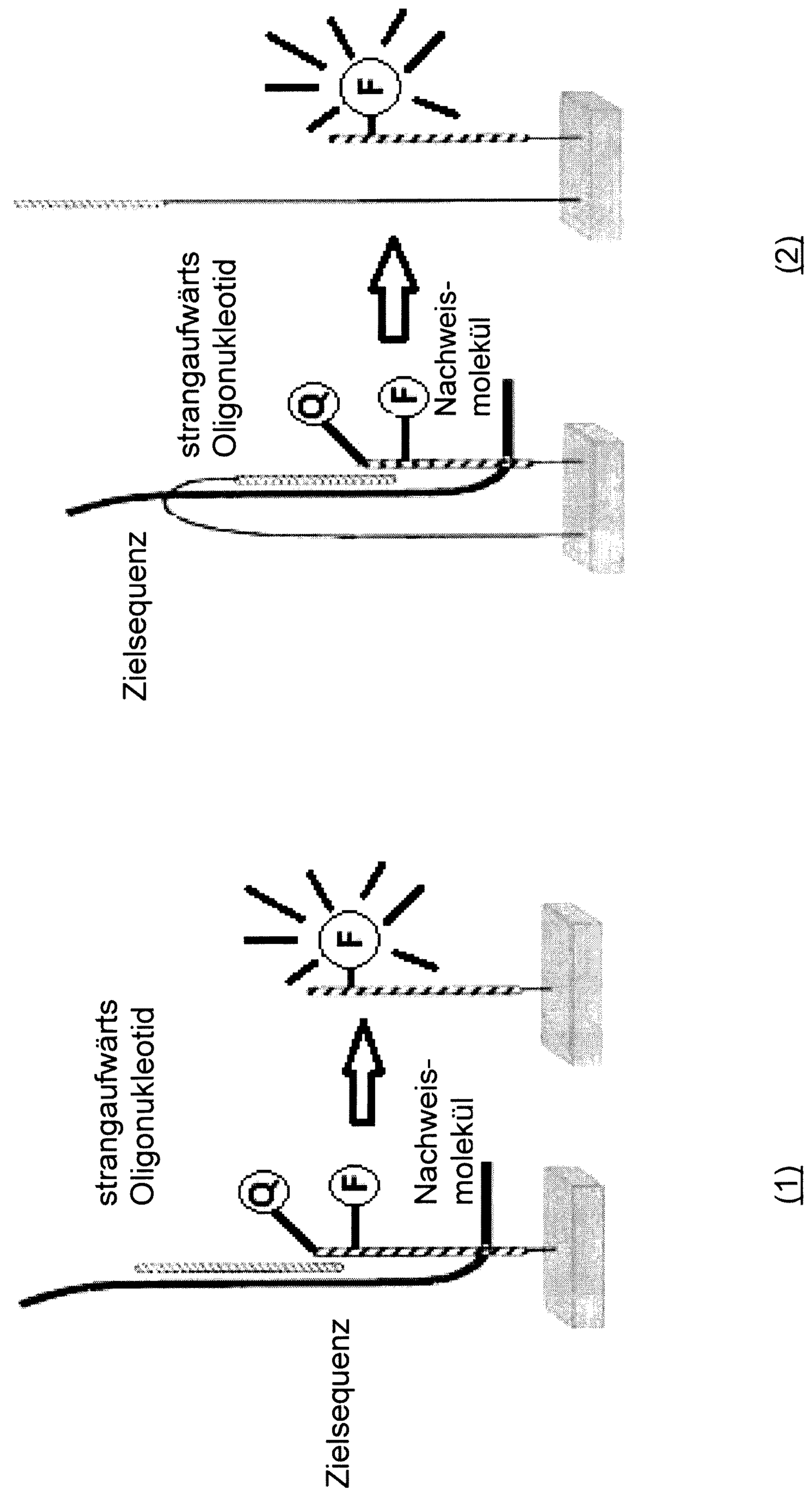
Zielsequenz
strangaufwärts
Oligonukleotid
Q
F
Nachweis-
molekül
F
(1)
Zielsequenz
strangaufwärts
Oligonukleotid
Q
F
Nachweis-
molekül
F
(2)

Fig. 1 (Fortsetzung)

(B)

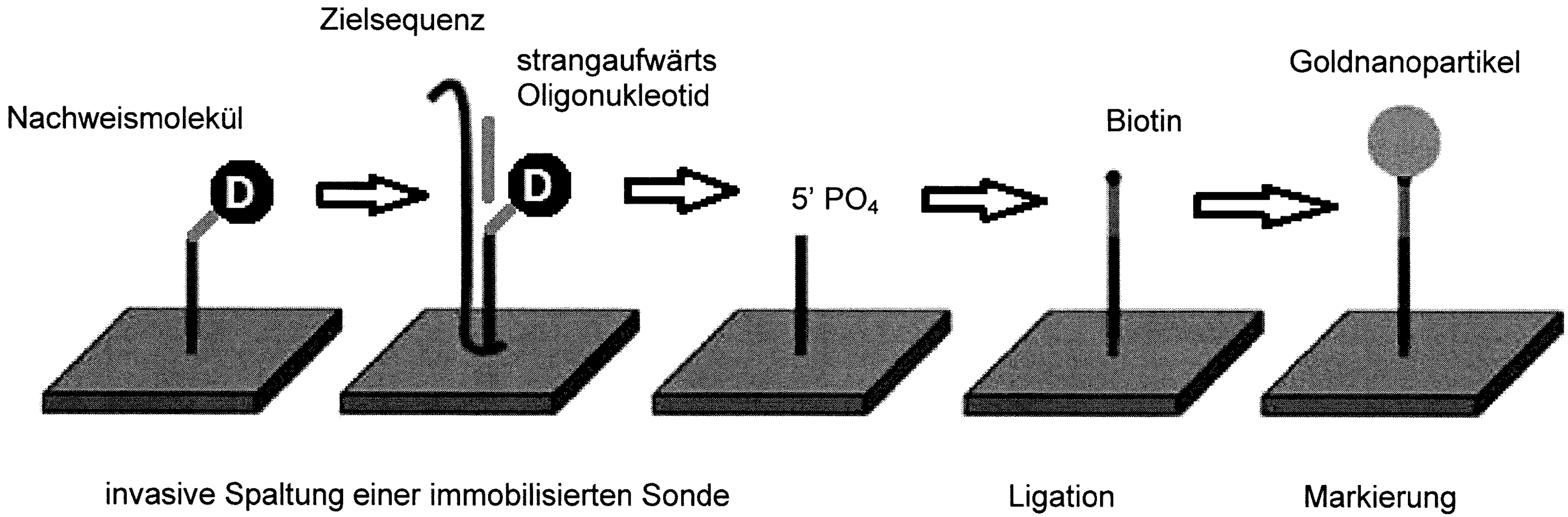

Fig. 1

(Fortsetzung)

(C)

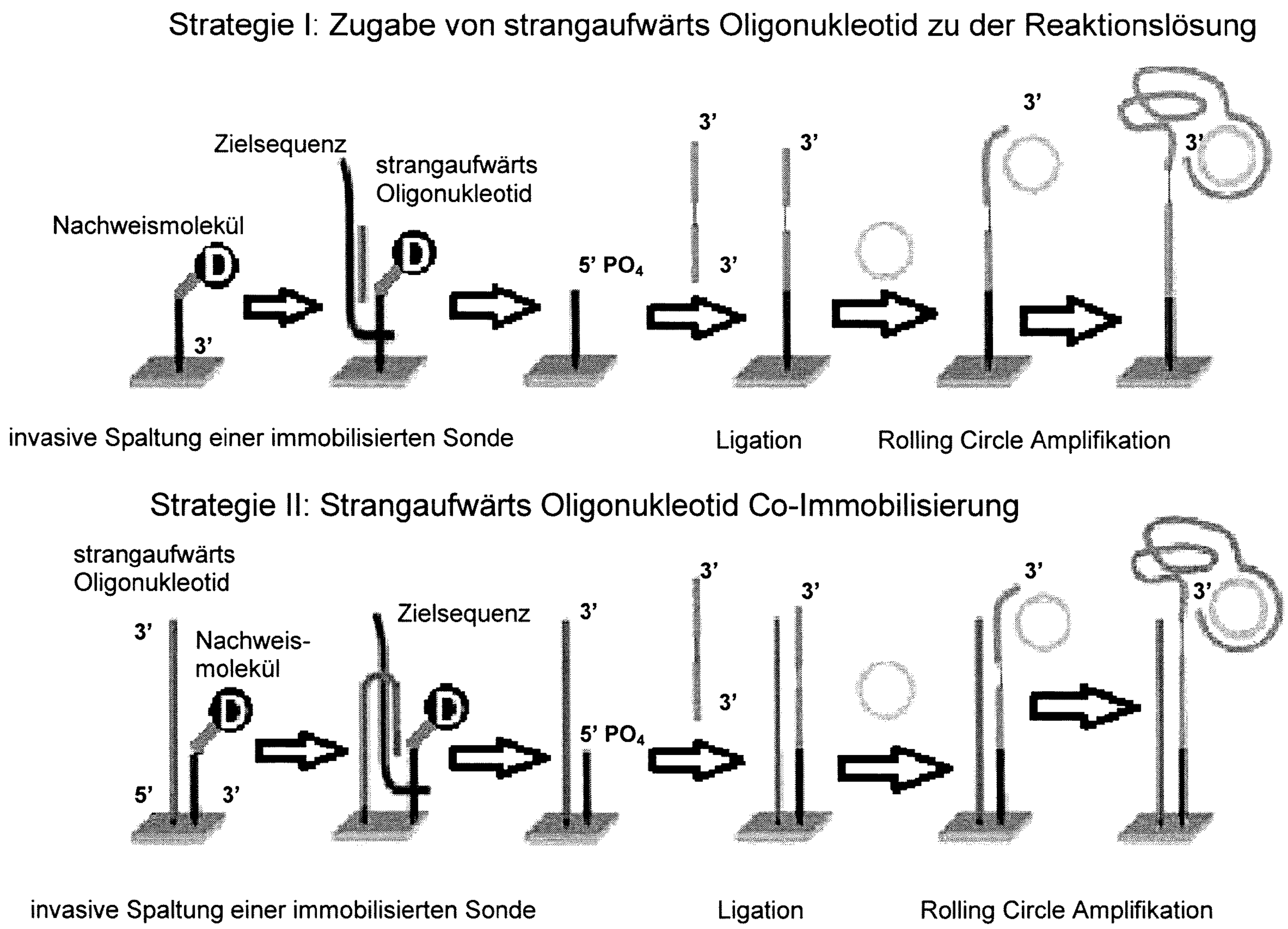

Fig.1 (Fortsetzung)

(D)

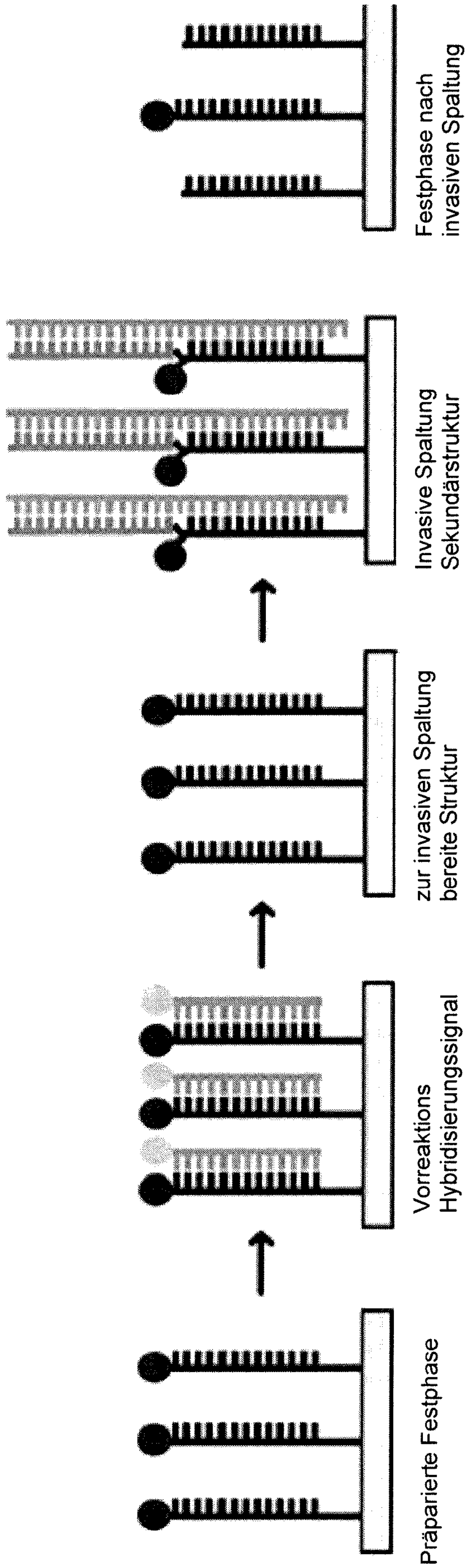

Fig. 2

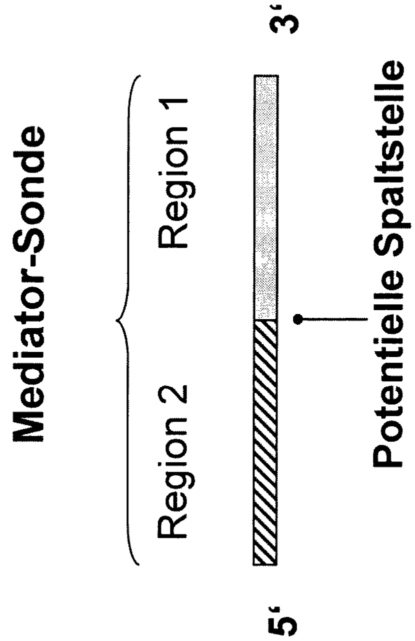
Mediator-Sonde
Region 2
Region 1
5'
3'
Potentielle Spaltstelle

Fig. 3

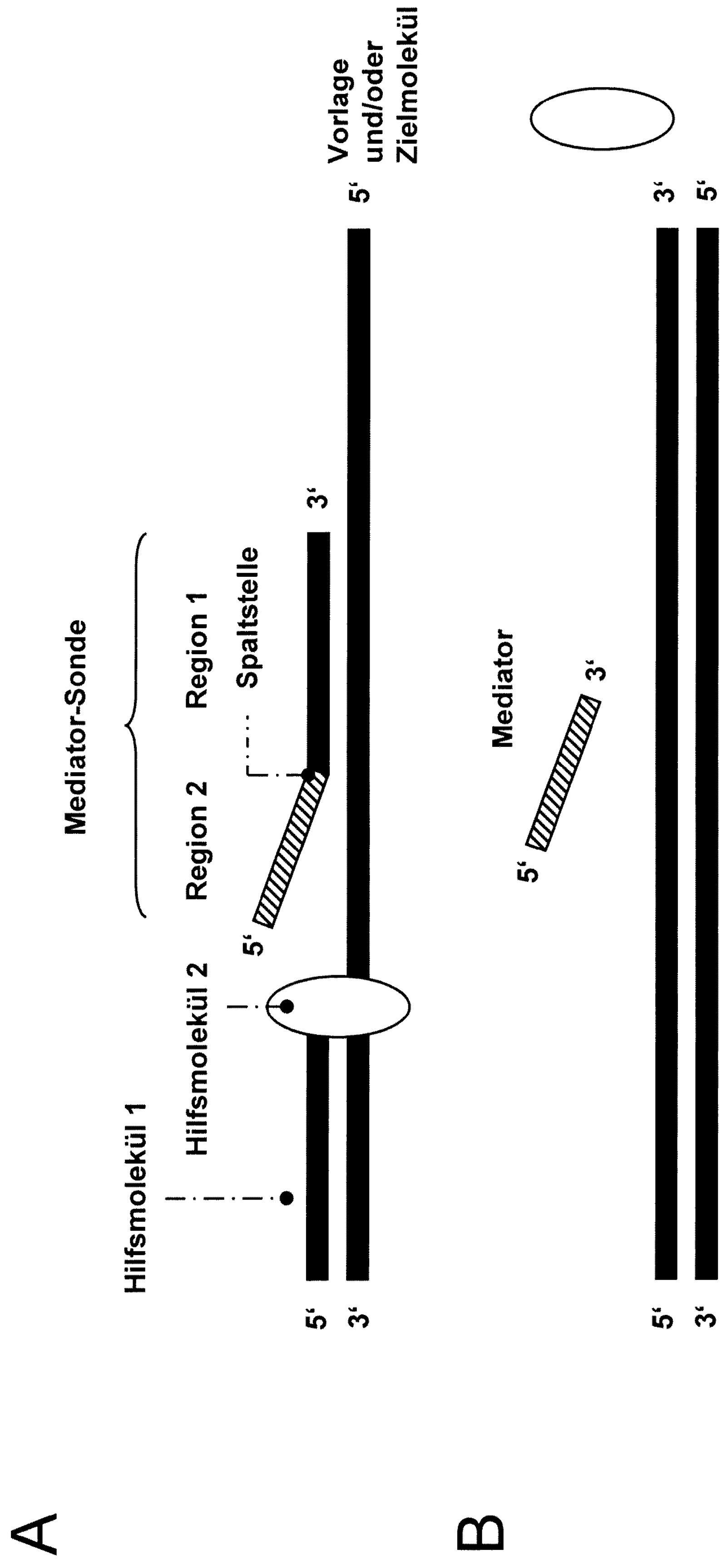
A
Hilfsmolekül 1
Hilfsmolekül 2
Mediator-Sonde
Region 2
Region 1
Spaltstelle
5'
3'
5'
3'
3'
5'
Vorlage
und/oder
Zielmolekül
B
Mediator
5'
3'
5'
3'
3'
5'

Fig. 4

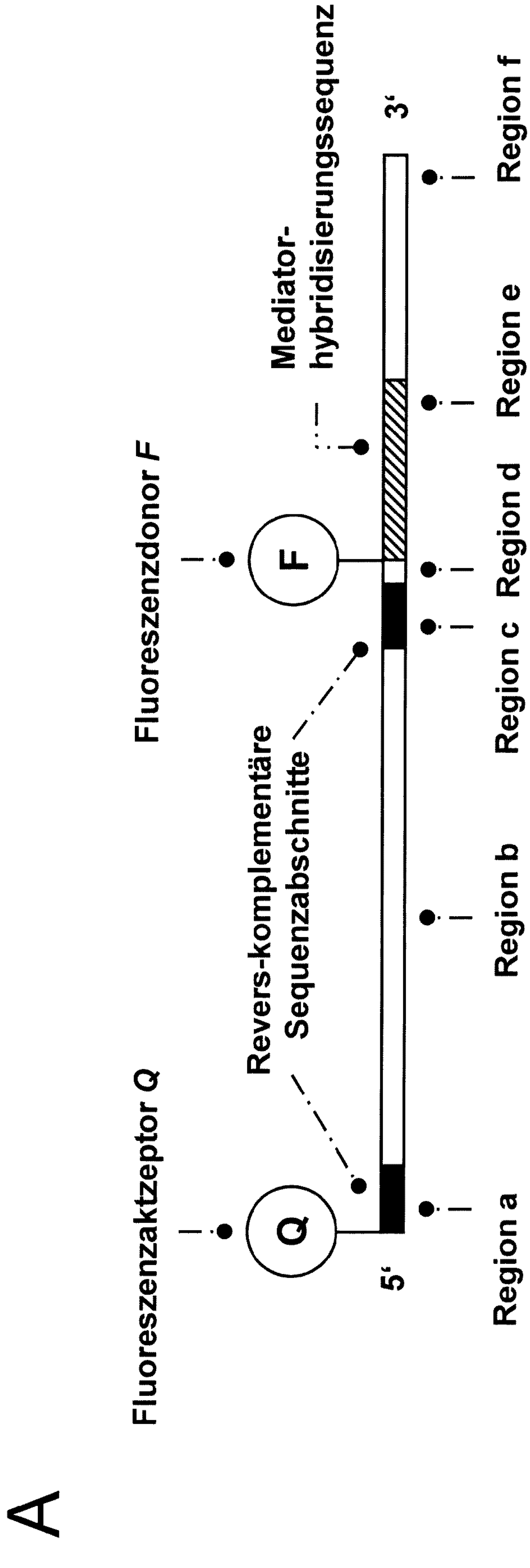
A
Fluoreszenzaktzeptor Q
Q
Revers-komplementäre Sequenzabschnitte
Fluoreszenzdonor F
F
Mediator-hybridisierungssequenz
5'
3'
Region a
Region b
Region c
Region d
Region e
Region f

Fig. 4 (Fortsetzung)

B

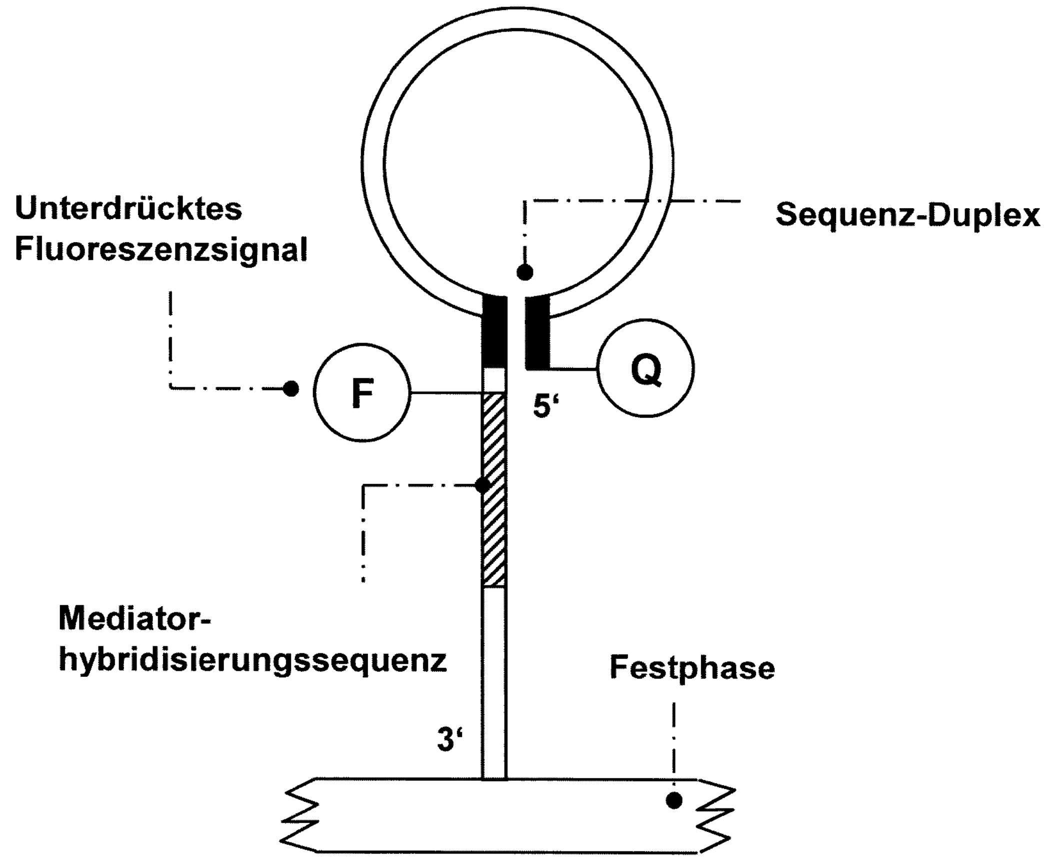

Fig. 5

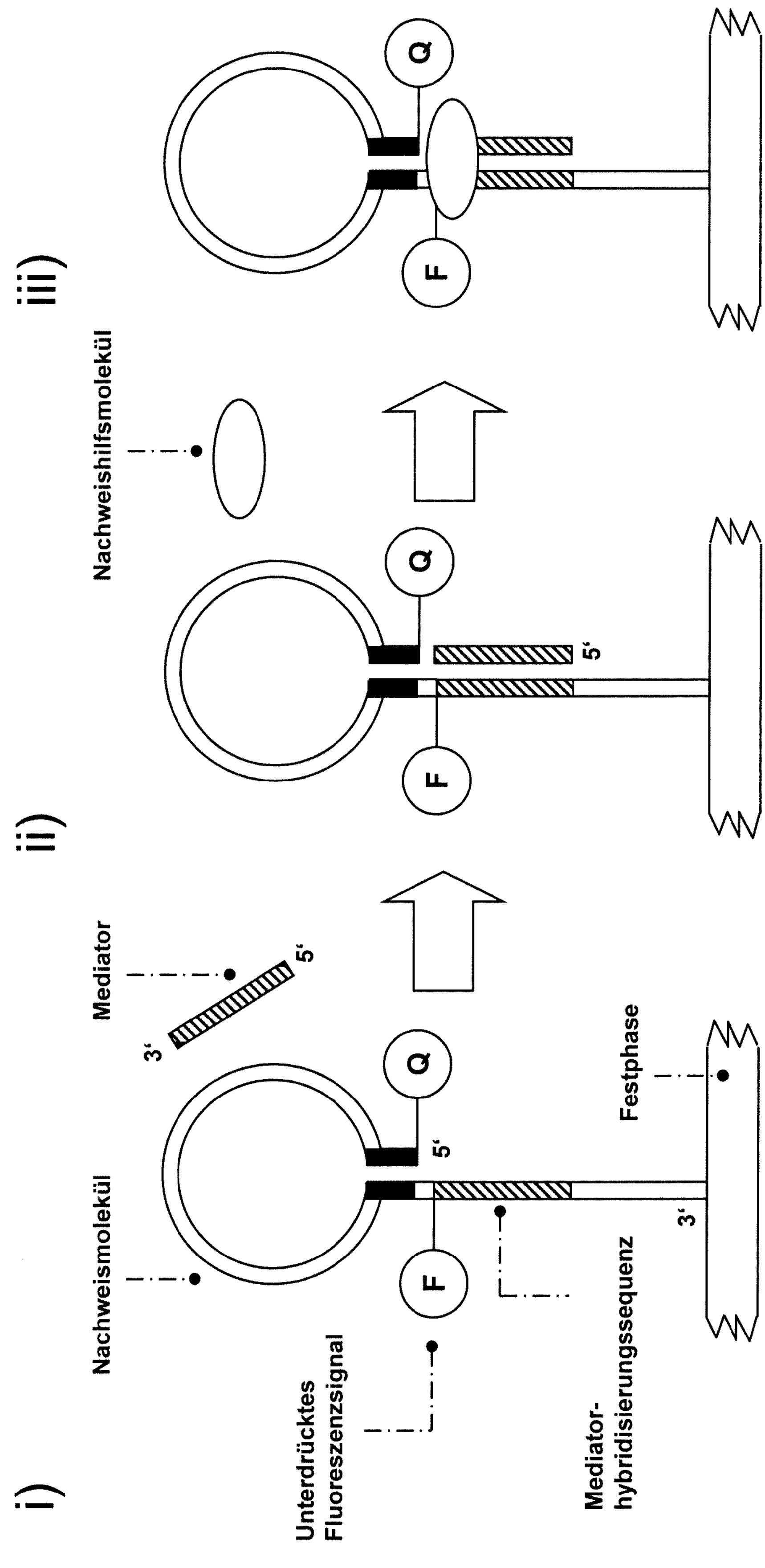
i)
ii)
iii)
Nachweismolekül
Mediator
Nachweishilfsmolekül
Unterdrücktes Fluoreszenzsignal
Mediator-hybridisierungssequenz
Festphase
F
Q
3'
5'

Fig. 5 (Fortsetzung)

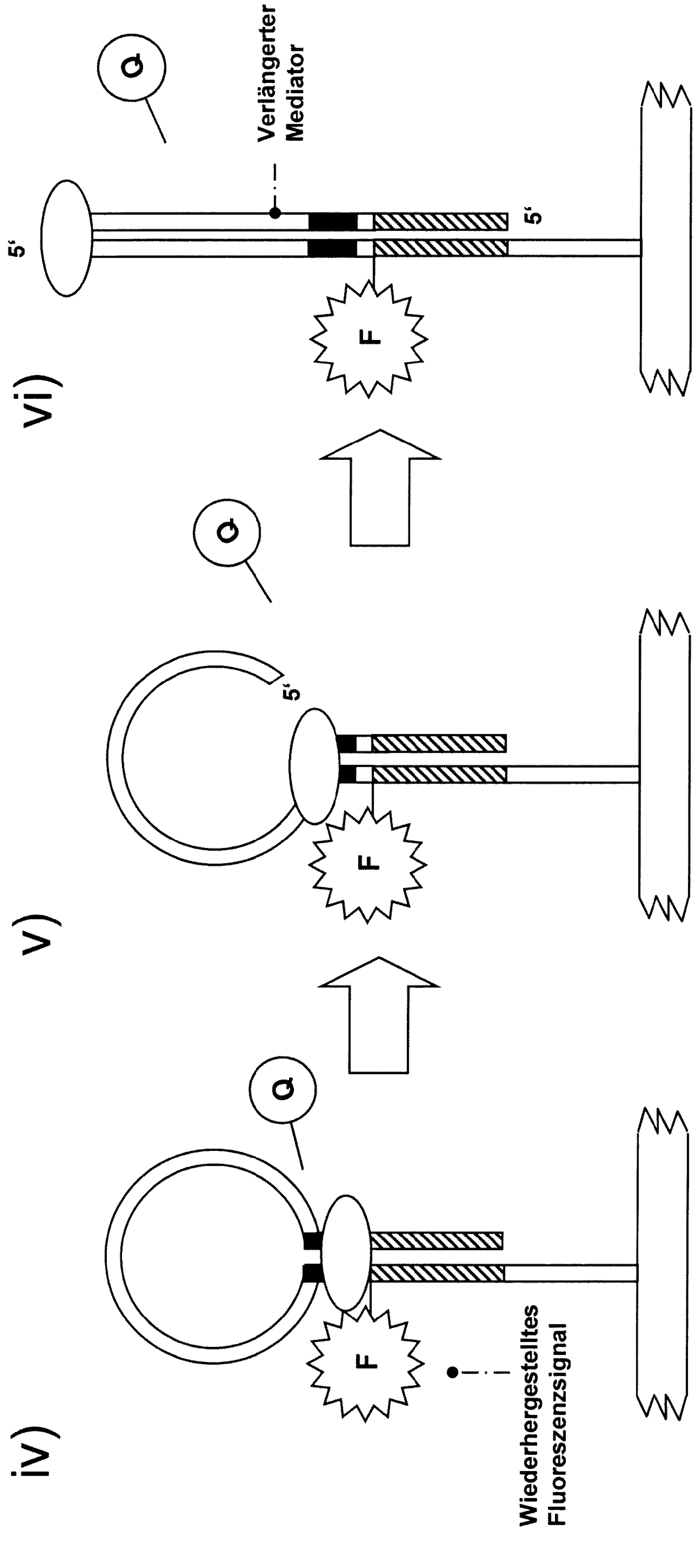

Fig. 6

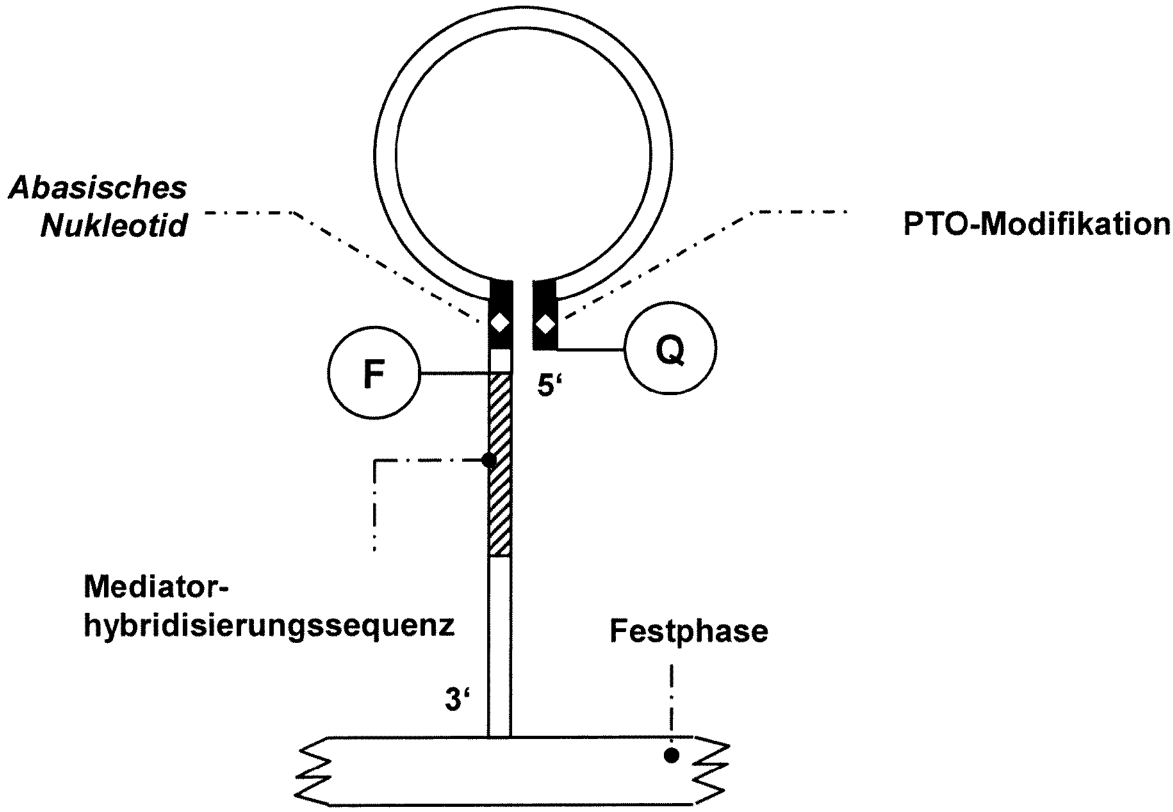
*Abasisches Nukleotid*
PTO-Modifikation
F
Q
5‘
Mediator-hybridisierungssequenz
Festphase
3‘

Fig. 7

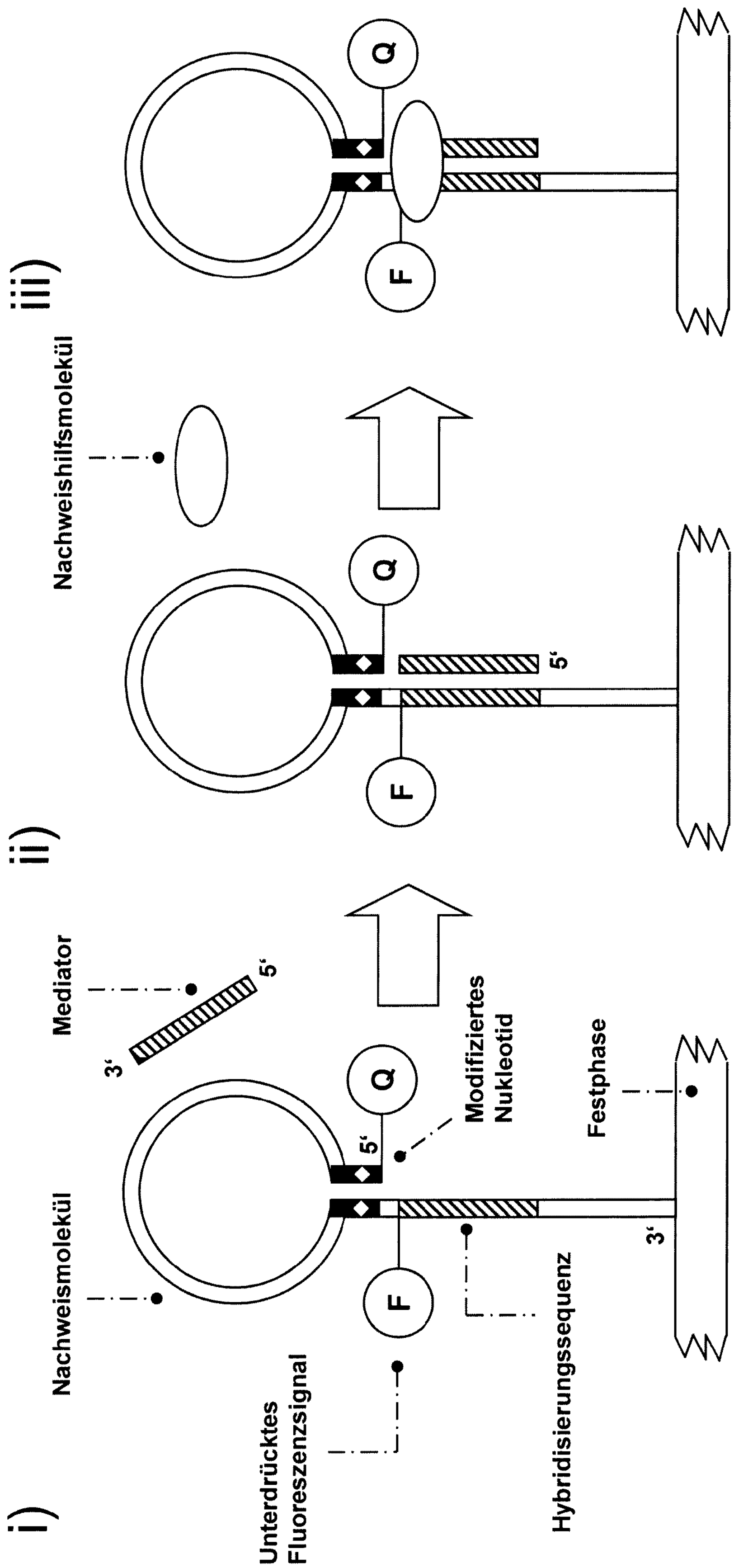
i)
ii)
iii)
Nachweismolekül
Mediator
Nachweishilfsmolekül
Unterdrücktes Fluoreszenzsignal
Modifiziertes Nukleotid
Hybridisierungssequenz
Festphase
F
Q
3'
5'

Fig. 7 (Fortsetzung)

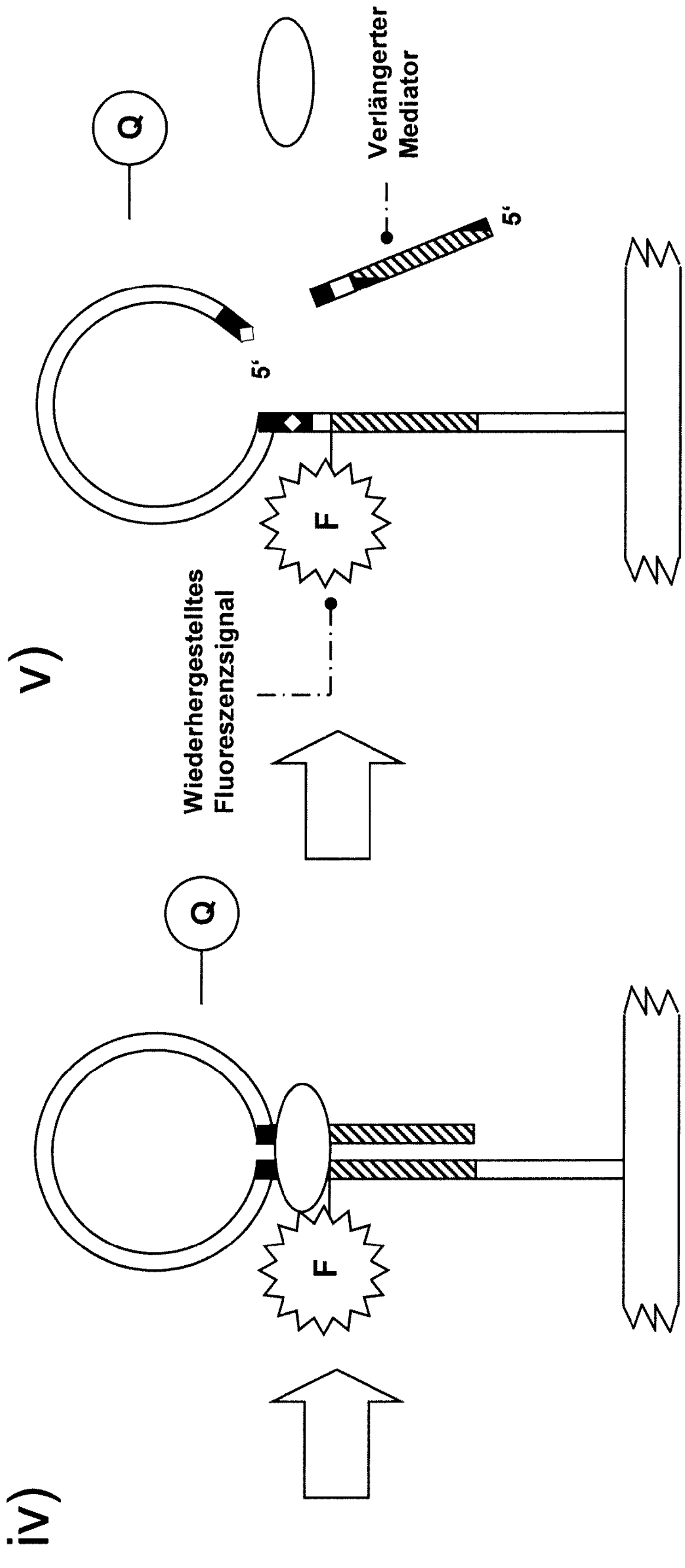

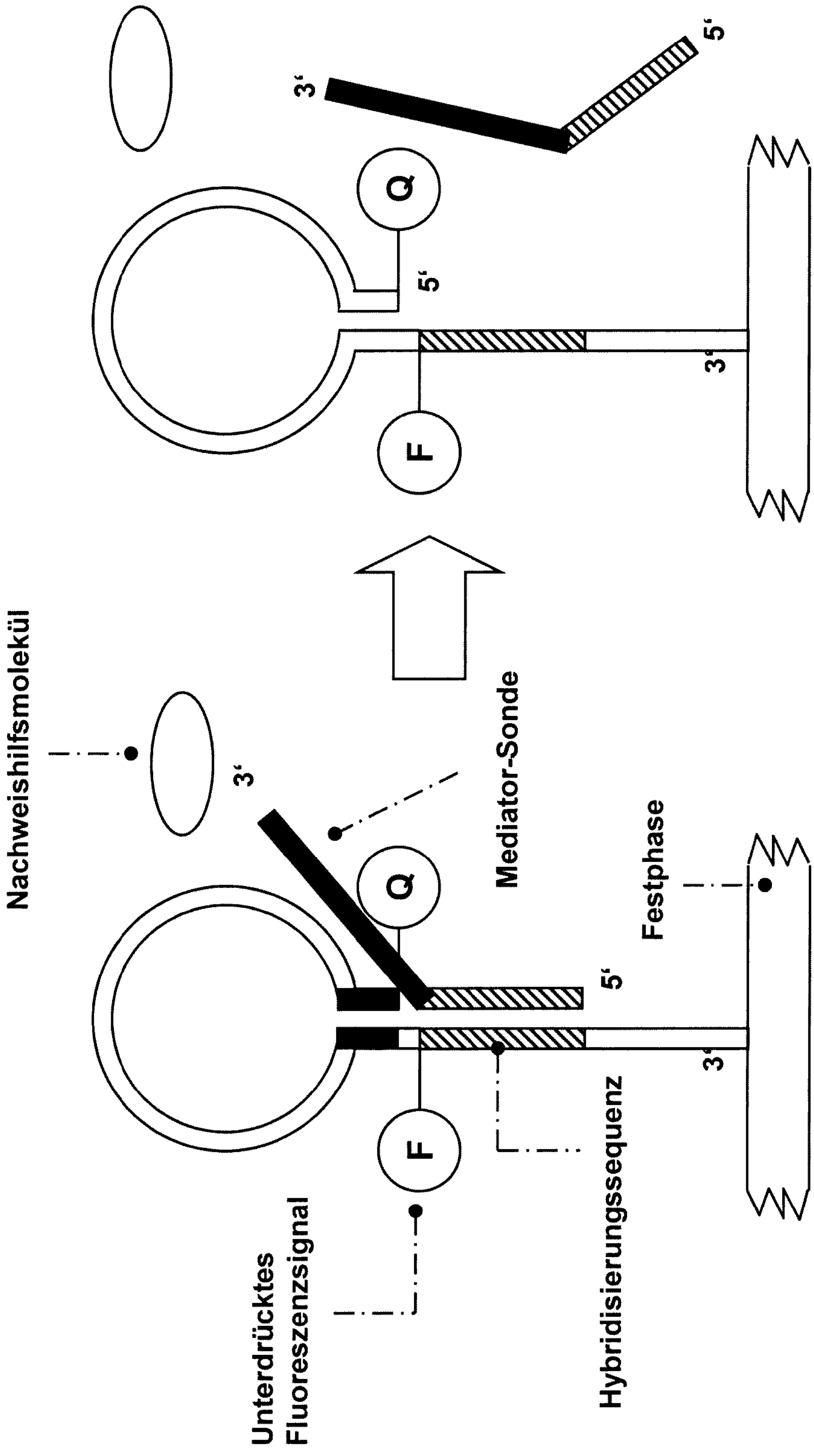
Nachweishilfsmolekül
Mediator-Sonde
Festphase
Unterdrücktes Fluoreszenzsignal
Hybridisierungssequenz
F
Q
3‘
5‘

Fig. 8

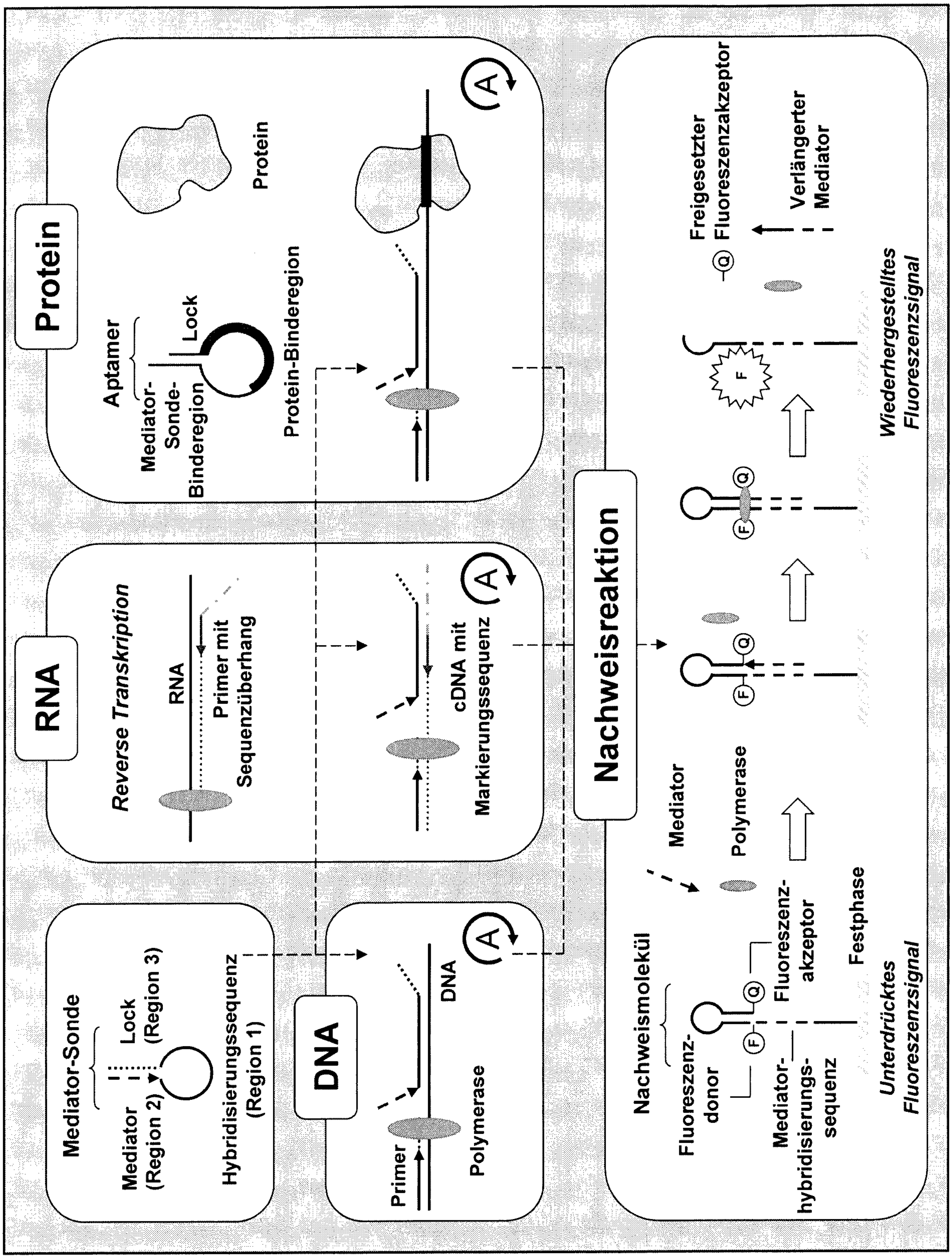
Mediator-Sonde
Mediator (Region 2)
Lock (Region 3)
Hybridisierungssequenz (Region 1)
DNA
Primer
Polymerase
DNA
A
RNA
Reverse Transkription
RNA
Primer mit Sequenzüberhang
cDNA mit Markierungssequenz
A
Protein
Aptamer
Mediator-Sonde-Binderegion
Lock
Protein-Binderegion
Protein
A
Nachweisreaktion
Nachweismolekül
Fluoreszenz-donor
Mediator-hybridisierungs-sequenz
F
Q
Fluoreszenz-akzeptor
Festphase
Unterdrücktes Fluoreszenzsignal
Mediator
Polymerase
F
Q
F
Q
F
Freigesetzter Fluoreszenzakzeptor
Q
Verlängerter Mediator
Wiederhergestelltes Fluoreszenzsignal

Fig. 9

Fig. 10

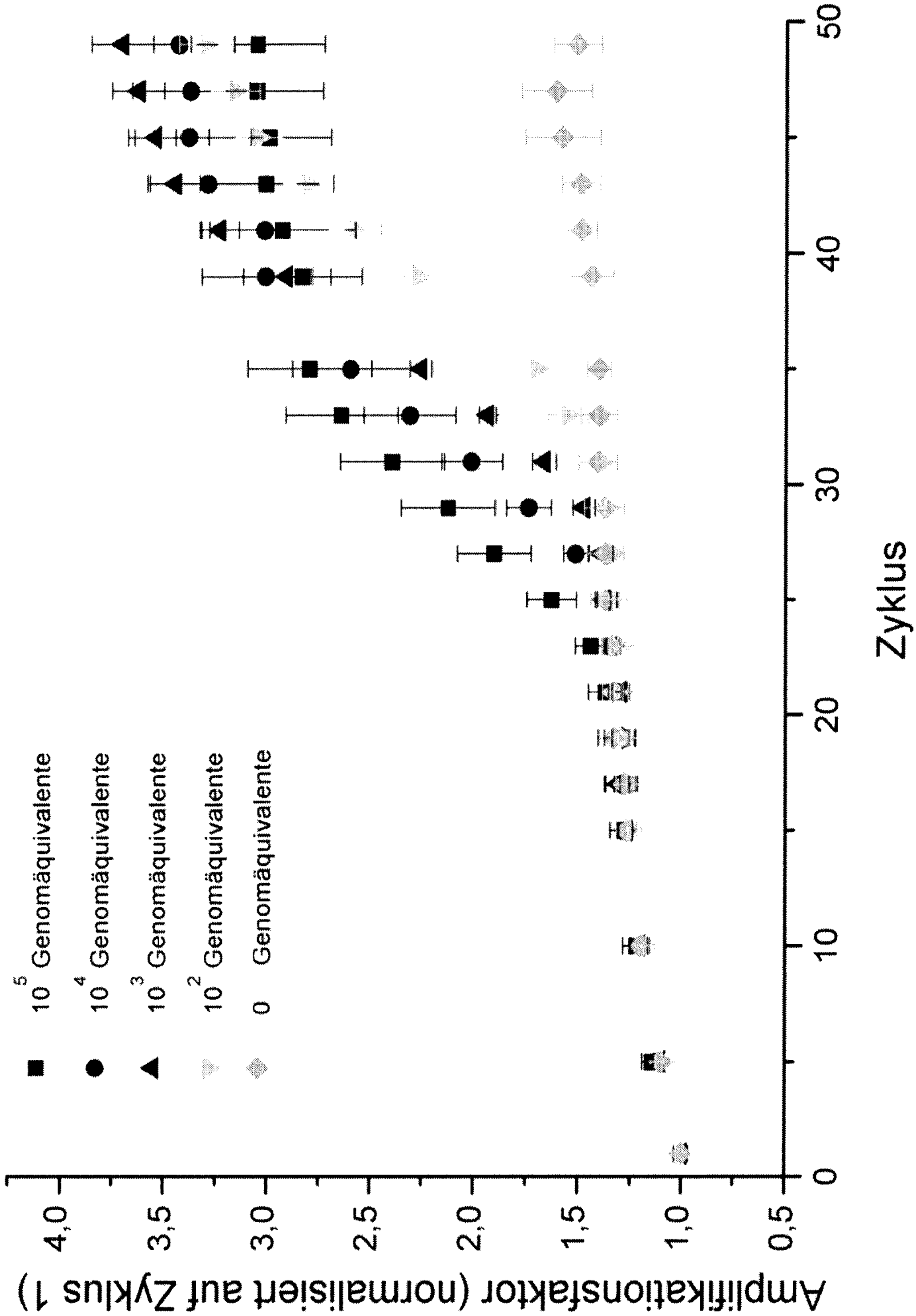

$10^5$ Genomäquivalente
$10^4$ Genomäquivalente
$10^3$ Genomäquivalente
$10^2$ Genomäquivalente
0 Genomäquivalente
Amplifikationsfaktor (normalisiert auf Zyklus 1)
4,0
3,5
3,0
2,5
2,0
1,5
1,0
0,5
0
10
20
30
40
50
Zyklus

Fig. 11

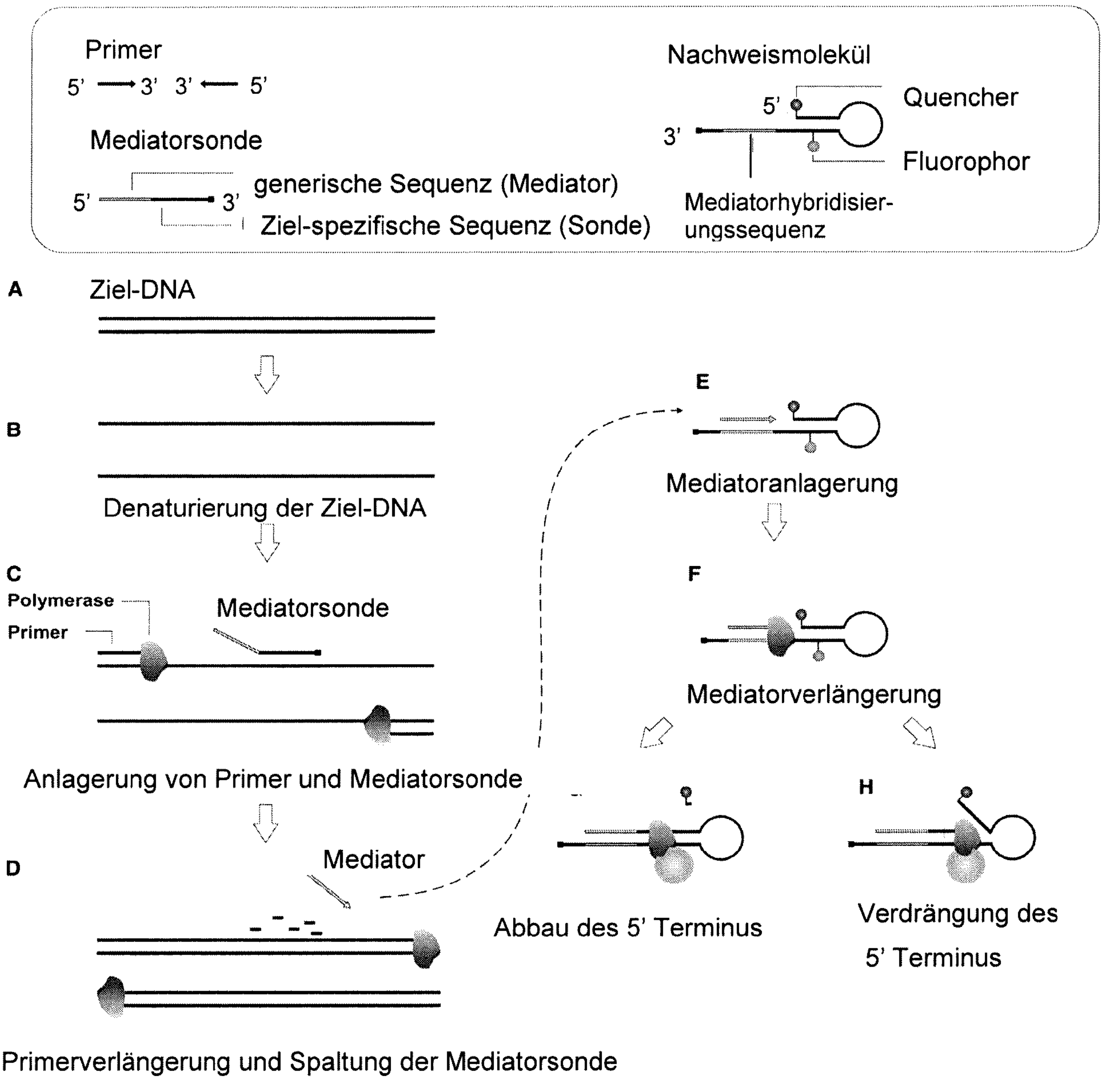

Primer
5' 3' 3' 5'
Mediatorsonde
5' 3'
generische Sequenz (Mediator)
Ziel-spezifische Sequenz (Sonde)
Nachweismolekül
5'
3'
Quencher
Fluorophor
Mediatorhybridisier-ungssequenz
A
Ziel-DNA
B
Denaturierung der Ziel-DNA
C
Polymerase
Primer
Mediatorsonde
Anlagerung von Primer und Mediatorsonde
D
Mediator
Primerverlängerung und Spaltung der Mediatorsonde
E
Mediatoranlagerung
F
Mediatorverlängerung
H
Abbau des 5' Terminus
Verdrängung des 5' Terminus

Fig. 12

A

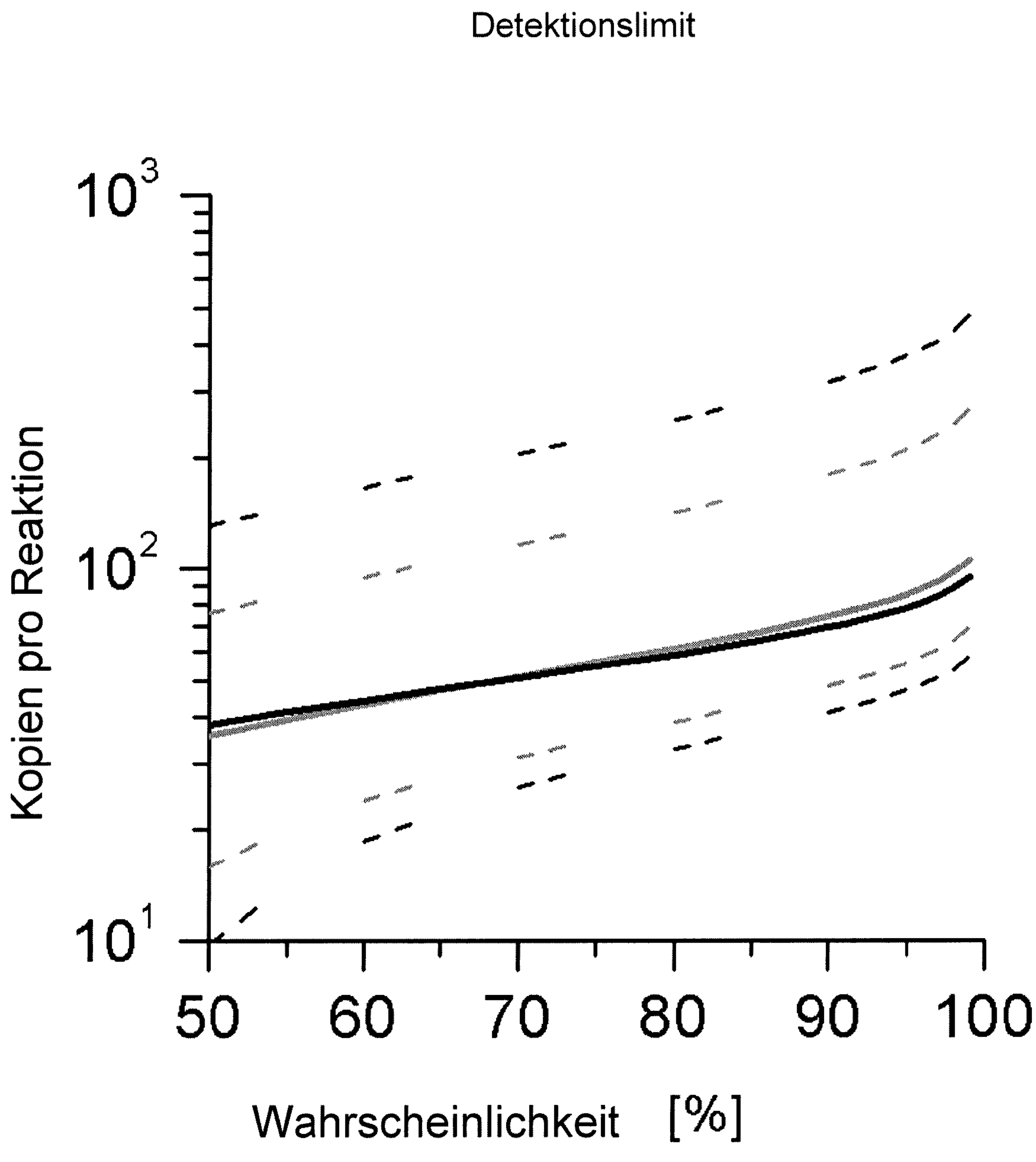
Detektionslimit
$10^3$
$10^2$
$10^1$
Kopien pro Reaktion
50
60
70
80
90
100
Wahrscheinlichkeit [%]

Fig. 12 (Fortsetzung)

B

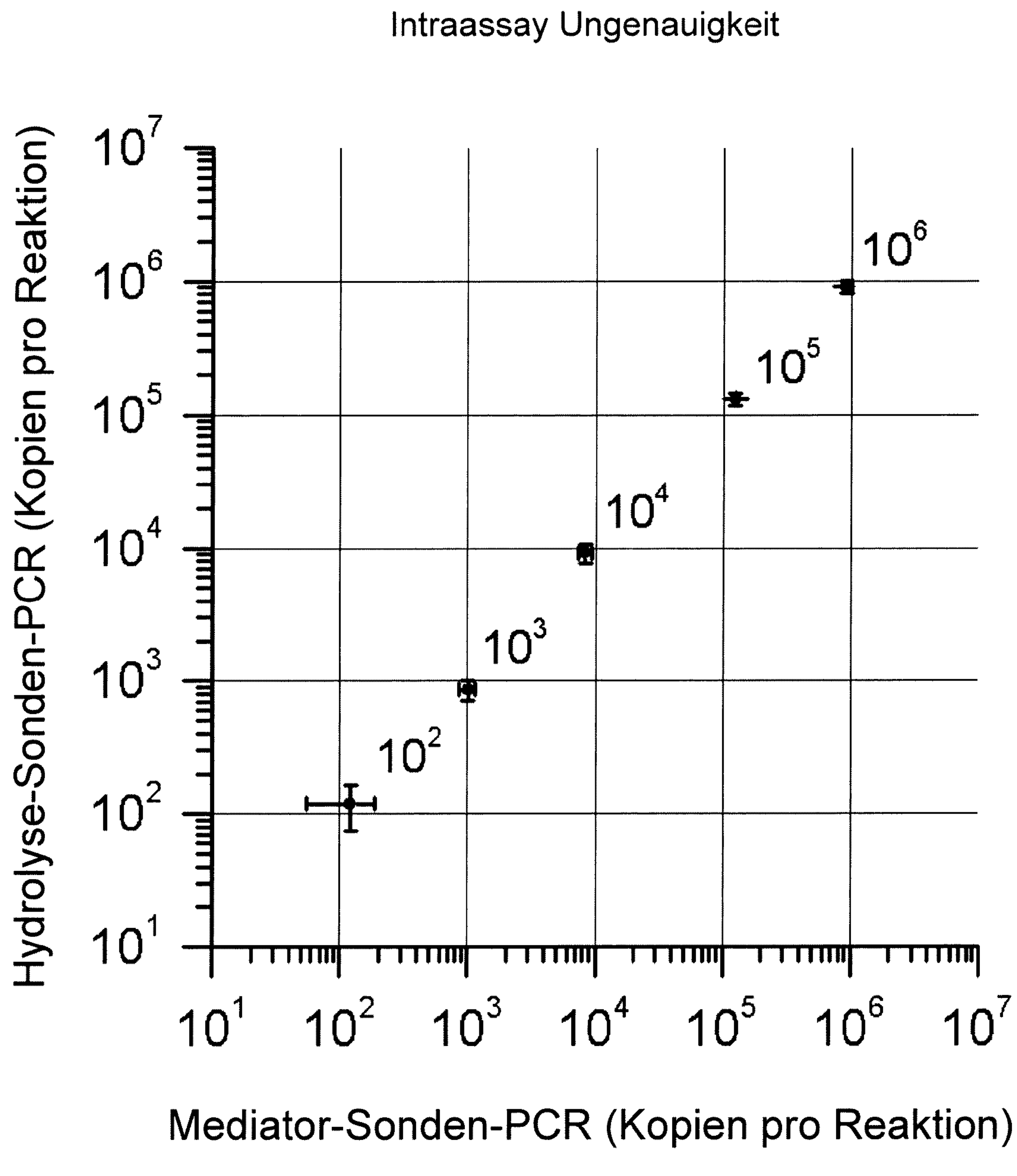

Fig. 12 (Fortsetzung)

C

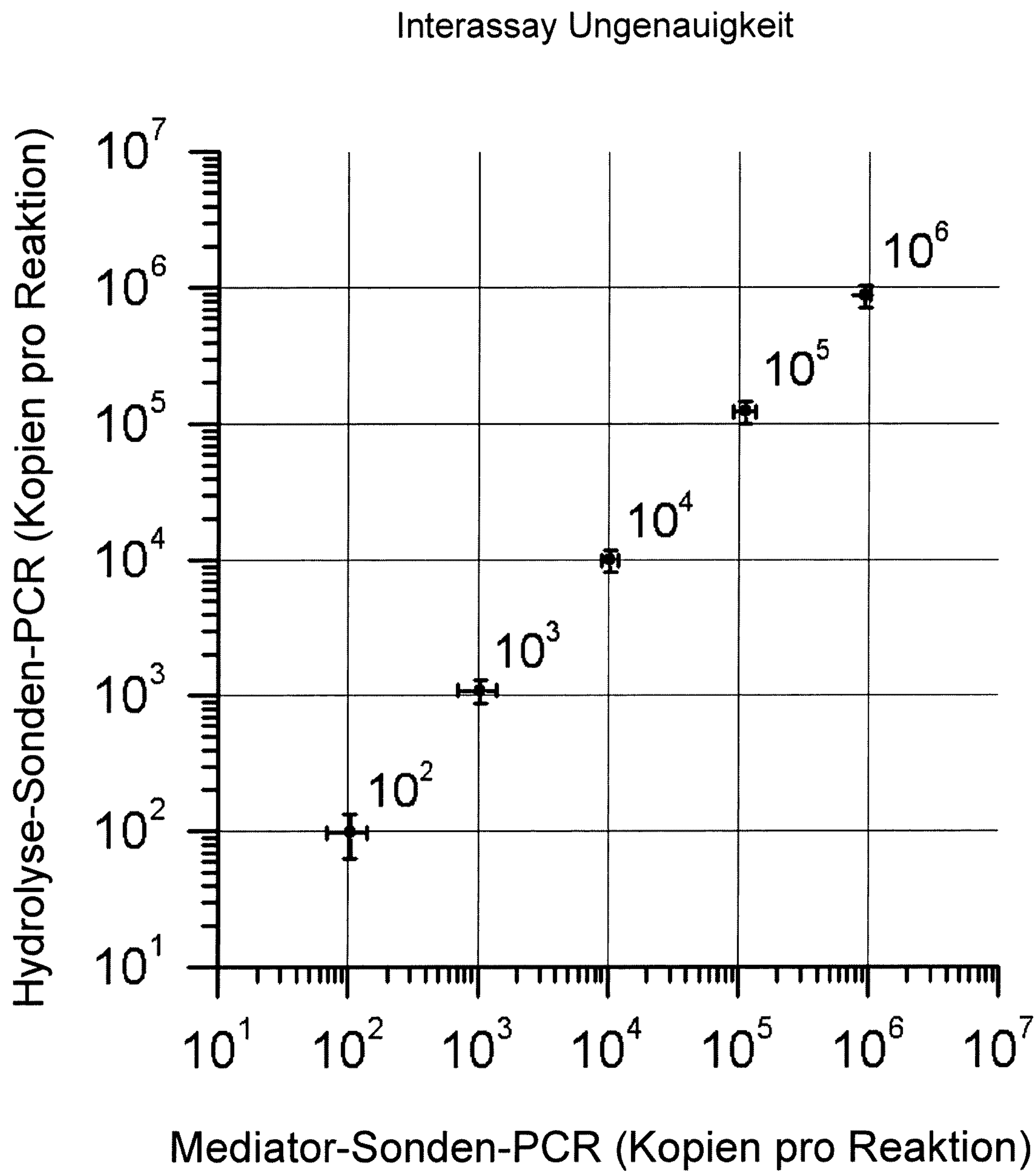

Fig. 12 (Fortsetzung)

D

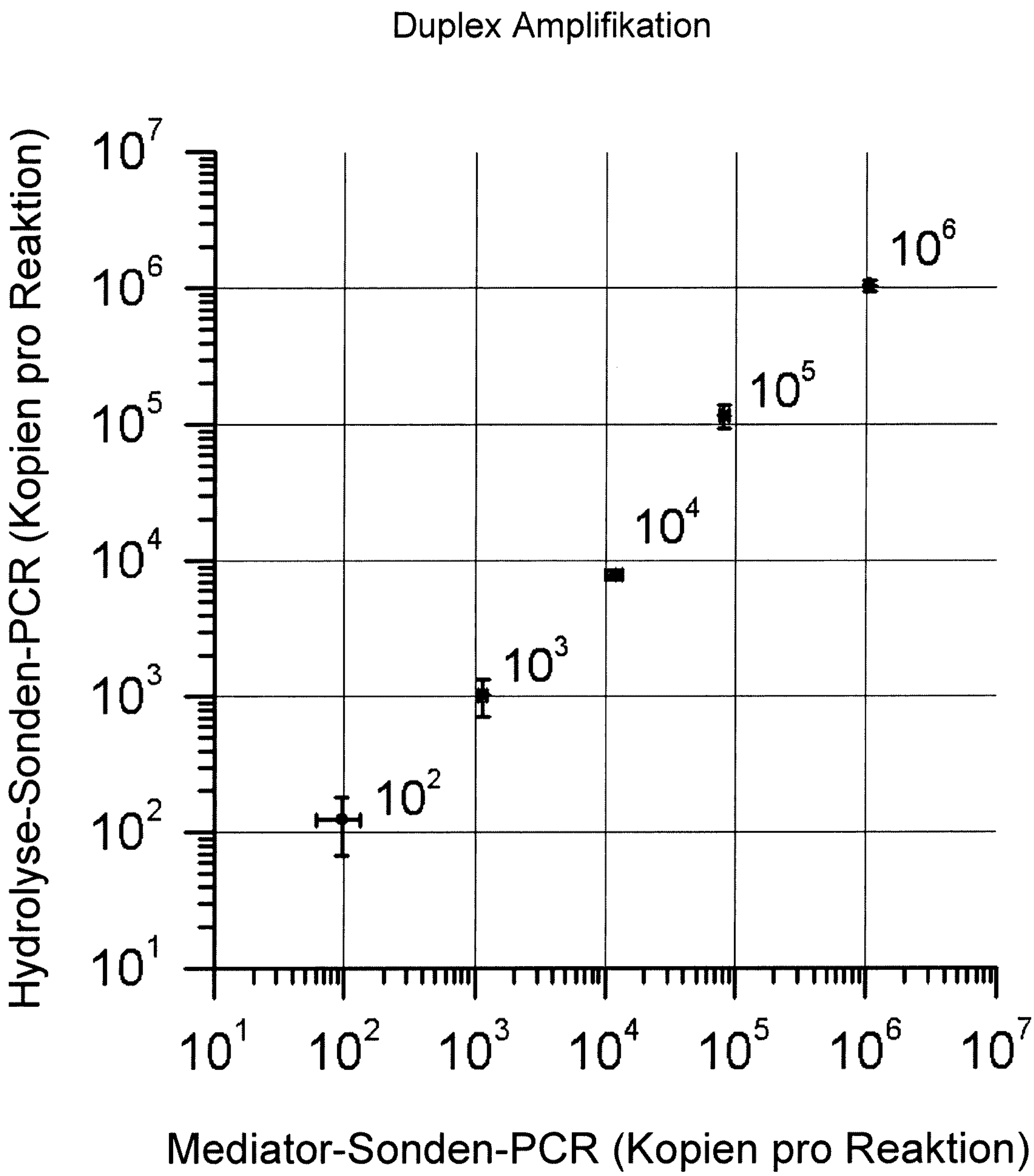

Fig.13

A

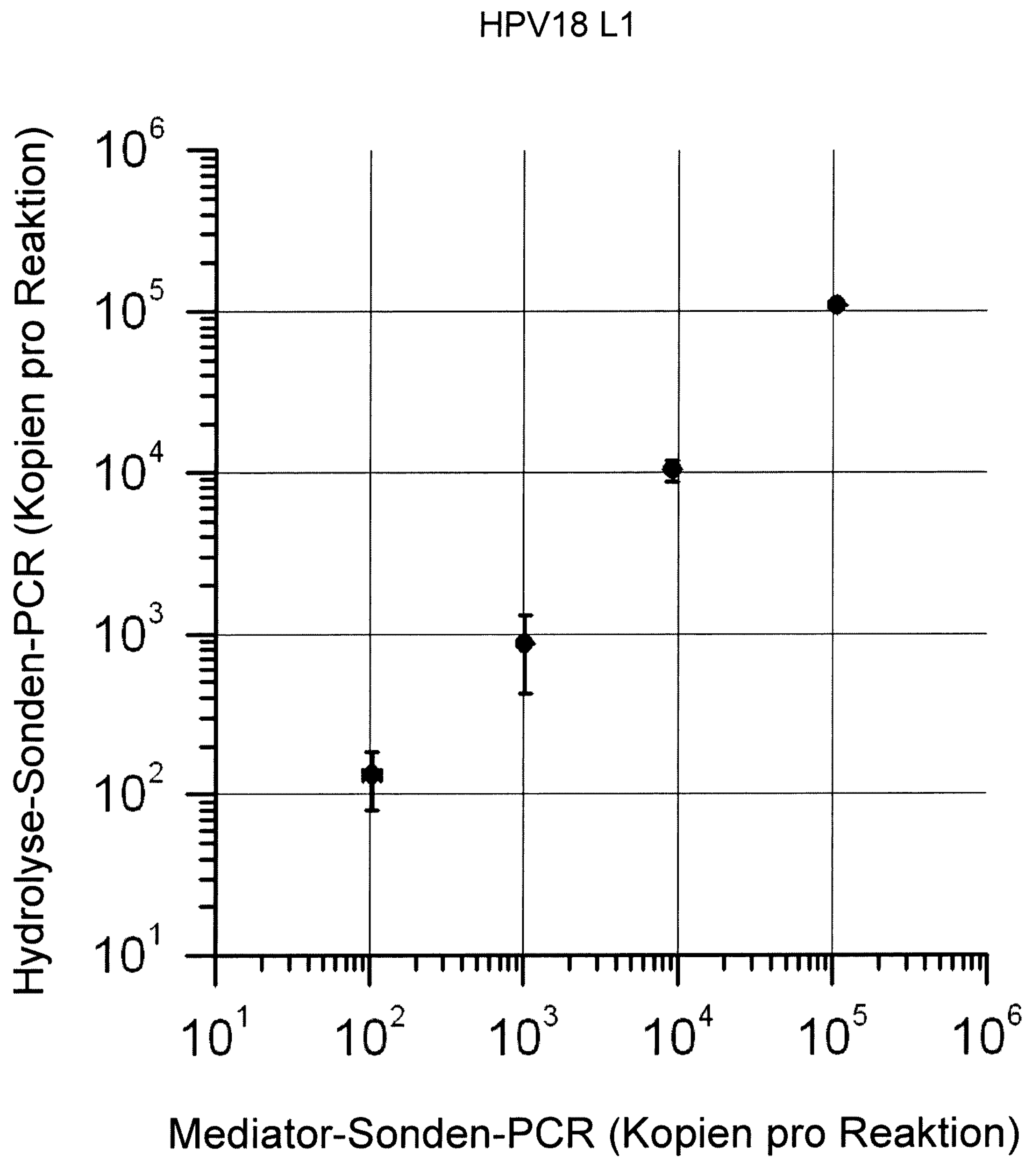
HPV18 L1
Hydrolyse-Sonden-PCR (Kopien pro Reaktion)
10^6
10^5
10^4
10^3
10^2
10^1
10^1
10^2
10^3
10^4
10^5
10^6
Mediator-Sonden-PCR (Kopien pro Reaktion)

Fig.13 (Fortsetzung)

B

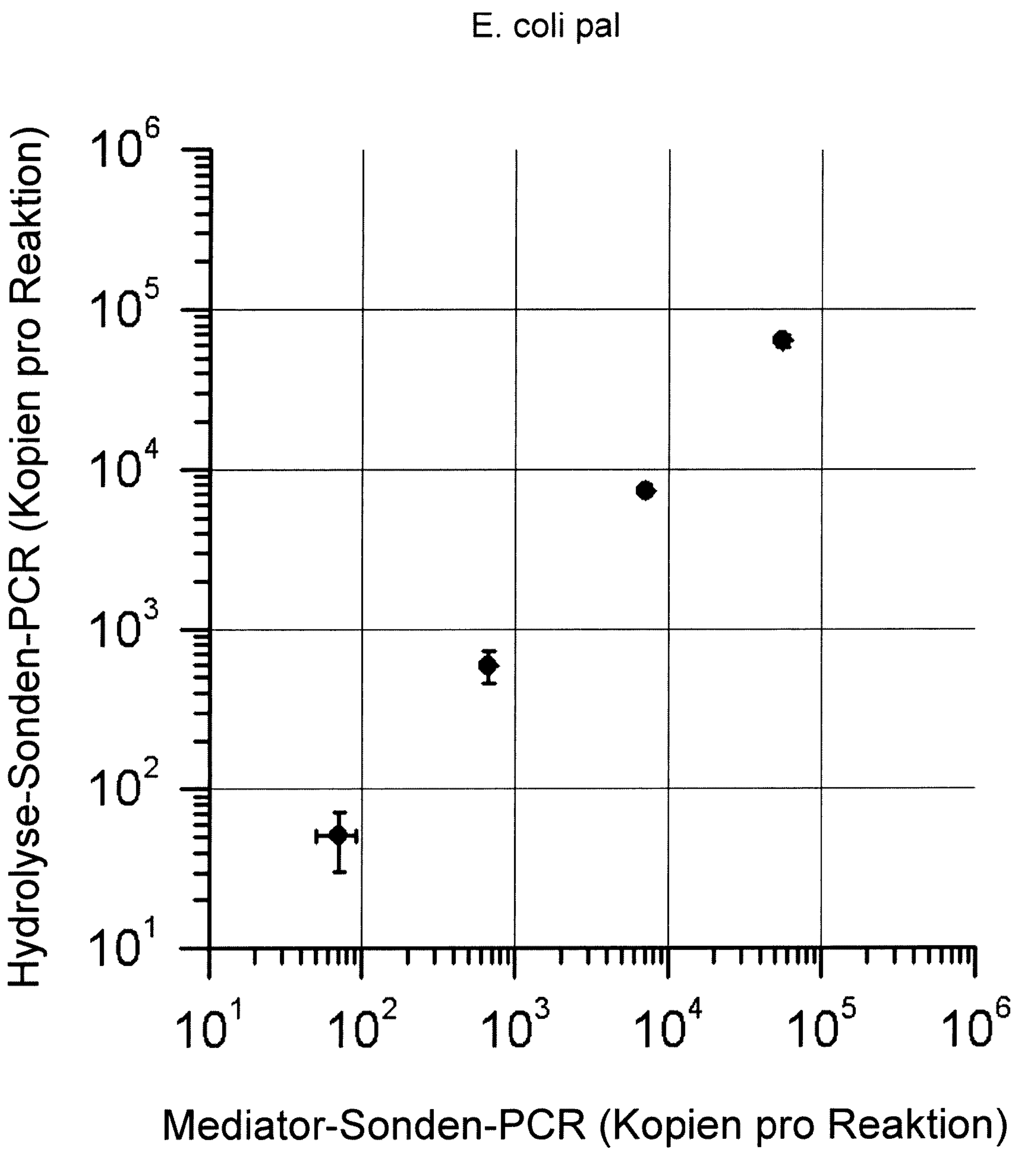

Fig. 13 (Fortsetzung)

C

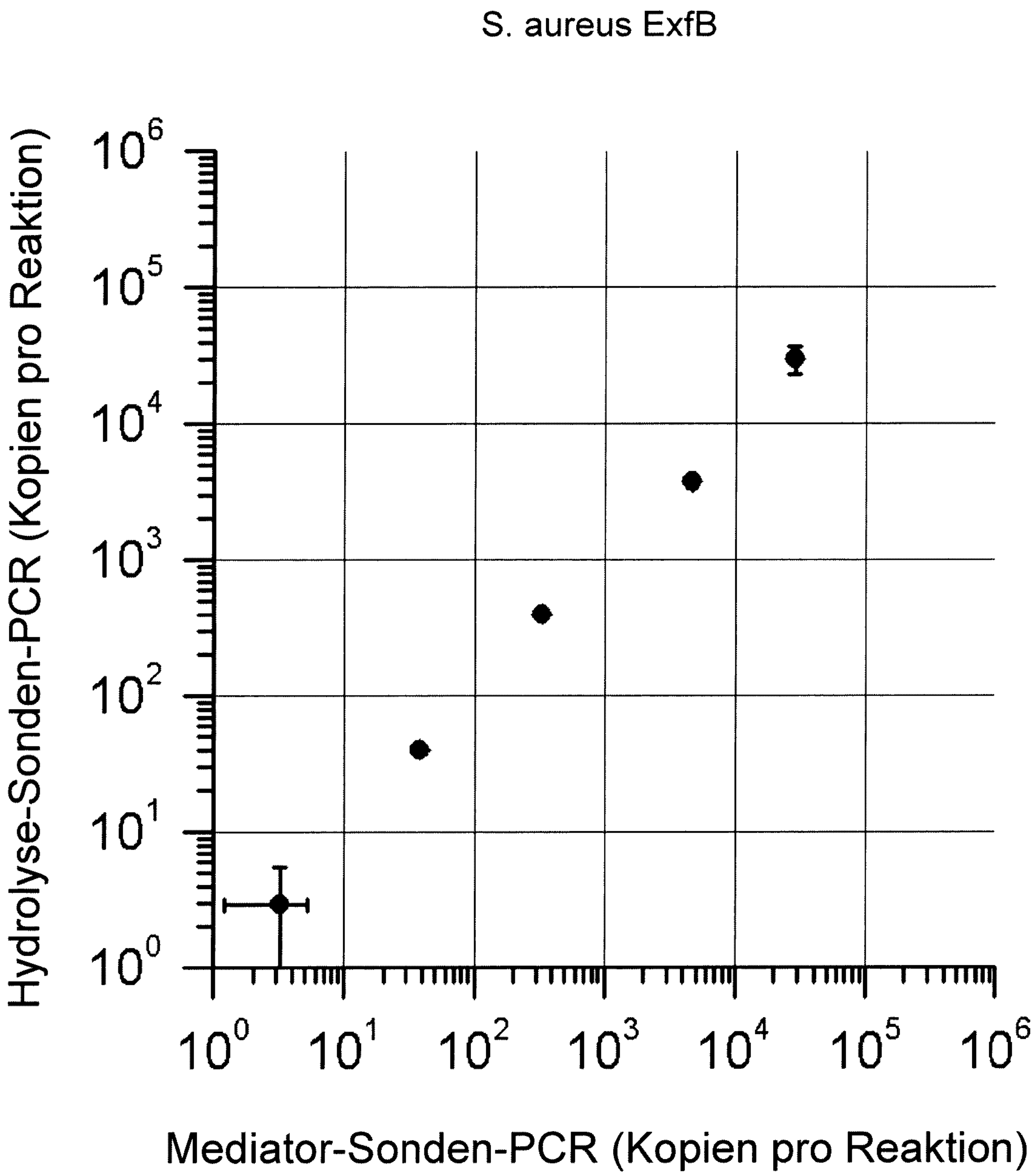

Fig.13 (Fortsetzung)

D

H. sapiens ACTB

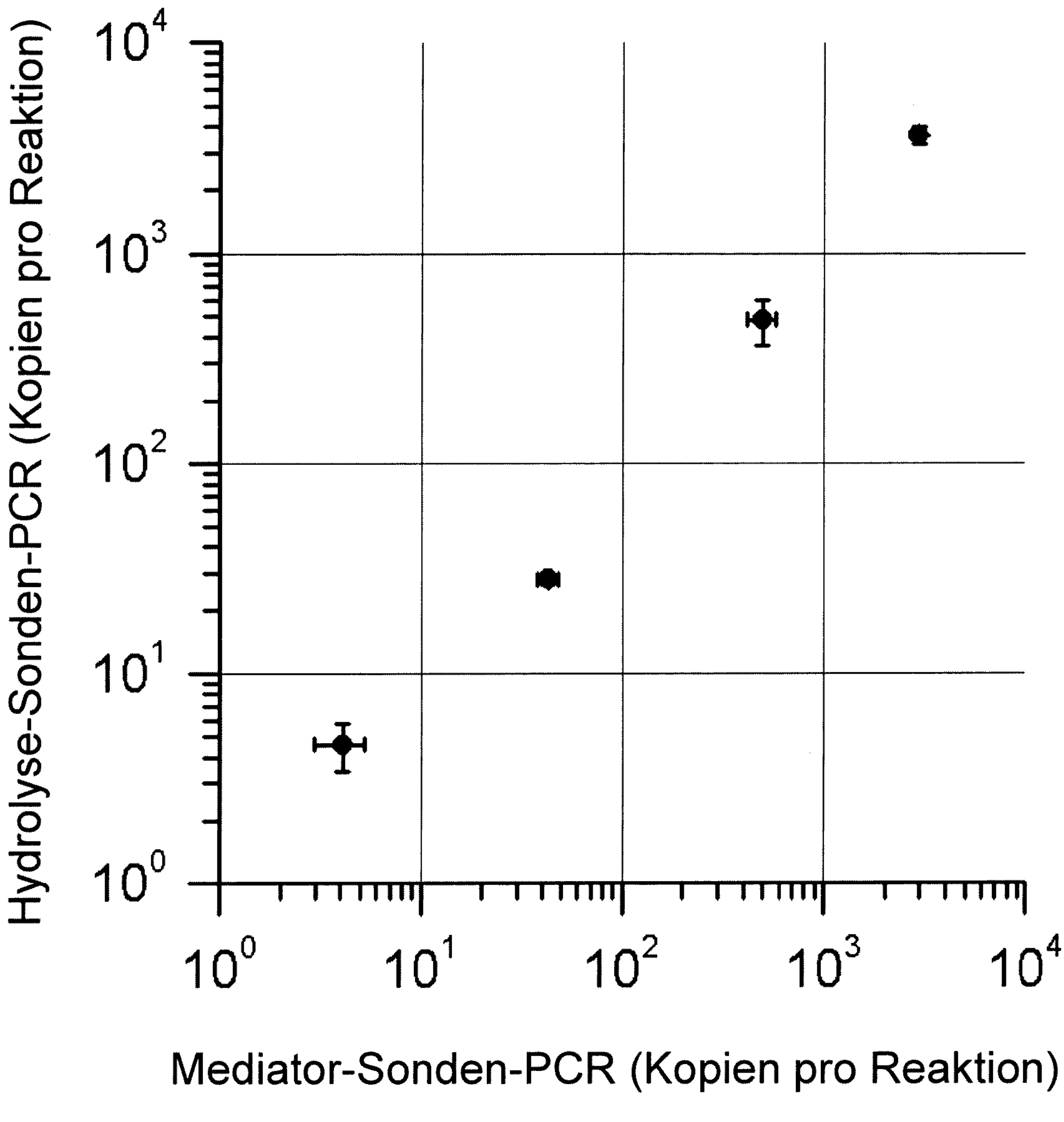

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5641658 A
- US 6300070 B1
- DE 10316159 A1
- US 5653939 A
- US 6099803 A
- US 20010034025 A1
- US 6238869 B1
- US 20020110826 A
- WO 102011055247 A


### In der Beschreibung aufgeführte Nicht-Patentliteratur


- **LIU,Q. et al.** Microarray-in-a-tube for detection of multiple viruses. *Clin Chem,* 2007, vol. 53, 188-194
- **PEMOV,A. et al.** DNA analysis with multiplex microarray-enhanced PCR. *Nucleic Acids Res.,* 2005, vol. 33, e11
- **ANDRESEN, D. ; NICKISCH-ROSENEGK, M. ; BIER, F.F.** Helicase dependent OnChipamplification and its use in multiplex pathogen detection. *Clin. Chim. Acta,* 2009, vol. 403, 244-248
- **LIU,H.P. et al.** TaqMan probe array for quantitative detection of DNA targets. *Nucleic Acids Research,* 2006, vol. 34
- **LOCKETT et al.** *Anal. Chem.,* 2007, vol. 79, 6031-6036
- **LU et al.** *Hum. Mutat.,* 2002, vol. 19, 416-422
- **LI et al.** *Analytical Chemistry,* 15. November 2006, vol. 78 (22), 7886-7890
- **NUOVO et al.** *journal of histochemistry and cytochemistry,* 01. Marz 1999, vol. 47 (3), 273-279
- **HALL et al.** *PNAS,* 18. Juli 2000, vol. 97 (15), 8272-8277
- **BAEUMNER,A.J. et al.** A universal nucleic acid sequence biosensor with nanomolar detection limits. *Anal. Chem.,* 2004, vol. 76, 888-894
- **LU,M.C. et al.** A surface invasive cleavage assay for highly parallel SNP analysis. *Hum. Mutat.,* 2002, vol. 19, 416-422
- **NIE,B. et al.** Quantitative detection of individual cleaved DNA molecules on surfaces using gold nanoparticles and scanning electron microscope imaging. *Anal. Chem.,* 2006, vol. 78, 1528-1534
- **CHEN,Y. et al.** Surface amplification of invasive cleavage products. *J. Amer. Chem. Soc.,* 2004, vol. 126, 3016-3017
- **LOCKETT,M.R. et al.** Molecular beacon-style hybridization assay for quantitative analysis of surface invasive cleavage reactions. *Anal. Chem.,* 2007, vol. 79, 6031-6036

SEQUENCE LISTING

<110> Albert-Ludwigs-Universität Freiburg

<120> Multianalyt Reportersystem

<130> XII 676/12

<150> 10 2011 055 247.2
<151> 2012-11-10

<160> 20

<170> PatentIn version 3.5

<210> 1
<211> 66
<212> DNA
<213> Artificial Sequence

<220>
<223> Sonde

<400> 1

**ccgcagaaga tgagatcgcg gtgttggtcg tagagcccag aacgattttt tttttttttt 60**

**tttttt 66**

<210> 2
<211> 66
<212> DNA
<213> Artificial Sequence

<220>
<223> Sonde

<400> 2

**ccgcagaaga tgagatcgcg gtgttcactg accgaactgg agcatttttt tttttttttt 60**

**tttttt 66**

<210> 3
<211> 22
<212> DNA
<213> Artificial Sequence

<220>
<223> Primer

<400> 3
ggcaattgcg gcatgttctt cc 22

<210> 4
<211> 22

<212> DNA
<213> Artificial Sequence

<220>
<223> Primer

<400> 4
tgttgcattt gcagacgagc ct 22

<210> 5
<211> 27
<212> DNA
<213> Artificial Sequence

<220>
<223> Sonde

<400> 5
atgcgaacgg cggcaacggc aacatgt 27

<210> 6
<211> 44
<212> DNA
<213> Artificial Sequence

<220>
<223> Sonde

<400> 6
aaatcgttct gggctctacg cgaacggcgg caacggcaac atgt 44

<210> 7
<211> 24
<212> DNA
<213> Artificial Sequence

<220>
<223> Primer

<400> 7
agatgcacgt actgctgaaa tgag 24

<210> 8
<211> 26
<212> DNA
<213> Artificial Sequence

<220>
<223> Primer

<400> 8
aataaagtac ggatcaacag ctaaac 26

<210> 9
<211> 20
<212> DNA
<213> Artificial Sequence

<220>
<223> Sonde

<400> 9
ccgcctactc ctggaccagg 20

<210> 10
<211> 48
<212> DNA
<213> Artificial Sequence

<220>
<223> Sonde

<400> 10
aaatcgttct gggctctacg gtattcacag tggtaaaggc ggacaaca 48

<210> 11
<211> 25
<212> DNA
<213> Artificial Sequence

<220>
<223> Primer

<400> 11
gctggcagct ctagattatt aactg 25

<210> 12
<211> 21
<212> DNA
<213> Artificial Sequence

<220>
<223> Primer

<400> 12
ggtcaggtaa ctgcacccta a 21

<210> 13
<211> 20
<212> DNA
<213> Artificial Sequence

<220>
<223> Sonde

<400> 13
ggttcctgca ggtggtggca 20

<210> 14
<211> 39
<212> DNA
<213> Artificial Sequence

<220>
<223> Sonde

<400> 14
aaatcgttct gggctctacg gttcctgcag gtggtggca 39

<210> 15
<211> 25
<212> DNA
<213> Artificial Sequence

<220>
<223> Primer

<400> 15
tcacccacac tgtgcccatc tacga 25

<210> 16
<211> 25
<212> DNA
<213> Artificial Sequence

<220>
<223> Primer

<400> 16
cagcggaacc gctcattgcc aatgg 25

<210> 17
<211> 26
<212> DNA
<213> Artificial Sequence

<220>
<223> Sonde

<400> 17
atgccctccc ccatgccatc ctgcgt 26

<210> 18
<211> 26
<212> DNA
<213> Artificial Sequence

<220>
<223> Sonde

<400> 18
atgccctccc ccatgccatc ctgcgt 26

<210> 19
<211> 43
<212> DNA
<213> Artificial Sequence

<220>
<223> Sonde

<400> 19
aaatcgttct gggctctacg ccctccccca tgccatcctg cgt 43

<210> 20
<211> 43
<212> DNA
<213> Artificial Sequence

<220>
<223> Sonde

<400> 20
atgctccagt tcggtcagtg ccctccccca tgccatcctg cgt 43